(19) Europäisches Patentamt
European Patent Office
Office européen des brevets

(11) EP 1 042 326 B1

(12) **FASCICULE DE BREVET EUROPEEN**

(45) Date de publication et mention de la délivrance du brevet:
**19.06.2002 Bulletin 2002/25**

(21) Numéro de dépôt: **98962511.6**

(22) Date de dépôt: **18.12.1998**

(51) Int Cl.7: **C07D 491/052**, A61K 31/44, C07D 491/14, C07D 498/14 // (C07D491/052, 311:00), C07D221:00

(86) Numéro de dépôt international:
**PCT/FR98/02785**

(87) Numéro de publication internationale:
**WO 99/32491 (01.07.1999 Gazette 1999/26)**

(54) **NOUVEAUX DERIVES DE L'ACRONYCINE, LEUR PROCEDE DE PREPARATION ET LES COMPOSITIONS PHARMACEUTIQUES QUI LES CONTIENNENT**

ACRONYCINDERIVATE, VERFAHREN ZU IHRER HERSTELLUNG UND DIESE ENTHALTENDE PHARMAZEUTISCHE ZUBEREITUNGEN

NOVEL ACRONYCINE DERIVATIVES, PREPARATION METHOD AND PHARMACEUTICAL COMPOSITIONS

(84) Etats contractants désignés:
**AT BE CH CY DE DK ES FI FR GB GR IE IT LI LU NL PT SE**

(30) Priorité: **19.12.1997 FR 9716131**

(43) Date de publication de la demande:
**11.10.2000 Bulletin 2000/41**

(73) Titulaire: **LES LABORATOIRES SERVIER**
**92200 Neuilly sur Seine (FR)**

(72) Inventeurs:
- **KOCH, Michel**
  **F-78170 La Celle Saint Cloud (FR)**
- **TILLEQUIN, François**
  **F-75014 Paris (FR)**
- **MICHEL, Sylvie**
  **F-75002 Paris (FR)**
- **ATASSI, Ghanem**
  **F-92210 Saint-Cloud (FR)**
- **PIERRE, Alain**
  **F-78580 Les Alluets le Roi (FR)**
- **PFEIFFER, Bruno**
  **F-95320 Saint Leu la Forêt (FR)**
- **RENARD, Pierre**
  **F-78150 Le Chesnay (FR)**

(56) Documents cités:
- **A. ELOMRI ET AL.: "Synthesis and cytotoxic and antitumor activity of esters in the 1,2-dihdroxy-1,2-dihydroacronycine series" JOURNAL OF MEDICINAL CHEMISTRY., vol. 39, no. 24, 1996, pages 4762-4764, XP002076704 WASHINGTON US cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 042 326 B1

Printed by Jouve, 75001 PARIS (FR)

## Description

La présente invention concerne de nouveaux dérivés de l'acronycine, leur procédé de préparation et les compositions pharmaceutiques qui les contiennent.

L'acronycine est un alcaloïde présentant des propriétés antitumorales mises en évidence dans des modèles expérimentaux (*J. Pharmacol. Sci.* 1966, 55 (8), 758-768). Cependant, malgré un large spectre d'activité, la faible solubilité de l'acronycine limite sa biodisponibilité et ne permet pas d'envisager son utilisation dans un mode d'administration de type injectable.

Diverses modifications chimiques ont été réalisées sur cette molécule, comme celles décrites dans l'article *J. Med. Chem.* 1996, 39, 4762-4766, et qui ont permis de résoudre en partie ce problème de solubilité des dérivés.

Les composés de l'invention, outre le fait qu'ils soient nouveaux, présentent des propriétés de solubilisation intéressantes et adaptées à une administration des produits sous formes liquides et présentent aussi de façon surprenante une activité in-vitro et in-vivo très supérieures à celles observées jusqu'ici. Les nouveaux analogues, découverts par la Demanderesse, possèdent ainsi des propriétés antitumorales qui les rendent particulièrement adaptés pour une utilisation dans le traitement des cancers et notamment des tumeurs solides.

Plus particulièrement, la présente invention concerne les composés de formule (1) :

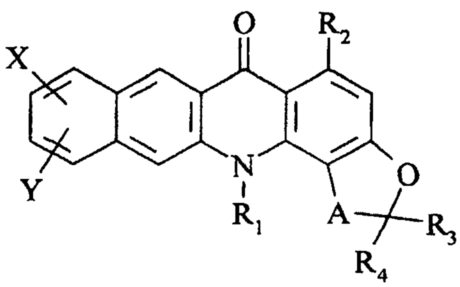

(I)

dans laquelle :

X, Y, identiques ou différents, indépendamment l'un de l'autre, représentent un atome d'hydrogène, d'halogène, un groupement hydroxy, mercapto, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, amino (éventuellement substitué par un ou deux groupements, identiques ou différents, alkyle ($C_1$-$C_6$) linéaires ou ramifiés eux-mêmes éventuellement substitués par un groupement alkoxy ($C_1$-$C_6$) linéaire ou ramifié ou par un groupement de formule $-NR'_7R'_8$ dans laquelle $R'_7$ et $R'_8$, identiques ou différents, indépendamment l'un de l'autre, représentent un atome d'hydrogène ou un groupement alkyle ($C_1$-$C_6$) linéaire ou ramifié), ou forment ensemble un groupement méthylènedioxy ou un groupement éthylènedioxy,

$R_1$ représente un atome d'hydrogène ou un groupement alkyle ($C_1$-$C_6$) linéaire ou ramifié,

$R_2$ représente :

- un atome d'hydrogène,
- un groupement hydroxy,
- un groupement alkyle ($C_1$-$C_6$) linéaire ou ramifié,
- un groupement alkoxy ($C_1$-$C_6$) linéaire ou ramifié, éventuellement substitué par un groupement :
  - * de formule $NR_7R_8$ dans laquelle $R_7$ et $R_8$, identiques ou différents, indépendamment l'un de l'autre, représentent un atome d'hydrogène, un groupement alkyle ($C_1$-$C_6$) linéaire ou ramifié, ou un groupement hydroxyalkyle ($C_1$-$C_6$) linéaire ou ramifié,
  - * ou par un hétérocycle saturé ou insaturé, monocyclique ou bicyclique, de 5 à 7 chaînons comportant un ou deux hétéroatomes choisis parmi oxygène, azote et soufre,
- un groupement alkylcarbonyloxy ($C_1$-$C_6$) linéaire ou ramifié,
- ou un groupement amino éventuellement substitué :

* par un ou deux groupements alkyles ($C_1$-$C_6$) linéaires ou ramifiés, identiques ou différents,
* par un groupement alkylcarbonyle ($C_1$-$C_6$) linéaire ou ramifié, éventuellement substitué par un groupement -$NR_7R_8$ avec $R_7$ et $R_8$ tels que définis précédemment,
* par un groupement de formule -$R_9$-$NR_7R_8$, dans laquelle $R_9$ représente un groupement alkylène ($C_1$-$C_6$) linéaire ou ramifié et $R_7$, $R_8$, identiques ou différents, indépendamment l'un de l'autre représentent un atome d'hydrogène, un groupement alkyle ($C_1$-$C_6$) linéaire ou ramifié, ou un groupement hydroxyalkyle ($C_1$-$C_6$) linéaire ou ramifié,
* par un groupement alkylène ($C_1$-$C_6$), linéaire ou ramifié, substitué par un hétérocycle saturé ou insaturé, monocyclique ou bicyclique, de 5 à 7 chaînons comportant un ou deux hétéroatomes choisis parmi oxygène, azote et soufre,
* ou par un groupement de formule

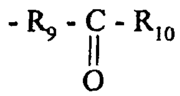

dans laquelle $R_9$ est tel que défini précédemment, et $R_{10}$ représente un groupement hydroxy ou alkoxy ($C_1$-$C_6$) linéaire ou ramifié,

$R_3$, $R_4$, identiques ou différents, indépendamment l'un de l'autre, représentent un atome d'hydrogène ou un groupement alkyle ($C_1$-$C_6$) linéaire ou ramifié,

A représente un groupement -CH=CH- ou un groupement éthylène de formule -$CH(R_5)$-$CH(R_6)$ dans laquelle $R_5$ et $R_6$, identiques ou différents, indépendamment l'un de l'autre, représentent :

-un atome d'hydrogène,
-un groupement hydroxy,
-un groupement alkoxy ($C_1$-$C_6$) linéaire ou ramifié,
-un groupement alkylcarbonyloxy ($C_1$-$C_6$) linéaire ou ramifié,
-un groupement arylcarbonyloxy,
-un groupement amino éventuellement substitué par un ou deux groupements alkyles ($C_1$-$C_6$) linéaires ou ramifiés, identiques ou différents, ou par un groupement acyle ($C_1$-$C_6$) linéaire ou ramifié,
-un groupement mercapto, un groupement alkylthio ($C_1$-$C_6$) linéaire ou ramifié ou un groupement arylthio,

ou $R_5$ et $R_6$ forment ensemble :

-un groupement

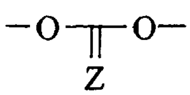 , 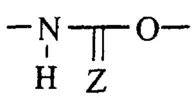 , 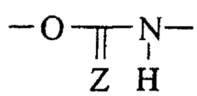

dans lesquels Z représente un atome d'oxygène ou un atome de soufre,

-un groupement -O-$(CH_2)_n$-O- dans lequel n est un entier compris entre 1 et 4 inclus,
-un groupement

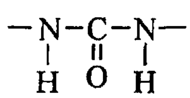 ,

-ou un groupement

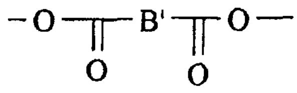 , 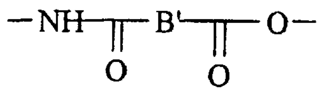 ,

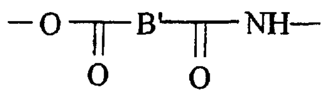

dans lesquels B' représente une liaison simple, un groupement alkylène ($C_1$-$C_6$) linéaire ou ramifié, ou un groupement alkénylène ($C_1$-$C_6$) linéaire ou ramifié,

ou $R_5$ et $R_6$ forment ensemble avec les atomes de carbone qui les portent, un groupement oxirane ou un groupement aziridine, éventuellement substitué sur l'atome d'azote par un groupement alkyle ($C_1$-$C_6$) linéaire ou ramifié,

étant entendu, que le terme "aryle" représente un groupement phényle ou naphtyle, comportant éventuellement une ou plusieurs substitutions, identiques ou différentes, choisies parmi hydroxy, halogéno, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié et alkylcarbonyloxy ($C_1$-$C_6$) linéaire ou ramifié,
leurs isomères optiques, N-oxydes, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les substituants $R_3$ et $R_4$ préférés selon l'invention sont les groupements alkyle ($C_1$-$C_6$) linéaires ou ramifiés, avec $R_3$ et $R_4$ identiques ou différents.

Les substituants $R_2$ préférés selon l'invention sont les groupements alkoxy ($C_1$-$C_6$) linéaires ou ramifiés, ou les groupements amino éventuellement substitués par un ou deux substituants tels que définis précédemment, et avantageusement les groupements amino substitués par un groupement de formule $-R_9$-$NR_7R_8$ avec $R_9$ est tel que défini précédemment, et $R_7$, $R_8$, identiques ou différents, représentent un atome d'hydrogène ou un groupement alkyl ($C_1$-$C_6$) linéaire ou ramifié.

D'une façon avantageuse, les composés préférés de l'invention sont ceux de formule (I) dans laquelle A représente un groupement -CH=CH- ou un groupement de formule $-CH(R_5)$-$CH(R_6)$- dans laquelle $R_5$ et $R_6$ représentent un groupement hydroxy ou alkylcarbonyloxy, ($C_1$-$C_6$) linéaire ou ramifié, ou $R_5$ et $R_6$ forment ensemble un groupement

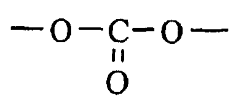 .

D'une façon particulièrement avantageuse, les composés préférés de l'invention sont les composés de formule (I) qui sont le :

* -(±)*cis* -1,2-diacétoxy-6-méthoxy-3,3,14-triméthyl-2,3,7,14-tétrahydro-1H-benzo[b]pyrano [3,2-h]-acridin-7-one,
* -*cis*-7-méthoxy-4,4,15-triméthyl-3a,8,15,15c-tétrahydro-4H-benzo[b][1,3]dioxolo[4',5':4,5] pyrano[3,2-h]acridin-2,8-dione,
* -6-méthoxy-3,3,14-triméthyl-7,14-dihydro-3H-benzo[b]pyrano[3,2-h]acridin-7-one,
* -6-(diéthylaminopropylamino)-3,3,14-triméthyl-7,14-dihydro-3H-benzo[b]pyrano[3,2-h] acridin-7-one,
* -(±)-*cis*-diacétoxy-6(diéthylaminopropylamino)-3,3,14-triméthyl-2,3,7,14-tétrahydro-1H-benzo[b]pyrano[3,2-h] acridin-7-one.

Les isomères optiques, N-oxydes, ainsi que le cas échéant, les sels d'addition à un acide ou à une base, pharmaceutiquement acceptable, des composés préférés font partie intégrante de l'invention.

La présente invention s'étend également au procédé de préparation des composés de formule (I), caractérisé en ce que l'on fait réagir :

- *soit un dérivé de l'acide 3-amino-2-naphtalène carboxylique (II) :*

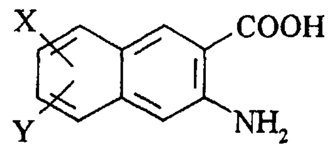

(II)

dans laquelle X et Y sont tels que définis dans la formule (I), avec un dérivé du phloroglucinol de formule (III) :

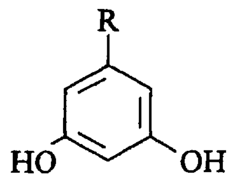

(III)

dans laquelle R représente un atome d'hydrogène, un groupement hydroxy ou un groupement alkyle ($C_1$-$C_6$) linéaire ou ramifié, pour conduire aux composés de formule (IV) :

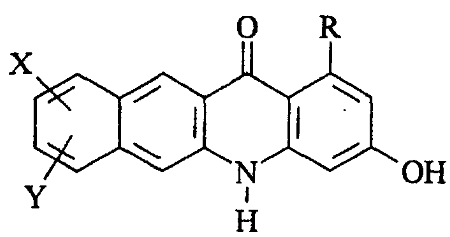

(IV)

dans laquelle X, Y et R sont tels que définis précédemment, qui est ensuite traité en condition basique dans un solvant aprotique tel que la diméthylformamide, par un alcyne de formule V :

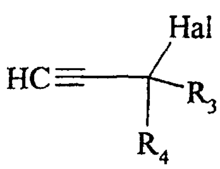

(V)

dans laquelle Hal représente un atome d'halogène et $R_3$ et $R_4$ sont tels que définis dans la formule (I), pour conduire aux composés de formule (I/a), cas particulier des composés de formule (I) :

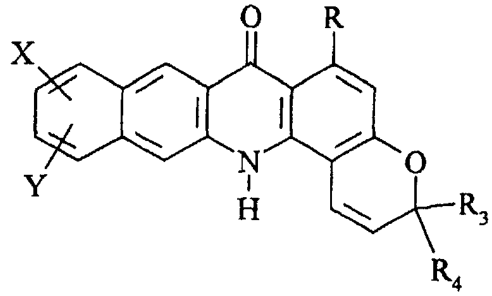

(I/a)

dans laquelle X, Y, R, $R_3$ et $R_4$ sont tels que définis précédemment,

- *soit un dérivé de l'acide 3-halogéno-2-naphtalène carboxylique de formule (VI) :*

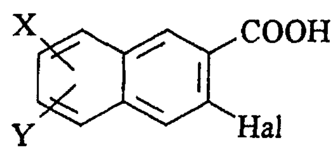

(VI)

dans laquelle X et Y sont tels que définis dans la formule (I) et Hal représente un atome d'halogène, tel que le chlore ou le brome,
avec un dérivé amino-chromène de formule (VII) :

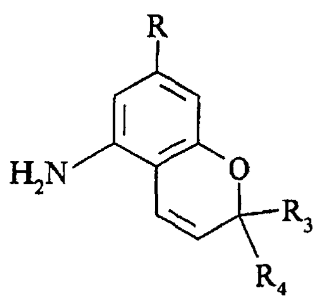

(VII)

dans laquelle $R_3$ et $R_4$ sont tels que définis dans la formule (I) et R a la même signification que précédemment, pour conduire également aux composés de formule (I/a), cas particulier des composés de formule (I) :

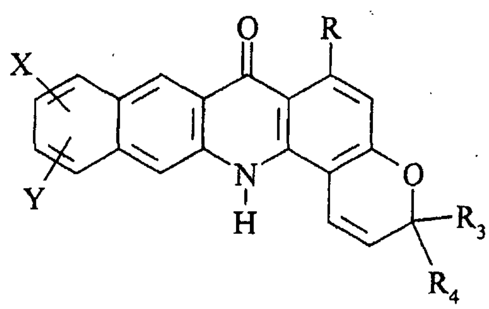

(I/a)

dans laquelle X, Y, R, $R_3$ et $R_4$ sont tels que définis précédemment,

composés de formule (I/a),
dont l'atome d'azote est éventuellement substitué, par action d'un halogénure d'alkyle ou d'un sulfate de dialkyle en présence d'un agent de déprotonation, tel que l'hydrure de sodium, en solvant polaire aprotique, de manière à obtenir les composés de formule (I/b), cas particulier des composés de formule (I):

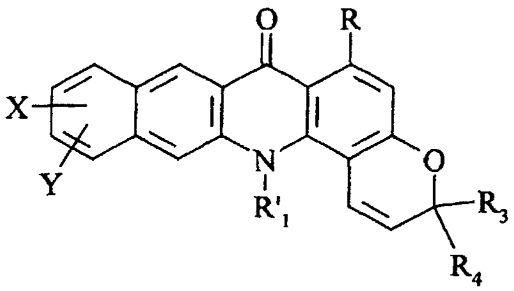

(I/b)

dans laquelle X, Y, R, $R_3$ et $R_4$ sont tels que définis précédemment, et $R'_1$ représente un groupement alkyl ($C_1$-$C_6$) linéaire ou ramifié,

composés de formule (I/b),
pouvant être soumis à l'action d'un agent alkylant tel qu'un sulfate de dialkyle, d'un agent acylant tel que l'anhydride acétique ou traité dans des conditions de réaction de Friedel et Craft, pour conduire aux composés de formule (I/c), cas particulier des composés de formule (I) :

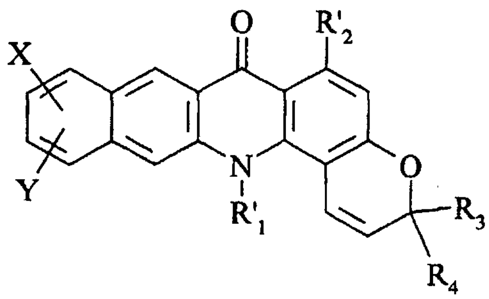

(I/c)

dans laquelle X, Y, $R'_1$, $R_3$ et $R_4$ sont tels que définis précédemment et $R'_2$ représente un groupement alkoxy éventuellement substitué par un groupement de formule $NR_7R_8$ tel que défini dans la formule (I), ou alkylcarbonyloxy ($C_1$-$C_6$) linéaire ou ramifié,

composé de formule (I/c),
pouvant être éventuellement traité, dans le cas où $R'_2$ représente un groupement alkoxy par exemple, par un dérivé amino de formule (VIII) :

$$HNR_aR_b \qquad (VIII)$$

dans laquelle $R_a$ représente un atome d'hydrogène ou un groupement alkyle ($C_1$-$C_6$) linéaire ou ramifié et $R_b$ représente un groupement alkyle, un groupement $R_9$-$NR_7R_8$ (dans lequel $R_9$ représente un groupement alkylène ($C_1$-$C_6$) linéaire ou ramifié, et $R_7$, $R_8$, identiques ou différents représentent un atome d'hydrogène, un groupement alkyle ($C_1$-$C_6$) linéaire ou ramifié, ou un groupement hydroxyalkyle ($C_1$-$C_6$) linéaire ou ramifié), un groupement hétérocycloalkylène (les termes alkylène et hétérocycle ayant la même signification que dans la formule (I)), ou un groupement

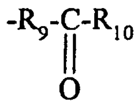

dans lequel $R_9$ et $R_{10}$ ont la même définition que dans la formule (I),
pour conduire aux composés de formule (I/d), cas particulier des composés de formule (I)

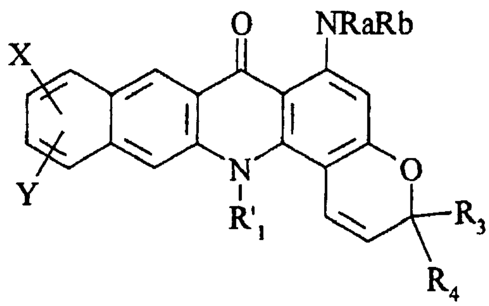

(I/d)

dans laquelle X, Y, $R'_1$, $R_3$,$R_4$, Ra et Rb sont tels que définis précédemment,
l'ensemble des composés de formule (I/a) à (I/d) formant le composé de formule (I/e) :

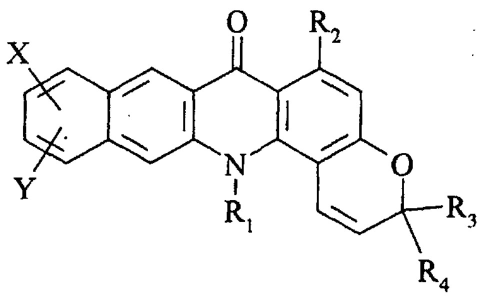

(I/e)

dans laquelle X, Y, $R_1$, $R_2$, $R_3$ et $R_4$ ont la même signification que dans la définition générale de la formule (I),

composé de formule (I/e) pouvant être soumis,

a) ⇨ soit à l'action d'un agent réducteur, pour conduire aux composés de formule (I/f), cas particulier des composés de formule (I):

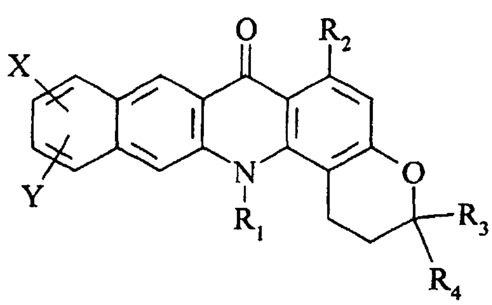

(I/f)

dans laquelle X, Y, $R_1$, $R_2$, $R_3$ et $R_4$ sont tels que définis précédemment,

b) ⇨ soit à l'action du tétroxyde d'osmium en milieu polaire et en présence de 4-Méthylmorpholine-N-oxyde, pour conduire aux composés de formules (I/g) et (I/g'), cas particuliers des composés de formule (I):

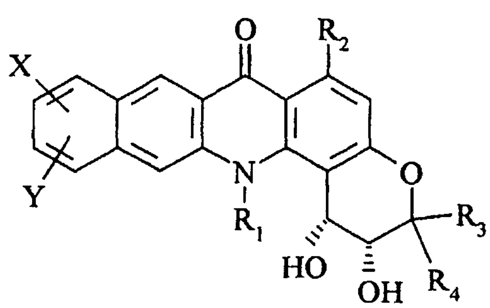

(I/g)

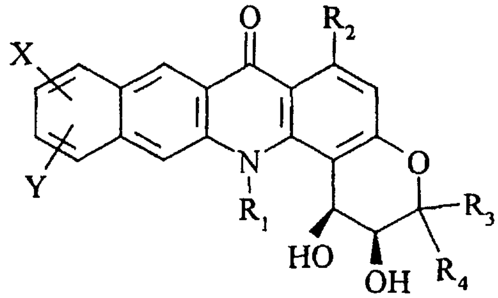

(I/g')

dans lesquelles X, Y, $R_1$, $R_2$, $R_3$ et $R_4$ sont tels que définis précédemment,
les composés de formule (I/g) et (I/g') pouvant aussi être obtenus séparément par synthèse chirale et notamment par *cis* dihydroxylation asymétrique à partir du composé (I/e) en utilisant des ligands chiraux du type pyridine ou phtalazine bisubstitués par des alcaloïdes de *Cinchona* comme la dihydroquinine et son diastéréoisomère dextrogyre la dihydroquinidine,
l'ensemble des composés de formules (I/g) et (I/g') formant les composés *cis*-diol de formule (*cis*-I/h) :

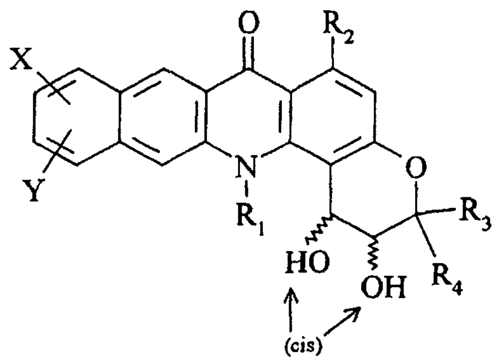

(cis-I/h)

dans laquelle X, Y, $R_1$, $R_2$, $R_3$ et $R_4$ sont tels que définis précédemment,
composés *cis*-diol de formule (*cis*-I/h) que l'on soumet éventuellement à l'action du N,N'-carbonyldiimidazole ou du N,N'-thiocarbonyldiimidazole en présence de 2-butanone, pour conduire aux composés de formule (*cis*-I/i), cas particulier des composés de formule (I) :

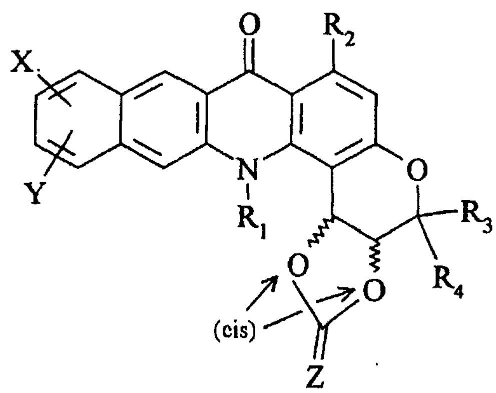

(cis I/i)

dans laquelle X, Y, $R_1$, $R_2$, $R_3$ et $R_4$ sont tels que définis précédemment, et Z représente un atome d'oxygène ou un atome de soufre,

c) ⇨ soit à l'action du permanganate de potassium en milieu polaire, pour conduire aux composés de formule (IX) :

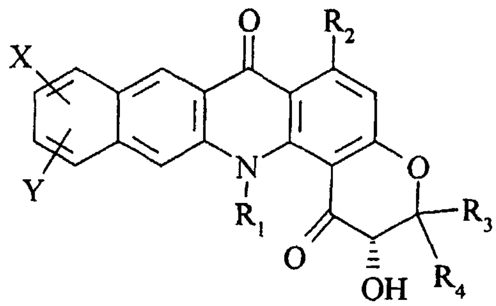

(IX)

dans laquelle X, Y, $R_1$, $R_2$, $R_3$ et $R_4$ sont tels que définis précédemment,

que l'on soumet à des conditions réductrices en présence de $NaBH_4$ par exemple, pour conduire aux composés de formule (I/j), cas particulier des composés de formule (I) :

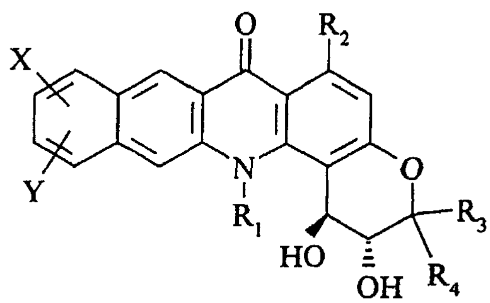

(I/j)

dans laquelle X, Y, $R_1$, $R_2$, $R_3$ et $R_4$ sont tels que définis précédemment,
l'ensemble des composés de formules (I/j) et (*cis*-I/h) formant les composés de formule (I/k)

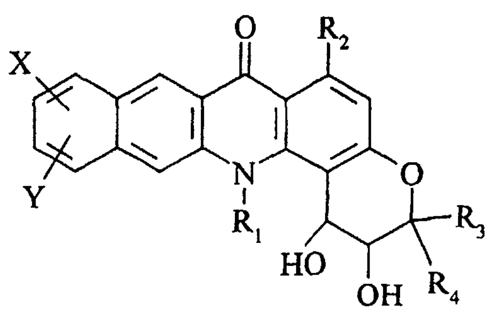

(I/k)

dans laquelle X, Y, $R_1$, $R_2$, $R_3$ et $R_4$ sont tels que définis précédemment,

composés de formule (I/k) que l'on soumet :

- soit à l'action d'un composé de formule (X) ou (XI) :

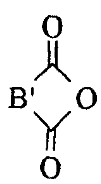

(X)

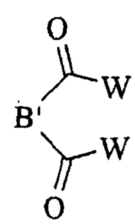

(XI)

dans lesquelles B' est tel que défini dans la formule (I), et W représente un atome d'halogène ou un groupement hydroxyle,
pour conduire aux composés de formule (I/l) :

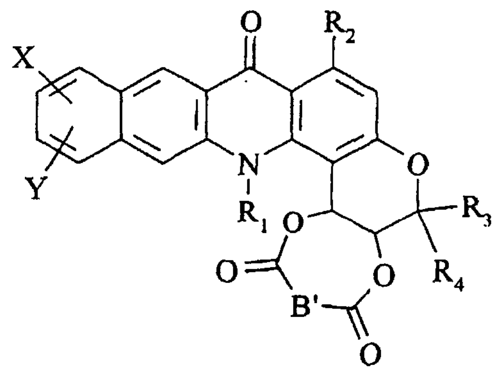

(I/l)

cas particulier des composés de formule (I), dans laquelle B', X, Y, $R_1$, $R_2$, $R_3$ et $R_4$ sont tels que définis précédemment,

- soit à l'action d'un dihalogénure d'alkyle ($C_1$-$C_6$) linéaire, pour conduire aux composés de formule (I/m), cas particulier des composés de formule (I) :

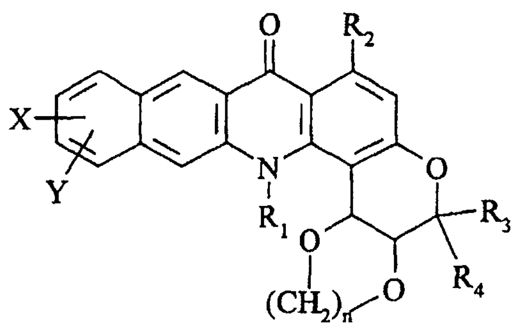

(I/m)

dans laquelle X, Y, $R_1$, $R_2$, $R_3$, $R_4$ et n sont tels que définis précédemment,

- soit à l'action d'un alcool de formule $R_{11}$-OH dans laquelle $R_{11}$ représente un groupement alkyle ($C_1$-$C_6$) linéaire ou ramifié, pour conduire aux composés de formule (I/n) :

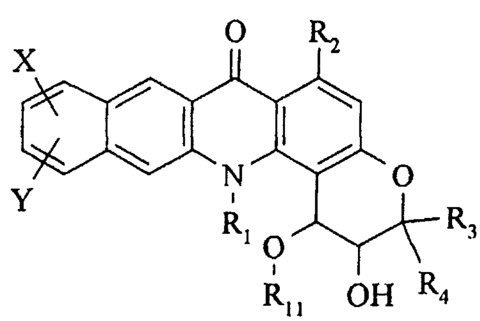

(I/n)

cas particulier des composés de formule (I), dans laquelle X, Y, $R_1$, $R_2$, $R_3$, $R_4$ et $R_{11}$ sont tels que définis précédemment,
composés de formule (I/n) dont la fonction alcool est estérifiée en présence d'une base faible, telle que la pyridine, par l'anhydride de formule $(R_{12}CO)_2O$, dans laquelle $R_{12}$ représente un groupement alkyle ($C_1$-$C_6$) linéaire ou ramifié ou un groupement aryle tel que défini précédemment,

pour conduire aux composés de formule (I/o), cas particulier des composés de formule (I) :

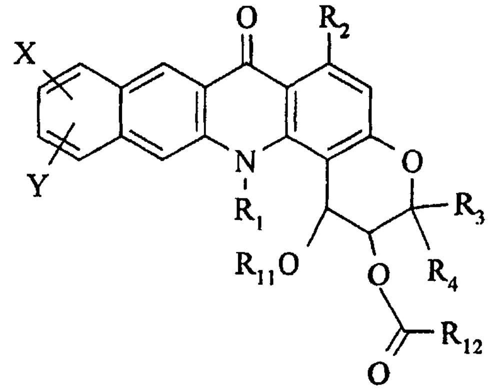

(I/o)

dans laquelle X, Y, $R_1$, $R_2$, $R_3$, $R_4$, $R_{11}$ et $R_{12}$ sont tels que définis précédemment,

❖ soit à l'action d'un iodure d'alkyle de formule $R'_{11}$-I dans laquelle $R'_{11}$ représente un groupement alkyle ($C_1$-$C_6$) linéaire ou ramifié, en présence de sel d'Argent, pour conduire aux composés de formule (I/p):

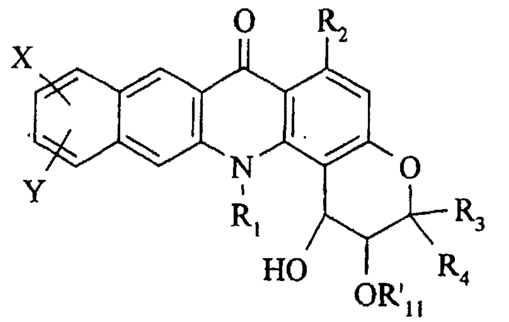

(I/p)

cas particulier des composés de formule (I), dans laquelle X, Y, $R_1$, $R_2$, $R_3$, $R_4$ et $R'_{11}$ sont tels que définis précédemment,
composés de formule (I/p) dont la fonction alcool est estérifiée par un anhydride de formule $(R'_{12}CO_2)O$ dans laquelle $R'_{12}$ représente un groupement alkyle ($C_1$-$C_6$) linéaire ou ramifié, ou un groupement aryle tel que défini précédemment, pour conduire aux composés de formule (I/q) :

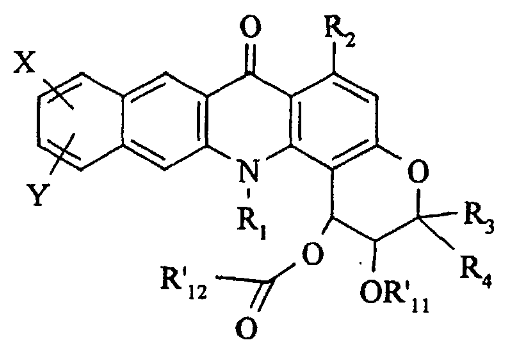

(I/q)

cas particulier des composés de formule (I), dans laquelle X, Y, $R_1$, $R_2$, $R_3$, $R_4$, $R'_{11}$ et $R'_{12}$ sont tels que définis précédemment,

❖ soit à l'action directe d'un anhydride de formule $(R_{12}CO_2)O$, en présence d'une base, telle que la triéthylamine, afin d'obtenir les composés de formule (I/r), cas particulier des composés de formule (I);

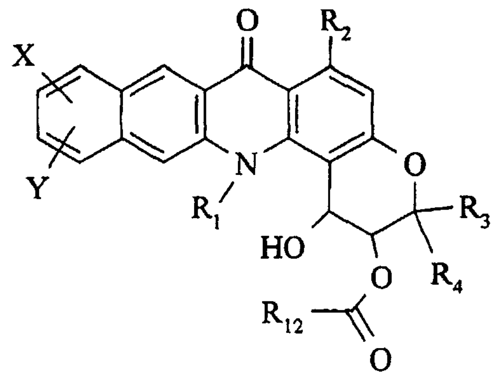

(I/r)

dans laquelle X, Y, $R_1$, $R_2$, $R_3$, $R_4$ et $R_{12}$ sont tels que définis précédemment, qui peut être soumis à nouveau, dans les mêmes conditions opératoires, à l'action de l'anhydride de formule $(R'_{12}CO_2)O$, pour conduire aux composés de formule (I/s):

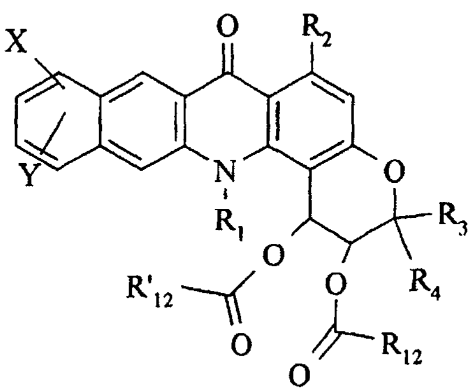

(I/s)

dans laquelle X, Y, $R_1$, $R_2$, $R_3$, $R_4$, $R_{12}$ et $R'_{12}$ sont tels que définis précédemment, les deux groupements $R_{12}$ et $R'_{12}$ pouvant être identiques ou différents,

❖ soit à des conditions de déhydratation en milieu acide pour conduire aux composés de formule (XII) :

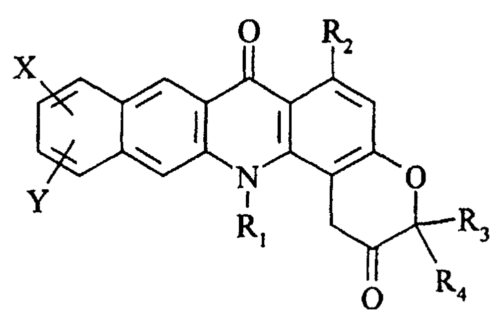

(XII)

dans laquelle X, Y, $R_1$, $R_2$, $R_3$ et $R_4$, sont tels que définis précédemment,
que l'on réduit en présence de $NaBH_4$ pour conduire aux composés de formule (I/t) :

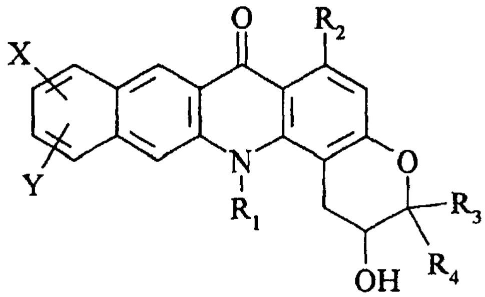

(I/t)

cas particulier des composés de formule (I), dans laquelle X, Y, $R_1$, $R_2$, $R_3$ et $R_4$, sont tels que définis précédemment,

d) ➪ soit à l'action d'un peracide, comme l'acide m-chloroperbenzoïque, pour conduire au composé de formule (I/u), cas particulier des composés de formule (I) :

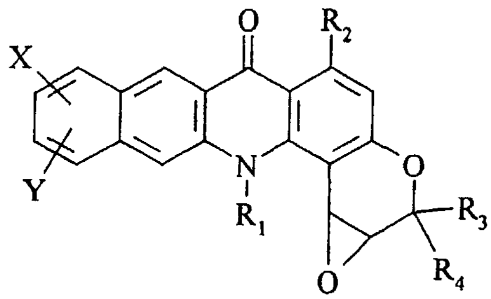

(I/u)

dans laquelle X, Y, $R_1$, $R_2$, $R_3$ et $R_4$, sont tels que définis précédemment,
composé de formule (I/u)

que l'on traite éventuellement par de l'ammoniac ou une amine primaire ou secondaire pour conduire aux composés de formule (I/v) et/ou (I/v') suivant la nature des réactifs, cas particulier des composés de formule (I):

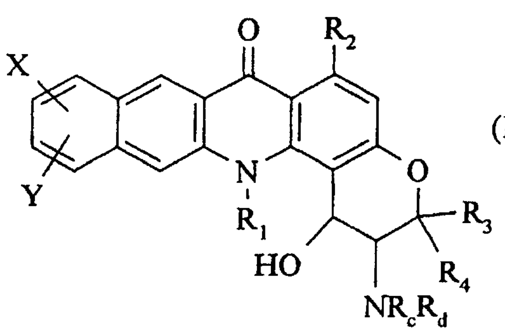

(I/v)

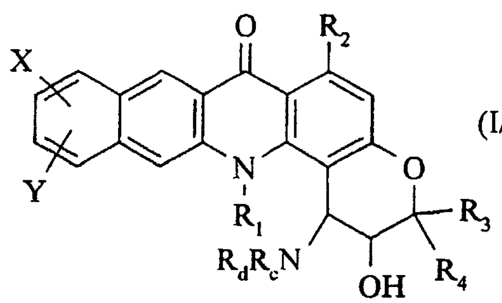

(I/v')

dans lesquelles X, Y, $R_1$, $R_2$, $R_3$ et $R_4$, sont tels que définis précédemment, et $R_c$ et $R_d$ représentent un atome d'hydrogène ou un groupement alkyle ($C_1$-$C_6$) linéaire ou ramifié,
composé de formule (I/v') pouvant également être obtenu directement à partir des composés de formule (I/k) par traitement de ceux-ci avec $NaN_3$ conduisant aux dérivés azide intermédiaires, suivi d'une hydrogénation en présence de palladium, conduisant alors aux composés de formule (I/v') dans laquelle Rc et Rd représentent un atome d'hydrogène,

<u>composé de formule (I/v) et (I/v')</u> pouvant être soumis :

- soit, dans le cas où Rc et Rd représentent un atome d'hydrogène, à l'action du N,N'-carbonyldiimidazole

ou du N,N'-thiocarbonyldiimidazole par exemple, pour conduire respectivement aux composés de formule (I/w) et (I/w'), cas particulier des composés de formule (I)

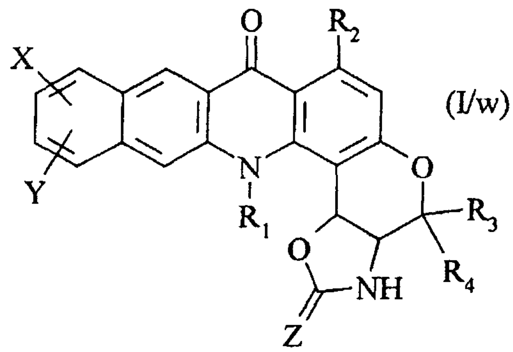
(I/w)

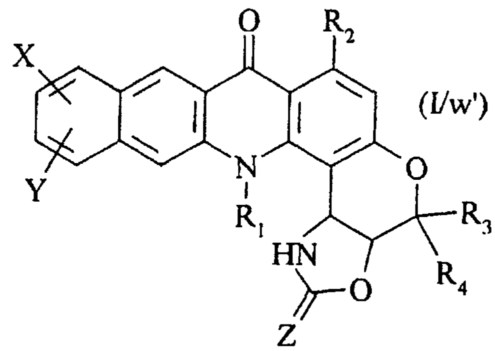
(I/w')

dans lesquelles X, Y, $R_1$, $R_2$, $R_3$ et $R_4$, sont tels que définis précédemment, et Z représente un atome d'oxygène ou un atome de soufre suivant la nature du réactif utilisé,

- soit à l'action d'un composé de formule (XI) $WO_2C\text{-}B'\text{-}CO_2W$ tel que défini précédemment, pour conduire respectivement au composé de formule (I/x) et (I/x'), cas particulier des composés de formule (I) :

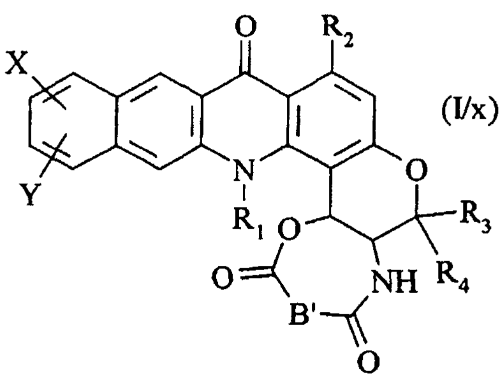
(I/x)

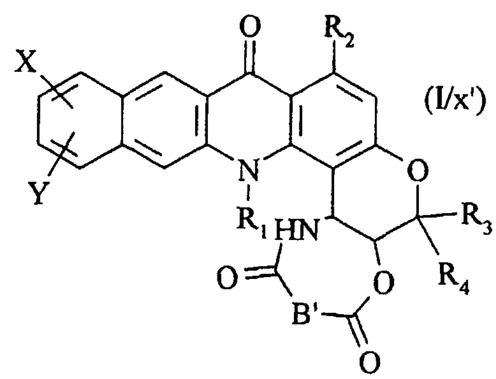
(I/x')

dans lesquelles B', X, Y, $R_1$, $R_2$, $R_3$ et $R_4$, sont tels que définis précédemment,

- soit à l'action du dibromure de triphénylphosphine en présence de triéthylamine pour conduire aux composés de formule (I/y), cas particulier des composés de formule (I)

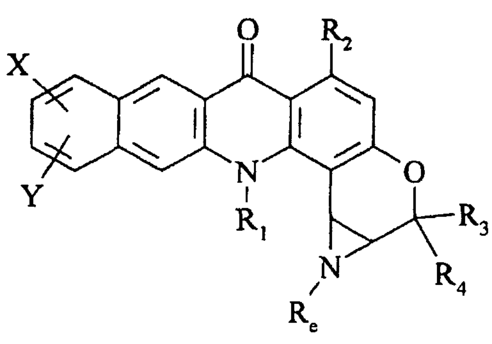
(I/y)

dans laquelle X, Y, $R_1$, $R_2$, $R_3$ et $R_4$, sont tels que définis précédemment, et $R_e$ représente un atome d'hydrogène ou un groupement alkyle ($C_1$-$C_6$)linéaire ou ramifié,

e) ⇨ soit à l'action de $NaN_3$ en présence d'eau oxygénée, suivi d'une étape de réduction par l'hydrure de tri-n-butylétain, par exemple, pour conduire aux composés de formule (I/z), cas particulier des composés de formule (I)

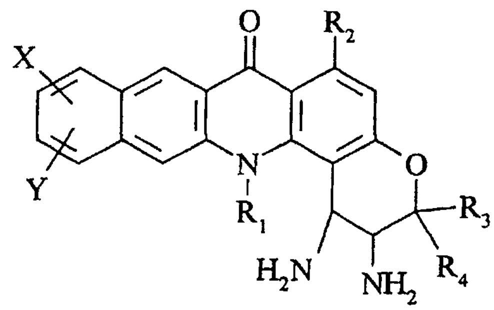

(I/z)

dans laquelle X, Y, $R_1$, $R_2$, $R_3$ et $R_4$ sont tels que définis précédemment, composés de formule (I/z) pouvant éventuellement être soumis à l'action du dioxyde de carbone, en présence de diphényl phosphite, pour conduire aux composés de formule (I/aa), cas particulier des composés de formule (I) :

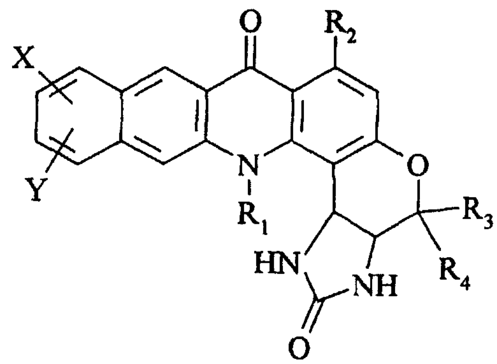

(I/aa)

dans laquelle X, Y, $R_1$, $R_2$, $R_3$ et $R_4$ sont tels que définis précédemment,

les composés (I/a-z), (I/g'), (I/v'-x'), (I/aa) et *(cis* I/h-I/i), forment l'ensemble des composés de l'invention que l'on purifie, le cas échéant, selon une technique classique de purification, qui peuvent, si on le désire, être séparés en leurs différents isomères optiques selon une technique classique de séparation et que l'on transforme, si on le souhaite, en leurs N-oxydes et, le cas échéant, en leurs sels d'addition à un acide ou à une base pharmaceutiquement acceptable.

Les composés de formule (II), (III), (VI) et (VII) sont soit des composés commerciaux, soit obtenus selon les méthodes classiques de synthèse organique et pour les composés de formule (VII) sont obtenus selon les conditions décrites dans *Chem. Ber*. 1978, 191, 439. La réaction de condensation entre les composés de formule (VI) et les composés de formule (VII) est notamment décrite dans la revue *Heterocycles,* 1992, 34(4), 799-806.

Les composés de formule (I) présentent des propriétés antitumorales particulièrement intéressantes. Ils ont un excellente cytotoxicité in vitro sur des lignées cellulaires, et une action sur le cycle cellulaire, et sont actifs in vivo. De plus, ces nouveaux composés se sont montrés beaucoup plus actifs et puissants que le composé de référence, l'acronycine. Ils possèdent, en outre, la propriété d'être solubles, permettant ainsi l'administration par voie intraveineuse. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses isomères optiques, N-oxydes ou un de ses sels d'addition à une base ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de

l'affection et la prise de traitements éventuels associés et s'échelonne de 0,5 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus.

Les structures des composés décrits dans les exemples et les préparations ont été déterminées selon les techniques spectrophotométriques usuelles (infra-rouge, résonance magnétique nucléaire,..).

**<u>Exemple 1</u> : 6-Hydroxy-3,3-diméthyl-7,14-dihydro-3H-benzo[b]pyrano[3,2-h] acridin-7-one.**

*<u>Stade A</u> : 1,3-Dihydroxy-5,12-dihydro-benzo[b]acridin-12-one*

A une solution de 5 g d'acide 3-amino-2-naphtalène carboxylique dans 50 ml d'heptane-1-ol sont additionnés 3,5 g de 1,3,5-trihydroxybenzène et 62,5 mg d'acide paratoluène sulfonique. Le mélange est maintenu sous agitation pendant 48 heures à reflux avec un Dean Stark, puis le mélange réactionnel est concentré sous vide. Le résidu est chromatographié sur gel de silice (éluant : cyclohexane/acétone : 90/10). Le produit isolé est cristallisé dans un mélange cyclohexane/acétone et permet d'obtenir 5,2 g du produit attendu.

*<u>Stade B</u> : 6-Hydroxy-3,3-diméthyl-7,14-dihydro-3H-benzo[b]pyrano[3,2-h]acridin-7-one*

A une solution de 2 g du produit du stade A dans 50 ml de diméhtylformamide anhydre, sous atmosphère inerte, sont additionnés 2 g de carbonate de potassium anhydre. Après 15 minutes d'agitation à 65 °C, 2,4 g d'iodure de potassium anhydre et 4,4 g de 3-chloro-3-méthyl-1-butyne sont ajoutés et le milieu réactionnel est maintenu 24 heures à 65 °C puis 1 heure 30 à 130 °C. Après refroidissement, la solution est hydrolysée puis extraite au dichlorométhane. Les phases organiques rassemblées sont lavées à l'eau, puis par une solution de potasse 1M, séchées sur sulfate de sodium, puis évaporées. Après chromatographie sur gel de silice (cyclohexane/acétone : 90/10), 1,10 g du produit attentu sont isolés.

**Point de fusion : 225 °C**

**<u>Exemple 2</u> : 6-Méthoxy-3,3,14-triméthyl-7,14-dihydro-3H-benzo[b]pyrano [3,2-h]acridin-7-one**

*<u>Stade C</u>*

A une solution de 0,5 g de produit de l'exemple 1 dans 20 ml de diméthylformamide anhydre, sont additonnés lentement, à 0 °C sous atmosphère inerte, 0,16 g d'hydrure de sodium, puis après 15 minutes 0,65 ml de diméthylsulfate (6 équivalents). Après 1 heures, le milieu réactionnel est hydrolysé par de la glace puis extrait à l'acétate d'éthyle. Après lavage de la phase organique par une solution aqueuse de soude, celle-ci est séchée sur sulfate de sodium, puis évaporée sous vide. Une chromatographie sur gel de silice (cyclohexane/acétone : 98/2) permet d'isoler 0,42 g du produit attendu.

**Point de fusion : 188 °C.**

**<u>Exemple 3</u> : 6-Hydroxy-3,3,14-triméthyl-7,14-dihydro-3H-benzo[b]pyrano [3,2-h]acridin-7-one**

On procède comme dans le stade C de l'exemple 2 en n'utilisant que 1,5 équivalents d'hydrure de sodium et 2 équivalents de diméthylsulfate.

**Point de fusion : 138 °C.**

**<u>Exemple 4</u> : (±) *cis*-1,2-Dihydroxy-6-méthoxy-3,3,14-triméthyl-2,3,7,14-tétrahydro-1H-benzo[b]pyrano[3,2-h] acridin-7-one**

A une solution de 2 g du produit de l'exemple 2 et de 0,9 g de 4-méthylmorpholine-N-oxyde monohydrate dans 40 ml d'un mélange t-butanol/tétrahydrofurane/eau (10/3/1) est additionné 2,5 % de tétroxyde d'osmium en solution dans 3,8 ml de 2-méthyl-2-propanol.

Après 2 jours à température ambiante, 105 ml d'une solution saturée en $NaHSO_3$ sont ajoutés et le milieu réactionnel est maintenu 1 heure sous agitation, puis extrait au dichlorométhane. Les phases organiques rassemblées sont séchées sur sulfate de sodium et concentrées sous vide. Une chromatographie sur gel de silice (dichlorométhane/méthanol : 95/5) permet d'isoler 1,3 g du produit attendu.

**Point de fusion : 194 °C.**

**Exemple 5 : (±)-*cis*-1,2-Diacétoxy-6-méthoxy-3,3,14-triméthyl-2,3,7,14-tétrahydro-1H-benzo[b]pyrano[3,2-h] acridin-7-one**

A une solution refroidie de 0,82 ml de pyridine et 0,82 ml d'anhydride acétique est ajoutée 0,33 g du produit de l'exemple 4. Après 6 jours à température ambiante, le milieu réactionnel est jeté sur de la glace. Il se forme un précipité qui est filtré, lavé par de l'eau puis séché sous vide. On obtient 0,36 g du produit attendu.
**Point de fusion : 163 °C.**

**Exemple 6 : (±)-*cis*-1,2-Dibenzoyloxy-6-méthoxy-3,3,14-triméthyl-2,3,7,14-tétrahydro-1H-benzo[b]pyrano [3,2-h]acridin-7-one**

On procède comme dans l'exemple 5 en utilisant l'anhydride benzoïque comme réactif.

**Exemple 7 : *cis*-7-Méthoxy-4,4,15-triméthyl-3a,8,15,15c-tétrahydro-4H-benzo[b][1,3] dioxolo [4',5':4,5] pyrano [3,2-h]acridin-2,8-dione**

A une solution de 0,12 g du produit de l'exemple 4 dans 5 ml de 2-butanone est additionné 0,24 g de N,N'-carbonyldiimidazole. Arpès 3 heures à reflux sous argon, le milieu réactionnel est hydrolysé par une solution aqueuse à 5 % de carbonate de sodium, puis extrait à l'acétate d'éthyle. Les phases organiques rassemblées sont séchées sur sulfate de sodium puis concentrées sous vide. Une chromatographie sur gel de silice (dichlorométhane/acétone : 2/1) permet d'isoler 0,75 g du produit attendu.
**Point de fusion : 176 °C.**

**Exemple 8: 6-(Diméthylaminoéthylamino)-3,3,14-triméthyl-7,14-dihydro-3H-benzo[b]pyrano[3,2-h]acridin-7-one**

A 0,15 g du produit de l'exemple 2 est additionné 4 ml de N,N-diméthylethylènediamine. Après 5 jours de réaction à 70 °C sous atmosphère inerte, le milieu réactionnel est évaporé sous pression réduite. Le résidu obtenu est chromatographié sur gel de silice (cyclohexane/acétate d'éthyle : 80/20) permettant d'isoler le produit attendu.
**Point de fusion : huile.**
**Spectre de masse : (DIC/$NH_3$) : m/z : 428 $(M+H)^+$**

**Exemple 9: 6-(Diméthylaminopropylamino)-3,3,14-triméthyl-7,14-dihydro-3H-benzo[b]pyrano[3,2-h]acridin-7-one**

On procède comme dans l'exemple 8 en utilisant comme réactif la N,N-diméthylpropyldiamine.
**Point de fusion : huile.**
**Spectre de masse : (DIC/$NH_3$) : m/z : 442 $(M+H)^+$**

**Exemple 10 : 6-(Diéthylaminopropylamino)-3,3,14-triméthyl-7,14-dihydro-3H-benzo[b]pyrano[3,2-h]acridin-7-one**

On procède comme dans l'exemple 8 en utilisant comme réactif la N,N-diétylpropyldiamine.
**Point de fusion : huile.**
**Spectre de masse : (DIC/$NH_3$) : m/z : 470 $(M+H)^+$**

**Exemple 11 : 6-[(3-Morpholine-4-yl)propylaminol-3,3,14-triméthyl-7,14-dihydro-3H-benzo[b]pyrano[3,2-h]acridin-7-one**

On procède comme dans l'exemple 8 en utilisant comme réactif la 4-(3-aminopropyl) morpholine.
**Spectre de masse : (DIC/$NH_3$) : m/z : 484 $(M+H)^+$**

**Exemple 12 : 6-[(2-Morpholine-4-yl)éthylamino]-3,3,14-triméthyl-7,14-dihydro-3H-benzo[b]pyrano[3,2-h]acridin-7-one**

On procède comme dans l'exemple 8 en utilisant comme réactif la 4-(2-aminoéthyl) morpholine.
**Spectre de masse : (DIC/$NH_3$): m/z : 470 $(M+H)^+$**

**Exemple 13 : 6-[(2-Pipéridine-1-yl)éthylamino]-3,3,14-triméthyl-7,14-dihydro-3H-benzo[b]pyrano[3,2-h]acridin-7-one**

On procède comme dans l'exemple 8 en utilisant comme réactif la 1-(2-aminoéthyl) pipéridine.
**Spectre de masse : (DIC/$NH_3$) : m/z : 468 $(M+H)^+$**

**Exemple 14 : (±)-*trans*-1,2-Dihydroxy-6-méthoxy-3,3,14-triméthyl-2,3,7,14-tétrahydro-1H-benzo[b]pyrano [3,2-h]acridin-7-one**

*Stade D : 1-Oxo-2-hydroxy-6-méthoxy-3,3,14-triméthyl-2,3,7,14-tétrahydro-1H-benzo[b] pyrano[3,2-h]acridine- 7-one*

A une solution de 3,5 g du produit de l'exemple 2 dans 50 ml d'acétone sont additionnés lentement à température ambiante une solution de 8,1 g de permanganate de potassium dilué dans 30 ml d'eau. Après 2 heures, la solution est concentrée sous pression réduite. Le résidu est alors mélangé à de la silice puis chromatographié sur gel de silice (dichlorométhane/méthanol : 98/2), permettant d'isoler le composé de l'exemple 4 présent en faible quantité, du produit attendu.

*Stade E : (±)-trans-1,2-Dihydroxy-6-méthoxy-3,3,14-triméthyl-2,,7,14-tétrahydro-1H-benzo[b]pyrano[3,2-h]acridine-7-one*

A une solution d'un gramme du composé obtenu dans le stade D, dissous dans 30 ml de méthanol est additionné 0,45 g de $NaBH_4$. Après 1 heure de réaction à 0 °C, la réaction est ramenée à température ambiante, le solvant est évaporé et le résidu est chromatographié sur gel de silice (dichlorométhane/méthanol : 95/5) permettant d'isoler le produit désiré.

**Exemple 15 : (±)-*cis*-1,2-Dihydroxy-6-(diméthylaminoéthylamino)-3,3,14-triméthyl-2,3,7,14-tétrahydro-1H-benzo[b]pyrano[3,2-h]acridin-7-one**

On procède comme dans l'exemple 4 en utilisant comme substrat le composé de l'exemple 8.

**Exemple 16 : (±)-*cis*-9-Méthoxy-6,6,17-triméthyl-3,4,5a,10,17,17c-hexahydro-2H,6H-benzo [b][1,4]dioxepino[2',3':4,5]pyrano[3,2-h]acridin-2,4,10-trione**

On procède comme dans l'exemple 5 en utilisant comme substrat le composé de l'exemple 4 et comme réactif le dichlorure d'acylmalonique.

**Exemple 17 : *cis*-7-(Diméthylaminoéthylamino)-4,4,15-triméthyl-3a,8,15,15c-tétrahydro-4H-benzo[b] [1,3] dioxolo[4',5' : 4,5]pyrano[3,2-h]acridin-2,8-dione**

On procède comme dans l'exemple 7 en utilisant comme substrat le composé de l'exemple 15.

**Exemple 18 : (±)-*cis*-1,2-Dihydroxy-6-(diméthylaminopropylamino)-3,3,14-triméthyl-2,3,7,14-tétrahydro-1H-benzo[b] pyrano[3,2-h]acridin-7-one**

On procède comme dans l'exemple 4 en utilisant comme substrat le composé de l'exemple 10.

**Exemple 19 : (±)-*cis*-1,2-Diacétoxy-6-(diméthylaminopropylamino)-3,3,14-triméthyl-2,3,7,14-tétrahydro-1H-benzo[b]pyrano[3,2-h]acridin-7-one**

On procède comme dans l'exemple 5 en utilisant comme substrat le composé de l'exemple 18.

**Exemple 20 : (±)-*cis*-1-Hydroxy-2-benzoyloxy-6-méthoxy-3,3,14-triméthyl-2,3,7,14-tétrahydro-1H-benzo[b]pyrano[3,2-h]acridin-7-one**

A une solution de 0,2 g du composé de l'exemple 4 dans 3 ml de pyridine est ajouté 0,125 g d'anhydride benzoïque. Après 36 heures d'agitation à température ambiante, le milieu réactionnel est concentré sous pression réduite. Le résidu est chromatographié sur gel de silice (éluant : acétate d'éthyle/toluène : 70/30).

**Exemple 21 : (±)-*cis*-1-Acétoxy-2-benzoyloxy-6-méthoxy-3,3,14-triméthyl-2,3,7,14-tétrahydro-1H-benzo[b]pyrano[3,2-h]acridin-7-one.**

A une solution de 0,1 g du composé de l'exemple 20 dans 2,5 ml de pyridine sont additionnés 2,5 ml d'anhydride acétique. Après 12 heures de réaction à température ambiante, le mélange réactionnel est concentré sous pression réduite. Une chromatographie sur gel de silice (éluant : dichlorométhane) permet d'isoler le produit attendu.

**Exemple 22 : (±)-1-Amino-2-hydroxy-6-méthoxy-3,3,14-triméthyl-2,3,7,14-tétrahydro-1H-benzo[b]pyrano[3,2-h] acridin-7-one**

*Etape 1 : (±)-1-Azido-2-hydroxy-6-méthoxy-3,3,14-triméthyl-2,3,7,14-tétrahydro-1H-benzo[b]pyrano[3,2-h]acridin-7-one*

A une solution de 0,15 g du composé de l'exemple 4, et 0,5 g de $NaN_3$ dans 6 ml de chloroforme, sont additionnés lentement à température ambiante 3 ml d'acide trifluoroacétique. Après 12 heures d'agitation, 1 équivalent de $NaN_3$ est ajouté et la réaction est maintenue à température ambiante pendant encore 12 heures. Le mélange réactionnel est ensuite lavé à l'eau puis par une solution saturée en NaCl, séché sur sulfate de sodium. Une chromatographie sur gel de silice (dichlorométhane/méthanol: 95/5) permet d'isoler le produit attendu.

*Etape 2 : (±)-1-Amino-2-hydroxy-6-méthoxy-3,3,14-triméthyl-2,3,7,14-tétrahydro-1H-benzo[b]pyrano[3,2-h]acridin-7-one.*

Une solution contenant 0,2 g du composé de l'étape 1 et 0,09 g de Pd/C dans 5 ml d'éthanol est agitée à température ambiante sous atmosphère d'$H_2$ pendant 48 heures. Le catalyseur est ensuite filtré et le filtrat est concentré sous vide. Le résidu est chromatographié sur gel de silice (dichlorométhane/méthanol: 95/5) permettant d'isoler le produit désiré.

**Exemple 23 : 7-Méthoxy-4,4,15-triméthyl-1,3a,4,8,15,15c-hexahydro-2H-benzo[b] [1,3]oxazolo[4',5':4,5]pyrano [3,2-h]acridine-2,8-dione**

On procède comme dans l'exemple 7 en utilisant le composé de l'exemple 22 comme substrat.

**Exemple 24 : 6-(Diéthylaminoéthylamino)-3,3,14-triméthyl-7,14-dihydro-3H-benzo[b] pyrano[3,2-h]acridin-7-one**

On procède comme dans l'exemple 8 en utilisant comme réactif le N,N-Diéthylaminoéthylamine.
**Spectre de masse : (DIC/$NH_3$): m/z : 456 $(M+H)^+$**

**Exemple 25 : 6-(Diméthylaminoéthyloxy)-3,3,14-triméthyl-7,14-dihydro-3H-benzo[b] pyrano[3,2-h]acridin-7-one**

A une solution, sous azote, de 0,2 g du composé de l'exemple 3 dans 20 ml de diméthylformamide, sont ajoutés 1 équivalent d'hydrure de sodium et 1 équivalent d'hydrochlorure de 2-diméthylaminoéthyl-chlorure. Après 48 heures à 70°C, le milieu réactionnel est refroidi puis versé sur 80 ml d'eau glacée et extrait au dichlorométhane. Après lavage et séchage sur $MgSO_4$, la solution est évaporée sous pression réduite. Une chromatographie sur gel de silice (acétate d'éthyle/cyclohexane: 80/20) permet d'isoler le produit attendu.
**Spectre de masse : (DIC/$NH_3$) : m/z : 429 $(M+H)^+$**

**Exemple 26 : 6-(Diméthylaminopropyloxy)-3,3,14-triméthyl-7,14-dihydro-3H-benzo[b]pyrano[3,2-h]acridin-7-one**

On procède comme dans l'exemple 25 en utilisant comme réactif l'hydrochlorure de 3-chloro-N,N'-diméthylpropylamine.
**Spectre de masse : (DIC/$NH_3$) : m/z : 443 $(M+H)^+$**

**Exemple 27 : 6-(Diéthylaminoéthyloxy)-3,3,14-triméthyl-7,14-dihydro-3H-benzo[b] pyrano[3,2-h]acridin-7-one**

On procède comme dans l'exemple 25 en utilisant comme réactif l'hydrochlorure de 2-diéthylaminoéthylchlorure.
**Spectre de masse : (DIC/$NH_3$) : m/z : 457 $(M+H)^+$**

<u>Exemple 28</u> **: 6-[(2-Morpholine-4-yl)éthyloxy]-3,3,14-triméthyl-7,14-dihydro-3H-benzo[b]pyrano[3,2-h]acridin-7-one**

On procède comme dans l'exemple 25 en utilisant comme réactif l'hydrochlorure de 4-(2-chloroéthyl)-morpholine.
**Spectre de masse : (DIC/$NH_3$) : m/z : 471 $(M+H)^+$**

<u>Exemple 29</u> **: 6-[(Carbométhoxy)méthylamino]-3,3,14-triméthyl-7,14-dihydro-3H-benzo[b]pyrano[3,2-h]acridin-7-one**

Une solution contenant 200 mg du composé de l'exemple 2 et 10 équivalents d'hydrochlorure de méthyl-2-aminoacétate dans 15 ml de diméthylformamide est maintenue sous argon à 70°C. Après 3 jours, le milieu réactionnel est concentré sous pression réduite et une chromatographie sur gel de silice (acétate d'éthyle/cyciohexane: 85/15) du résidu permet d'isoler le produit attendu.
**Spectre de masse : (DIC/$NH_3$) : m/z : 429 $(M+H)^+$**

<u>Exemple 30</u> **: 6-[(Carboéthoxy)méthylamino]-3,3,14-triméthyl-7,14-dihydro-3H-benzo [b]pyrano[3,2-hlacridin-7-one**

On procède comme dans l'exemple 29 en utilisant comme réactif l'éthyl-2-aminoacétate.
**Spectre de masse : (DIC/$NH_3$) : m/z : 443 $(M+H)^+$**

<u>Exemple 31</u> **: 6-[(Carbométhoxy)propylamino]-3,3,14-triméthyl-7,14-dihydro-3H-benzo[b]pyrano[3,2-h]acridin-7-one**

On procède comme dans l'exemple 29 en utilisant comme réactif l'acide 4-aminobutyrique, puis on soumet le produit ainsi obtenu à des conditions d'estérification en présence de méthylate de sodium.
**Spectre de masse : (DIC/$NH_3$) : m/z : 457 $(M+H)^+$**

<u>Exemple 32</u> **: 6-[(Carbométhoxy)butylamino]-3,3,14-triméthyl-7,14-dihydro-3H-benzo [b]pyrano[3,2-h]acridin-7-one**

On procède comme dans l'exemple 31 en utilisant comme réactif premier l'acide 5-aminovalérique.

<u>Exemple 33</u> **: *cis*-6-(Diméthylaminopropylamino)-1,2-dihydroxy-3,3,14-triméthyl-2,3,7,14-tétrahydro-1H-benzo [b]pyrano[3,2-h]acridin-7-one**

On procède comme dans l'exemple 4 en utilisant comme substrat le composé de l'exemple 9.

<u>Exemple 34</u> **: *cis*-6-[(2-Morpholin-4-yl)éthylamino]-1,2-dihydroxy-3,3,14-triméthyl-2,3,7,14-tétrahydro-1H-benzo[b]pyrano[3,2-h]acridin-7-one**

On procède comme dans l'exemple 4 en utilisant comme substrat le composé de l'exemple 12.

<u>Exemple 35</u> **: *cis*-6-[(2-Pipéridin-1-yl)éthylamino]-1,2-dihydroxy-3,3,14-triméthyl-2,3,7,14-tétrahydro-1H-benzo [b]pyrano[3,2-h]acridin-7-one**

On procède comme dans l'exemple 4 en utilisant comme substrat le composé de l'exemple 13.

<u>Exemple 36</u> **: *trans*-6-(Diméthylaminoéthylamino)-1,2-dihydroxy-3,3,14-triméthyl-2,3,7,14-tétrahydro-1H-benzo [b]pyrano[3,2-h]acridin-7-one**

On procède comme dans l'exemple 14 des stades D à E en utilisant comme substrat le composé de l'exemple 8.

Exemple 37 : *cis*-6-(Diméthylaminoéthyloxy)-1,2-dihydroxy-3,3,14-triméthyl-2,3,7,14-tétrahydro-1H-benzo[b]pyrano[3,2-h]acridin-7-one

On procède comme dans l'exemple 4 en utilisant comme substrat le composé de l'exemple 25.

Exemple 38 : *cis*-6-(Diméthylaminopropyloxy)-1,2-dihydroxy-3,3,14-triméthyl-2,3,7,14-tétrahydro-1H-benzo[b]pyrano[3,2-h]acridin-7-one

On procède comme dans l'exemple 4 en utilisant comme substrat le composé de l'exemple 26.

Exemple 39 : *cis*-6-[(2-Morpholine-4-yl)éthyloxy]-1,2-dihydroxy-3,3,14-triméthyl-2,3,7,14-tétrahydro-1H-benzo[b]pyrano[3,2-h]acridin-7-one

On procède comme dans l'exemple 4 en utilisant comme substrat le composé de l'exemple 28.

Exemple 40: *cis*-6-[(Carbométhoxy)méthylamino]-1,2-dihydroxy-3,3,14-triméthyl-2,3,7,14-tétrahydro-1H-benzo[b]pyrano[3,2-h]acridin-7-one

On procède comme dans l'exemple 4 en utilisant comme substrat le composé de l'exemple 29.

Exemple 41: *cis*-6-[(Carbométhoxy)propylamino]-1,2-dihydroxy-3,3,14-triméthyl-2,3,7,14-tétrahydro-1H-benzo[b]pyrano[3,2-h]acridin-7-one

On procède comme dans l'exemple 4 en utilisant comme substrat le composé de l'exemple 31.

Exemple 42 : *cis*-1,2-Diacétoxy-6-(diméthylaminopropylamino)-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 33.

Exemple 43 : *cis*-1,2-Diacétoxy-6-[(2-morpholine-4-yl)éthylamino]-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 34.

Exemple 44 : (*cis*)-1,2-Diacétoxy-6-[(2-pipéridine-1-yl)éthylamino-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 35.

Exemple 45 : (*trans*)-1,2-Diacétoxy-6-(diméthylaminoéthylamino)-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 36.

Exemple 46 : *cis*-1,2-Diacétoxy-6-(diméthylaminoéthyloxy)-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 37.

Exemple 47: *cis*-1,2-Diacétoxy-6-(diméthylaminopropyloxy)-3,3,14-triméthyl-2,3,7,14-tétrahydro-1H-benzo[b]pyrano[3,2-h]acridin-7-one

On procède comme dans l'exemple 5 en utilisant comme substrat le composé de l'exemple 38.

**Exemple 48 : *cis*-1,2-Diacétoxy-6-[(carbométhoxy)méthylamino]-3,3,14-triméthyl-2,3,7,14-tétrahydro-1H-benzo[b]pyrano[3,2-h]acridin-7-one**

On procède comme dans l'exemple 5 en utilisant comme substrat le composé de l'exemple 40.

**Exemple 49 : *cis*-1,2-Diacétoxy-6[(carbométhoxy)propylamino]-3,3,14-triméthyl-2,3,7,14-tétrahdyro-1H-benzo [b]pyrano[3,2-h]acridin-7-one**

On procède comme dans l'exemple 5 en utilisant comme substrat le composé de l'exemple 41.

**Exemple 50 : *cis*-7-(Diméthylaminopropylamino)-4,4,15-triméthyl-3a,8,15,15c-tétrahydro-4H-benzo[b][1,3] dioxolo-[4',5':4,5]pyrano[3,2-h]acridin-2,8**-dione

On procède comme dans l'exemple 7 en utilisant comme substrat le composé de l'exemple 33.

**Exemple 51 : *cis*-6-[(2-Morpholine-4-yl)éthylamino]-4,4,15-triméthyl-3a,8,15,15c-tétrahydro-4H-benzo[b][1,3] dioxolo[4',5':4,5]pyrano[3,2-h]acridin-2,8**-dione

On procède comme dans l'exemple 7 en utilisant comme substrat le composé de l'exemple 34.

**Exemple 52 : *cis*-6-[(2-Pipéridine-1-yl)éthylamino]-4,4,15-triméthyl-3a,8,15,15c-tétrahydro-4H-benzo[b][1,3] dioxolo[4',5':4,5]pyrano[3,2-h]acridin-2,8-dione**

On procède comme dans l'exemple 7 en utilisant comme substrat le composé de l'exemple 35.

**Exemple 53 : *cis*-6-(Diméthylaminoéthyloxy)-4,4,15-triméthyl-3a,8,15,15c-tétrahydro-4H-benzo[b][1,3]dioxolo [4',5':4,5]pyrano[3,2-h]acridin-2,8-dione**

On procède comme dans l'exemple 7 en utilisant comme substrat le composé de l'exemple 37.

**Exemple 54 : *cis*-6-[(Carbométhoxy)propylamino]-4,4,15-triméthyl-3a,8,15,15c-tétrahydro-4H-benzo[b][1,3] dioxolo[4',5':4,5]pyrano[3,2-h]acridin-2,8-dione**

On procède comme dans l'exemple 7 en utilisant comme substrat le composé de l'exemple 41.

**Exemple 55 : *cis*-7-Méthoxy-4,4,15-triméthyl-2-thioxo-3a,8,15,15c-tétrahydro-4H-benzo[b][1,3]dioxolo[4',5':4,5]pyrano[3,2-h]acridin-8-one**

On procède comme dans l'exemple 7 en utilisant comme réactif le N,N'-thiocarbonyldiimidazole.

**Exemple 56 : *cis*-7-(Diméthylaminoéthylamino)-4,4,15-triméthyl-2-thioxo-3a,8,15,15c-tétrahydro-4H-benzo[b] [1,3]dioxolo[4',5':4,5]pyrano[3,2-h]acridin-8-one**

On procède comme dans l'exemple 55 en utilisant comme substrat dans la première étape le composé de l'exemple 15.

**Exemple 57 : 1,2-diamino-6-méthoxy-3,3,14-triméthyl-2,3,7,14-tétrahydro-1H-benzo[b]pyrano[3,2-h]acridin-7-one**

Le produit est isolé par chromatographie sur gel de silice à partir de l'exemple 22 où il se forme comme co-produit lors de la synthèse de ce composé.

**Exemple 58 :7-Méthoxy-4,4,15-triméthyl-1,2,3,3a,4,8,15,15c-octahydro-benzo[b] imidazo[4',5':4,5]pyrano [3,2-h]acridin-2,8-dione**

Le composé de l'exemple 57 est traité selon les conditions décrites dans *Tetrahedron. Lett.* 1974, 1191.

**<u>Exemple 59</u> : 7-Méthoxy-4,4,15-triméthyl-2-thioxo-1,3a,4,8,15,15c-hexahydro-2H-benzo[b][1,3]oxazolo[4',5':4,5]pyrano[3,2-h]acridin-8-one**

On procède comme dans l'exemple 55 en utilisant comme substrat le composé de l'exemple 22.

**<u>Exemple 60</u> : 9-Méthoxy-6,6,17-triméthyl-1,3,4,5a,6,10,17,17c-octahydro-2H-benzo[b] [1,4]oxazepino[3',2':4,5] pyrano[3,2-h]acridin-2,4,10-trione**

On procède comme dans l'exemple 16 en utilisant comme substrat le composé de l'exemple 22.

**<u>Exemple 61</u> : 10,11-Dichloro-6-hydroxy-3,3-diméthyl-7,14-dihydro-3H-benzo[b] pyrano[3,2-h]acridin-7-one**

On procède comme dans l'exemple 1 des stades A à B en utilisant comme substrat au stade A l'acide 3-amino-6,7-dichloro-2-naphtalène carboxylique.

**<u>Exemple 62</u> : 10,11-Dichloro-6-méthoxy-3,3,14-triméthyl-7,14-dihydro-3H-benzo [b]pyrano[3,2-h]acridin-7-one**

On procède comme dans l'exemple 2 en utilisant comme substrat le composé de l'exemple 61.

**<u>Exemple 63</u> : *cis*-10,11-Dichloro-1,2-dihydroxy-6-méthoxy-3,3,14-triméthyl-2,3,7,14-tétrahydro-1H-benzo[b]pyrano[3,2-h]acridin-7-one**

On procède comme dans l'exemple 4 en utilisant comme substrat le composé de l'exemple 62.

**<u>Exemple 64</u>** : **6-Hydroxy-9,12-diméthoxy-3,3-diméthyl-7,14-dihydro-3H-benzo [b]pyrano[3,2-h]acridin-7-one**

On procède comme dans l'exemple 1 des stades A à B en utilisant comme substrat au stade A l'acide 3-amino-5,8-diméthoxy-naphtalène carboxylique.

**<u>Exemple 65</u>: 6,9,12-triméthoxy-3,3,14-triméthyl-7,14-dihydro-3H-benzo[b]pyrano [3,2-h]acridin-7-one**

On procède comme dans l'exemple 2 en utilisant comme substrat le composé de l'exemple 64.

**<u>Exemple 66</u> : 10,11-Di-*tert*-butyl-6-hydroxy-3,3-diméthyl-7,14-dihydro-3H-benzo[b]pyrano[3,2-h]acridin-7-one**

On procède comme dans l'exemple 1 des stades A à B en utilisant comme substrat au stade A l'acide 3-amino-6,7-di-*tert*-butyl-naphtalène carboxylique.

**<u>Exemple 67</u> : 10,11-Di-*tert*-butyl-6-méthoxy-3,3,14-triméthyl-7,14-dihydro-3H-benzo[b]pyrano[3,2-h]acridin-7-one**

On procède comme dans l'exemple 2 en utilisant comme substrat le composé de l'exemple 66.

**<u>Exemple 68</u>: *cis*-10,11-Di-*tert*-butyl-1,2-dihydroxy-6-méthoxy-3,3,14-triméthyl-2,3,7,14-tétrahydro-IH-benzo[b] pyrano[3,2-h]acridin-7-one**

On procède comme dans l'exemple 4 en utilisant comme substrat le composé de l'exemple 67.

**<u>Exemple 69</u>: *cis*-1,2-Diacétoxy-10,11-di-*tert*-butyl-6-méthoxy-3,3,14-triméthyl-2,3,7,14-tétrahydro-1H-benzo[b] pyrano[3,2-h]acridin-7-one**

On procède comme dans l'exemple 5 en utilisant comme substrat le composé de l'exemple 68.

**<u>Exemple 70</u>: 10,11-Di-*tert*-butyl-6-(diméthylaminoéthylamino)-3,3,14-triméthyl-7,14-dihydro-3H-benzo[b]pyrano[3,2-h]acridin-7-one**

On procède comme dans l'exemple 8 en utilisant comme substrat le composé de l'exemple 67.

## ETUDE PHARMACOLOGIQUE DES COMPOSES DE L'INVENTION

### Exemple 71 : Activité in vitro

La leucémie murine L1210 a été utilisée in vitro. Les cellules sont cultivées dans du milieu de culture RPMI 1640 complet contenant 10 % de serum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res., 47, 936-942, (1987)). Les résultats sont exprimés en $IC_{50}$, concentration en cytotoxique qui inhibe à 50 % la prolifération des cellules traitées. Les résultats obtenus sont rassemblés dans le tableau 1.

Tableau 1

| Cytotoxicité pour les cellules L 1210 en culture | |
|---|---|
| **Produits** | **Cytotoxicité $IC_{50}$ (µM)** |
| Exemple 7 | 0,023 |
| Acronycine | 27,0 |

Tous les produits de l'invention sont plus puissants que le composé de référence constitué par l'acronycine.

### Exemple 72 : Activité in vivo

***1- Activité antitumorale sur la lignée P 388.***

La lignée P 388 (leucémie murine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Six souris de 18 à 20 g ont été utilisées par groupe expérimental. Les produits ont été administrés par voie intrapéritonéale au jour 1.
L'activité antitumorale est exprimée en % de T/C:

$$T/C\ \%\ (\text{survie}) = \frac{\text{Temps de survie médian des animaux traités}}{\text{Temps de survie médian des animaux contrôles}} \times 100$$

Le tableau 2 indique l'activité antitumorale obtenue aux doses optimales.

Tableau 2

| Activité antitumorale sur la lignée P 388 | | | | |
|---|---|---|---|---|
| **Produit** | **Schéma** | **Voie** | **Dose optimale (mg/kg)** | **T/C %** |
| Exemple 7 | J1 | i.p. | 12,5 | 327 |
| Acronycine | J1 | i.p. | 200 | 125 |

Le produit de l'exemple 7 est très actif dans ce modèle, alors que l'acronycine n'est que marginalement active.

***2- Activité antitumorale sur le carcinome du colon humain HT29***

La lignée HT29 a été fournie par l'ATCC (American Type Culture Collection, Rockville, USA). Les cellules tumorales ont été inoculées à des souris Nude Swiss femelles ($10^7$ cellules par animal) par voie sous-cutanée. Les tumeurs ont ensuite été prélevées, dissociées en fragments qui ont été greffés sur des souris Nude par voie sous-cutanée. Lorsque le volume tumoral a atteint 50 à 100 $mm^3$, les souris ont été réparties en groupes expérimentaux comprenant 8 (groupe témoin) ou 6 (groupes traités) animaux (jour 0). Les produits ont été administrés par voie i.v. 1 fois par semaine pendant 3 semaines. Les tumeurs ont été mesurées 2 fois par semaine et les volumes tumoraux calculés selon la formule :

$$volume\ (mm^3) = longueur\ (mm) \times largeur^2\ (mm^2) / 2$$

Les résultats sont exprimés en % $T/C = \frac{(Vt/V0)\ \text{médian du groupe traité}}{(Vt/V0)\ \text{médian du groupe témoin}} \times 100$

V0 et Vt étant respectivement le volume initial et le volume au temps de mesure t.

Le tableau 3 indique l'activité antitumorale obtenue aux doses optimales pour l'exemple 5 :

Tableau 3

| Activité antitumorale sur la lignée HT29 | | | | |
|---|---|---|---|---|
| **Produit** | **Schéma** | **Voie** | **Dose Optimale (mg/kg)** | **T/C % au jour 32** |
| Exemple 5 | J0, 7,14 | i.v. | 3.12 | 17 |

Le composé de l'exemple 5 inhibe la croissance de la tumeur HT29, qui est, de façon générale, peu sensible aux agents antitumoraux. Ce produit est très actif dans ce modèle, l'inhibition étant de 83 % au jour 32, soit 18 jours après la dernière administration.

**Exemple 73 : Composition pharmaceutique : comprimés**

| Formule de préparation pour 1000 comprimés dosés à 10 mg : | |
|---|---|
| Composé de l'exemple 7 | 10 g |
| Lactose | 40 g |
| Stéarate de Magnésium | 10 g |
| Amidon de blé | 15 g |
| Amidon de Maïs | 15 g |
| Silice | 3 g |
| Hydroxypropylcellulose | 5 g |

## Revendications

1. Composé de formule (I) :

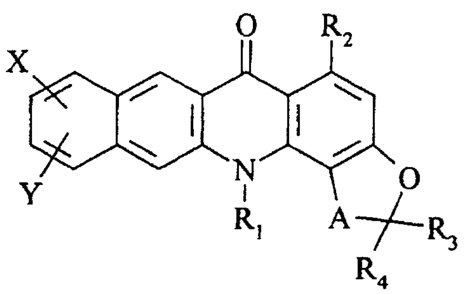

(I)

dans laquelle :

X, Y, identiques ou différents, indépendamment l'un de l'autre, représentent un atome d'hydrogène, d'halogène, un groupement hydroxy, mercapto, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, amino (éventuellement substitué par un ou deux groupements, identiques ou différents, alkyle ($C_1$-$C_6$) linéaires ou ramifiés eux-mêmes éventuellement substitués par un groupement alkoxy ($C_1$-$C_6$) linéaire ou ramifié ou par un groupement de formule $-NR'_7R'_8$ dans laquelle $R'_7$ et $R'_8$, identiques ou différents, indépendamment l'un de l'autre, représentent un atome d'hydrogène ou un groupement alkyle ($C_1$-$C_6$) linéaire ou ramifié), ou forment ensemble un groupement méthylènedioxy ou un groupement éthylènedioxy,

$R_1$ représente un atome d'hydrogène ou un groupement alkyle ($C_1$-$C_6$) linéaire ou ramifié,

$R_2$ représente :

- un atome d'hydrogène,
- un groupement hydroxy,
- un groupement alkyle ($C_1$-$C_6$) linéaire ou ramifié,
- un groupement alkoxy ($C_1$-$C_6$) linéaire ou ramifié, éventuellement substitué par un groupement :

  * de formule $NR_7R_8$ dans laquelle $R_7$ et $R_8$, identiques ou différents, indépendamment l'un de l'autre, représentent un atome d'hydrogène, un groupement alkyle ($C_1$-$C_6$) linéaire ou ramifié, ou un groupement hydroxyalkyle ($C_1$-$C_6$) linéaire ou ramifié,
  * ou par un hétérocycle saturé ou insaturé, monocyclique ou bicyclique, de 5 à 7 chaînons comportant un ou deux hétéroatomes choisis parmi oxygène, azote et soufre,

- un groupement alkylcarbonyloxy ($C_1$-$C_6$) linéaire ou ramifié,
- ou un groupement amino éventuellement substitué :

  * par un ou deux groupements alkyles ($C_1$-$C_6$) linéaires ou ramifiés, identiques ou différents,
  * par un groupement alkylcarbonyle ($C_1$-$C_6$) linéaire ou ramifié, éventuellement substitué par un groupement $-NR_7R_8$ avec $R_7$ et $R_8$ tels que définis précédemment,
  * par un groupement de formule $-R_9-NR_7R_8$, dans laquelle $R_9$ représente un groupement alkylène ($C_1$-$C_6$) linéaire ou ramifié et $R_7$, $R_8$, identiques ou différents, indépendamment l'un de l'autre représentent un atome d'hydrogène, un groupement alkyle ($C_1$-$C_6$) linéaire ou ramifié, ou un groupement hydroxyalkyle ($C_1$-$C_6$) linéaire ou ramifié,
  * par un groupement alkylène ($C_1$-$C_6$), linéaire ou ramifié, substitué par un hétérocycle saturé ou insaturé, monocyclique ou bicyclique, de 5 à 7 chaînons comportant un ou deux hétéroatomes choisis parmi oxygène, azote et soufre,
  * ou par un groupement de formule

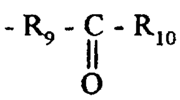

dans laquelle $R_9$ est tel que défini précédemment, et $R_{10}$ représente un groupement hydroxy ou alkoxy ($C_1$-$C_6$) linéaire ou ramifié,

$R_3$, $R_4$, identiques ou différents, indépendamment l'un de l'autre, représentent un atome d'hydrogène ou un groupement alkyle ($C_1$-$C_6$) linéaire ou ramifié,

A représente un groupement -CH=CH- ou un groupement éthylène de formule $-CH(R_5)-CH(R_6)$ dans laquelle $R_5$ et $R_6$, identiques ou différents, indépendamment l'un de l'autre, représentent :

- un atome d'hydrogène,
- un groupement hydroxy,
- un groupement alkoxy ($C_1$-$C_6$) linéaire ou ramifié,
- un groupement alkylcarbonyloxy ($C_1$-$C_6$) linéaire ou ramifié,
- un groupement arylcarbonyloxy,
- un groupement amino éventuellement substitué par un ou deux groupements alkyles ($C_1$-$C_6$) linéaires ou ramifiés, identiques ou différents, ou par un groupement acyle ($C_1$-$C_6$) linéaire ou ramifié,
- un groupement mercapto, un groupement alkylthio ($C_1$-$C_6$) linéaire ou ramifié ou un groupement arylthio,

ou $R_5$ et $R_6$ forment ensemble :

- un groupement

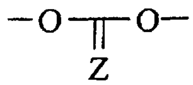 , 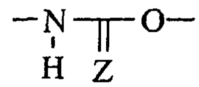 , 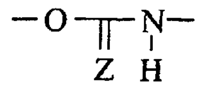

dans lesquels Z représente un atome d'oxygène ou un atome de soufre,

- un groupement $-O-(CH_2)_n-O-$ dans lequel n est un entier compris entre 1 et 4 inclus,
- un groupement

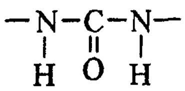 ,

- ou un groupement

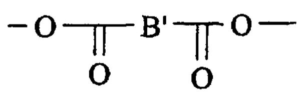 , 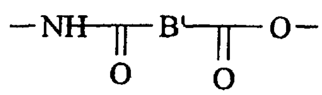 ,

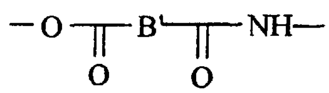

dans lesquels B' représente une liaison simple, un groupement alkylène ($C_1$-$C_6$) linéaire ou ramifié, ou un groupement alkénylène ($C_1$-$C_6$) linéaire ou ramifié,

ou $R_5$ et $R_6$ forment ensemble avec les atomes de carbone qui les portent, un groupement oxirane ou un groupement aziridine, éventuellement substitué sur l'atome d'azote par un groupement alkyle ($C_1$-$C_6$) linéaire ou ramifié,

étant entendu, que le terme "aryle" représente un groupement phényle ou naphtyle, comportant éventuellement une ou plusieurs substitutions, identiques ou différentes, choisies parmi hydroxy, halogéno, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié et alkylcarbonyloxy ($C_1$-$C_6$) linéaire ou ramifié,
leurs isomères optiques, N-oxydes, ainsi que leurs sels d'addition à un acide ou à une base pharmaceutiquement acceptable.

2. Composés de formule (I) selon la revendication 1 **caractérisés en ce que** $R_3$ et $R_4$, identiques ou différents, représentent un groupement alkyle ($C_1$-$C_6$) linéaire ou ramifié, leurs isomères optiques, N-oxydes ainsi que leurs sels d'addition à un acide ou à une base pharmaceutiquement acceptable.

3. Composés de formule (I) selon la revendication 1 **caractérisés en ce que** $R_2$ représente un groupement alkoxy ($C_1$-$C_6$) linéaire ou ramifié, ou un groupement amino éventuellement substitué par un ou deux substituants tels que définis dans la revendication 1, leurs isomères optiques, N-oxydes ainsi que leurs sels d'addition à un acide ou à une base pharmaceutiquement acceptable.

4. Composés de formule (I) selon l'une quelconque des revendications 1 ou 3 **caractérisés en ce que** $R_2$ représente un groupement amino substitué par un groupement de formule $-R_9-NR_7R_8$ dans laquelle $R_9$ représente un groupement alkylène ($C_1$-$C_6$) linéaire ou ramifié, et $R_7$, $R_8$, identiques ou différents, représentent un atome d'hydrogène ou un groupement alkyle ($C_1$-$C_6$) linéaire ou ramifié, leurs isomères optiques, N-oxydes, ainsi que leurs sels d'addition à un acide ou à une base pharmaceutiquement acceptable.

5. Composés de formule (I) selon la revendication 1 **caractérisés en ce que** A représente un groupement -CH=CH- ou un groupement de formule $-CH(R_5)-CH(R_6)-$ dans laquelle $R_5$ et $R_6$ représentent un groupement hydroxy ou alkylcarbonyloxy ($C_1$-$C_6$) linéaire ou ramifié, ou $R_5$ et $R_6$ forment ensemble un groupement

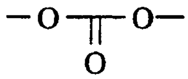 ,

leurs isomères optiques, N-oxydes ainsi que leurs sels l'addition à un acide ou à une base pharmaceutiquement acceptable.

**6.** Composé de formule (I) selon l'une quelconque des revendications 1 à 5 qui est le (±)-*cis*-1,2-diacétoxy-6-méthoxy-3,3,14-triméthyl-2,3,7,14-tétrahydro-1H-benzo[b]pyrano [3,2-h]-acridine-7-one.

**7.** Composé de formule (I) selon l'une quelconque des revendications 1 à 5 qui est le *cis*-7-méthoxy-4,4,15-triméthyl-3a,8,15,15c-tétrahydro-4H-benzo[b][1,3]dioxolo[4',5':4,5]pyrano [3,2-h] acridin-2,8-dione.

**8.** Composé de formule (I) selon l'une quelconque des revendications 1 à 5 qui est le 6-méthoxy-3,3,14-triméthyl-7,14-dihydro-3H-benzo[b]pyrano[3,2-h]acridin-7-one.

**9.** Composé de formule (I) selon l'une quelconque des revendications 1 à 5 qui est le 6-(diéthylaminopropylamino)-3,3,14-triméthyl-7,14-dihydro-3H-benzo[b]pyrano[3,2-h] acridin-7-one.

**10.** Composé de formule (I) selon l'une quelconque des revendications 1 à 5 qui est le (±)-*cis*-Diacétoxy-6-(diétylaminopropylamino)-3,3,14-triméthyl-2,3,7,14-tétrahydro-1H-benzo [b]pyrano[3,2-h]acridin-7-one.

**11.** Procédé de préparation des composés de formule (I) selon la revendication 1 **caractérisé en ce que** l'on fait réagir :

- *soit un dérivé de l'acide 3-amino-2-naphtalène carboxylique (II) :*

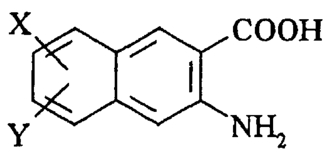

(II)

dans laquelle X et Y sont tels que définis dans la formule (I),
avec un dérivé du phloroglucinol de formule (III) :

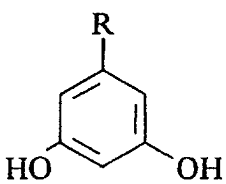

(III)

dans laquelle R représente un atome d'hydrogène, un groupement hydroxy ou un groupement alkyle ($C_1$-$C_6$) linéaire ou ramifié,
pour conduire aux composés de formule (IV) :

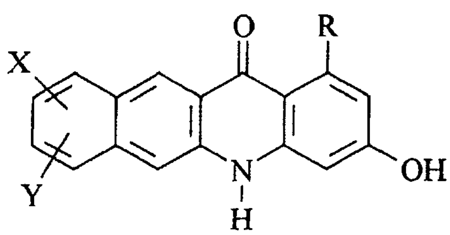

(IV)

dans laquelle X, Y et R sont tels que définis précédemment,
qui est ensuite traité en condition basique dans un solvant aprotique tel que la diméthylformamide, par un alcyne de formule V :

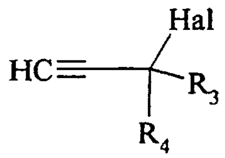

(V)

dans laquelle Hal représente un atome d'halogène et $R_3$ et $R_4$ sont tels que définis dans la formule (I), pour conduire aux composés de formule (I/a), cas particulier des composés de formule (I):

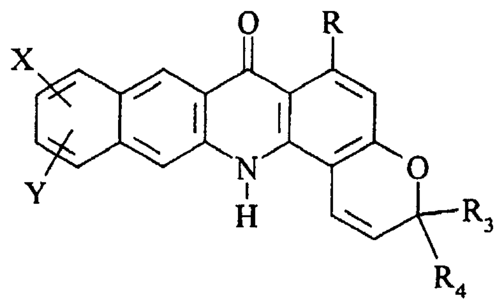

(I/a)

dans laquelle X, Y, R, $R_3$ et $R_4$ sont tels que définis précédemment,

- *soit un dérivé de l'acide 3-halogéno-2-naphtalène carboxylique de formule (VI) :*

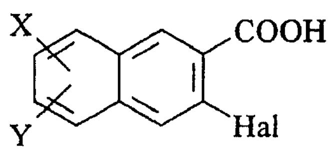

(VI)

dans laquelle X et Y sont tels que définis dans la formule (I) et Hal représente un atome d'halogène, tel que le chlore ou le brome,
avec un dérivé amino-chromène de formule (VII) :

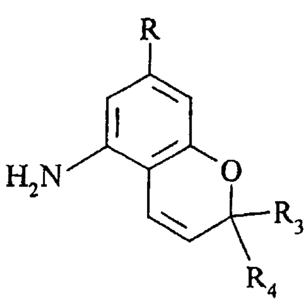

(VII)

dans laquelle $R_3$ et $R_4$ sont tels que définis dans la formule (I) et R a la même signification que précédemment, pour conduire également aux composés de formule (I/a), cas particulier des composés de formule (I):

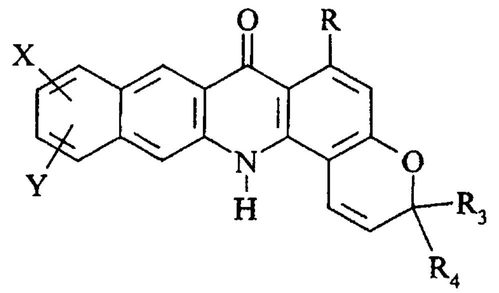

(I/a)

dans laquelle X, Y, R, $R_3$ et $R_4$ sont tels que définis précédemment,

<u>composés de formule (I/a)</u>,
dont l'atome d'azote est éventuellement substitué, par action d'un halogénure d'alkyle ou d'un sulfate de dialkyle en présence d'un agent de déprotonation, tel que l'hydrure de sodium, en solvant polaire aprotique, de manière à obtenir les composés de formule (I/b), cas particulier des composés de formule (I) :

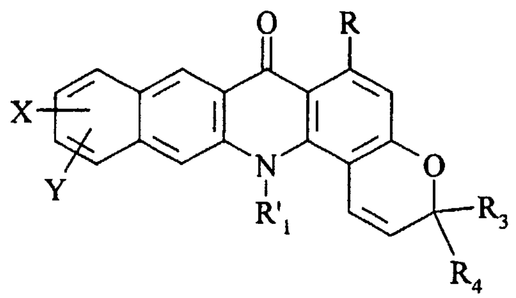

(I/b)

dans laquelle X, Y, R, $R_3$ et $R_4$ sont tels que définis précédemment, et $R'_1$ représente un groupement alkyl ($C_1$-$C_6$) linéaire ou ramifié,

<u>composés de formule (I/b),</u>
pouvant être soumis à l'action d'un agent alkylant tel qu'un sulfate de dialkyle, d'un agent acylant tel que l'anhydride acétique ou traité dans des conditions de réaction de Friedel et Craft, pour conduire aux composés de formule (I/c), cas particulier des composés de formule (I) :

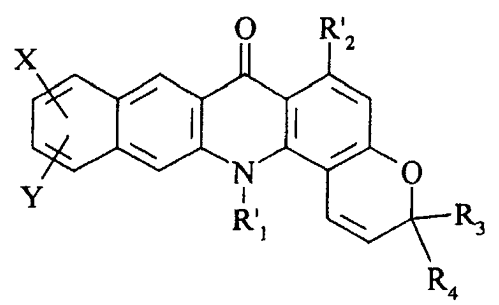

(I/c)

dans laquelle X, Y, $R'_1$, $R_3$ et $R_4$ sont tels que définis précédemment et $R'_2$ représente un groupement alkoxy éventuellement substitué par un groupement de formule $NR_7R_8$ tel que défini dans la formule (I), ou alkylcarbonyloxy ($C_1$-$C_6$) linéaire ou ramifié,

<u>composé de formule (I/c)</u>,
pouvant être éventuellement traité, dans le cas où $R'_2$ représente un groupement alkoxy par exemple, par un dérivé amino de formule (VIII) :

$$HNR_aR_b \qquad (VIII)$$

dans laquelle $R_a$ représente un atome d'hydrogène ou un groupement alkyle ($C_1$-$C_6$) linéaire ou ramifié et $R_b$ représente un groupement alkyle, un groupement $R_9$-$NR_7R_8$ (dans lequel $R_9$ représente un groupement alkylène ($C_1$-$C_6$) linéaire ou ramifié, et $R_7$, $R_8$, identiques ou différents représentent un atome d'hydrogène, un groupement alkyle ($C_1$-$C_6$) linéaire ou ramifié, ou un groupement hydroxyalkyle ($C_1$-$C_6$) linéaire ou ramifié), un groupement hétérocycloalkylène (les termes alkylène et hétérocycle ayant la même signification que dans la formule (I)), ou un groupement

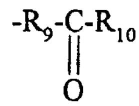

dans lequel $R_9$ et $R_{10}$ ont la même définition que dans la formule (I),
pour conduire aux composés de formule (I/d), cas particulier des composés de formule (I):

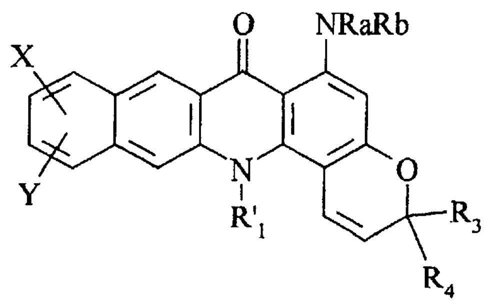

(I/d)

dans laquelle X, Y, $R'_1$, $R_3$,$R_4$, Ra et Rb sont tels que définis précédemment,
l'ensemble des composés de formule (I/a) à (I/d) formant le composé de formule (I/e):

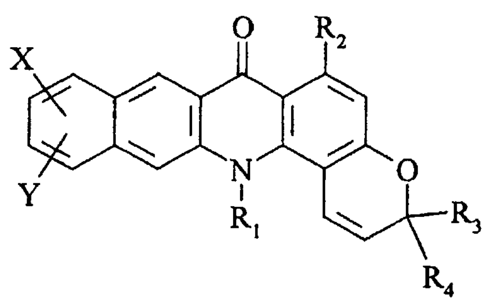

(I/e)

dans laquelle X, Y, $R_1$, $R_2$, $R_3$ et $R_4$ ont la même signification que dans la définition générale de la formule (I),

composé de formule (I/e) pouvant être soumis,

a) ⇨ soit à l'action d'un agent réducteur, pour conduire aux composés de formule (I/f), cas particulier des composés de formule (I):

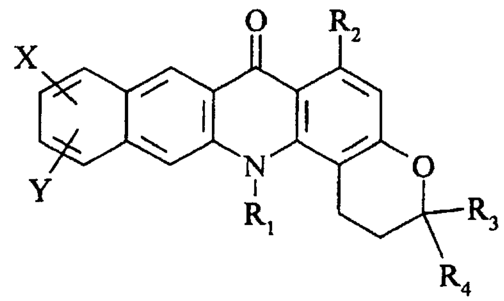

(I/f)

dans laquelle X, Y, $R_1$, $R_2$, $R_3$ et $R_4$ sont tels que définis précédemment,

b) ⇨ soit à l'action du tétroxyde d'osmium en milieu polaire et en présence de 4-Méthylmorpholine-N-oxyde, pour conduire aux composés de formules (I/g) et (I/g'), cas particuliers des composés de formule (I) :

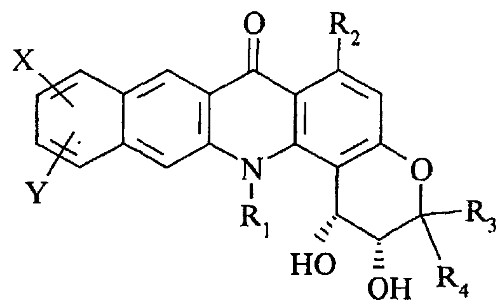

(I/g)

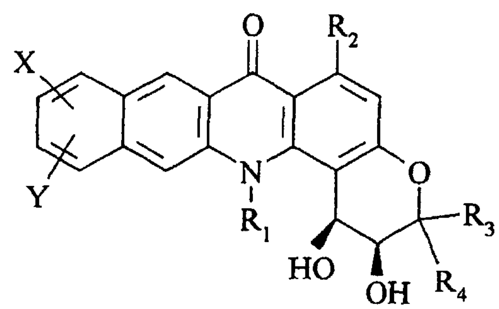

(I/g')

dans lesquelles X, Y, $R_1$, $R_2$, $R_3$ et $R_4$ sont tels que définis précédemment,
les composés de formule (I/g) et (I/g') pouvant aussi être obtenus séparément par synthèse chirale et notamment par *cis* dihydroxylation asymétrique à partir du composé (I/e) en utilisant des ligands chiraux du type pyridine ou phtalazine bisubstitués par des alcaloïdes de *Cinchona* comme la dihydroquinine et son diastéréoisomère dextrogyre la dihydroquinidine,
l'ensemble des composés de formules (I/g) et (I/g') formant les composés *cis*-diol de formule (*cis*-I/h) :

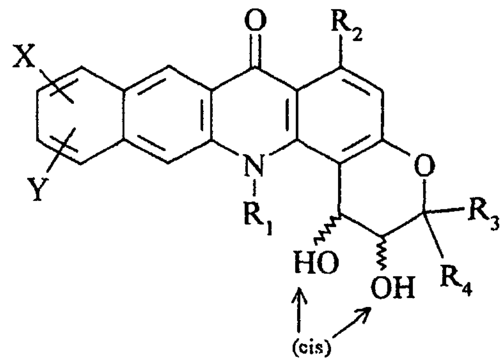

(cis-I/h)

dans laquelle X, Y, $R_1$, $R_2$, $R_3$ et $R_4$ sont tels que définis précédemment,
composés *cis*-diol de formule (*cis*-I/h) que l'on soumet éventuellement à l'action du N,N'-carbonyldiimidazole ou du N,N'-thiocarbonyldiimidazole en présence de 2-butanone, pour conduire aux composés de formule (*cis*-I/i), cas particulier des composés de formule (I) :

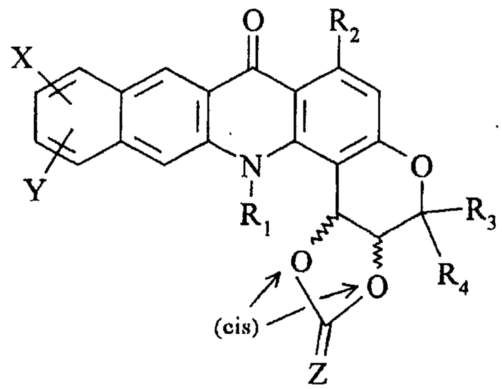

(cis I/i)

dans laquelle X, Y, $R_1$, $R_2$, $R_3$ et $R_4$ sont tels que définis précédemment, et Z représente un atome d'oxygène ou un atome de soufre,

c) ⇨ soit à l'action du permanganate de potassium en milieu polaire, pour conduire aux composés de formule (IX) :

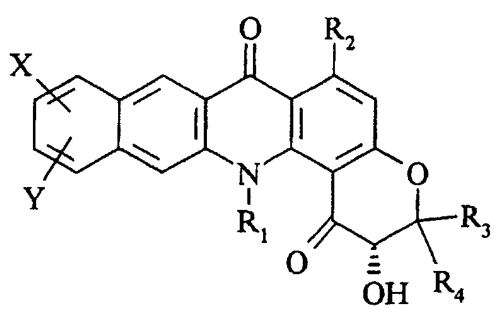

(IX)

dans laquelle X, Y, $R_1$, $R_2$, $R_3$ et $R_4$ sont tels que définis précédemment,
que l'on soumet à des conditions réductrices en présence de $NaBH_4$ par exemple, pour conduire aux composés de formule (I/j), cas particulier des composés de formule (I) :

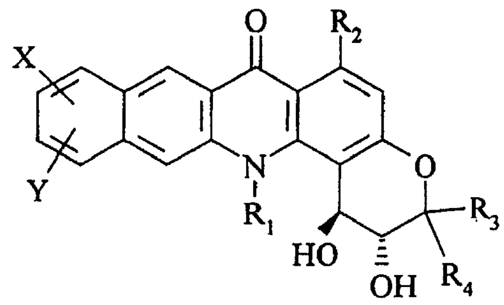

(I/j)

dans laquelle X, Y, $R_1$, $R_2$, $R_3$ et $R_4$ sont tels que définis précédemment,
l'ensemble des composés de formules (I/j) et (*cis*-I/h) formant les composés de formule (I/k)

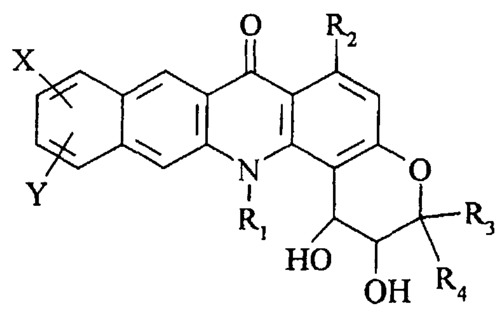

(I/k)

dans laquelle X, Y, $R_1$, $R_2$, $R_3$ et $R_4$ sont tels que définis précédemment,

composés de formule (I/k) que l'on soumet :

- soit à l'action d'un composé de formule (X) ou (XI) :

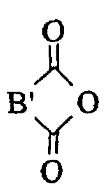

(X)

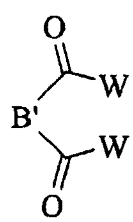

(XI)

dans lesquelles B' est tel que défini dans la formule (I), et W représente un atome d'halogène ou un groupement hydroxyle,
pour conduire aux composés de formule (I/l)

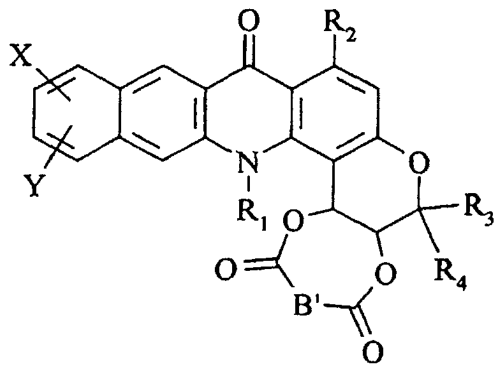

(I/l)

cas particulier des composés de formule (I), dans laquelle B', X, Y, $R_1$, $R_2$, $R_3$ et $R_4$ sont tels que définis précédemment,

❖ soit à l'action d'un dihalogénure d'alkyle ($C_1$-$C_6$) linéaire, pour conduire aux composés de formule (I/m), cas particulier des composés de formule (I):

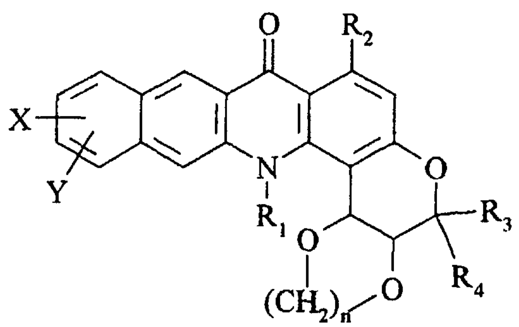

(I/m)

dans laquelle X, Y, $R_1$, $R_2$, $R_3$, $R_4$ et n sont tels que définis précédemment,

❖ soit à l'action d'un alcool de formule $R_{11}$-OH dans laquelle $R_{11}$ représente un groupement alkyle ($C_1$-$C_6$) linéaire ou ramifié,
pour conduire aux composés de formule (I/n) :

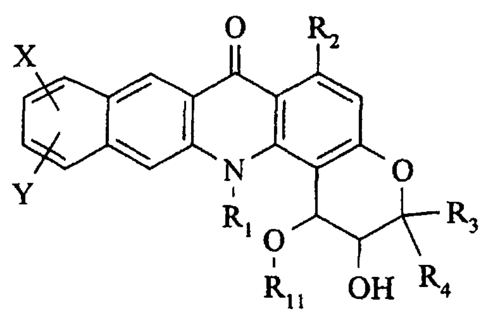

(I/n)

cas particulier des composés de formule (I), dans laquelle X, Y, $R_1$, $R_2$, $R_3$, $R_4$ et $R_{11}$ sont tels que définis précédemment,
composés de formule (I/n) dont la fonction alcool est estérifiée en présence d'une base faible, telle que la pyridine, par l'anhydride de formule $(R_{12}CO)_2O$, dans laquelle $R_{12}$ représente un groupement alkyle ($C_1$-$C_6$) linéaire ou ramifié ou un groupement aryle tel que défini précédemment,
pour conduire aux composés de formule (I/o), cas particulier des composés de formule (I):

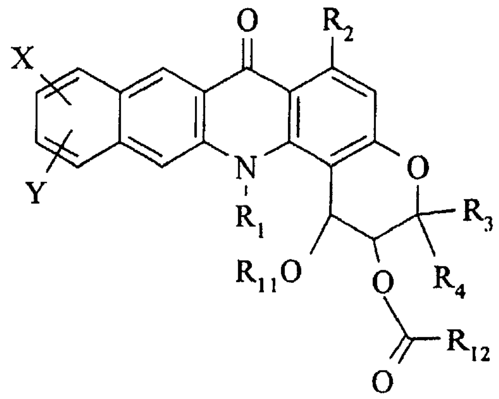

(I/o)

dans laquelle X, Y, $R_1$, $R_2$, $R_3$, $R_4$, $R_{11}$ et $R_{12}$ sont tels que définis précédemment,

- soit à l'action d'un iodure d'alkyle de formule $R'_{11}$-I dans laquelle $R'_{11}$ représente un groupement alkyle ($C_1$-$C_6$) linéaire ou ramifié, en présence de sel d'Argent, pour conduire aux composés de formule (I/p) :

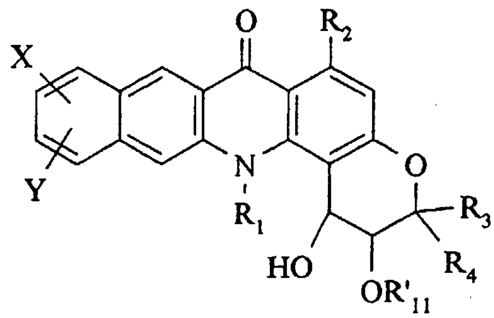

(I/p)

cas particulier des composés de formule (I), dans laquelle X, Y, $R_1$, $R_2$, $R_3$, $R_4$ et $R'_{11}$ sont tels que définis précédemment,
composés de formule (I/p) dont la fonction alcool est estérifiée par un anhydride de formule $(R'_{12}CO)_2O$ dans laquelle $R'_{12}$ représente un groupement alkyle ($C_1$-$C_6$) linéaire ou ramifié, ou un groupement aryle tel que défini précédemment, pour conduire aux composés de formule (I/q) :

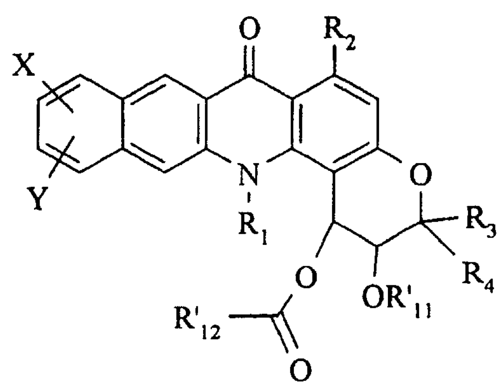

(I/q)

cas particulier des composés de formule (I), dans laquelle X, Y, $R_1$, $R_2$, $R_3$, $R_4$, $R'_{11}$ et $R'_{12}$ sont tels que définis précédemment,

- soit à l'action directe d'un anhydride de formule $(R_{12}CO)_2O$, en présence d'une base, telle que la triéthylamine, afin d'obtenir les composés de formule (I/r), cas particulier des composés de formule (I):

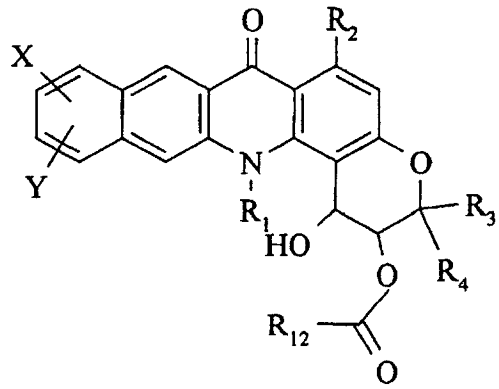

(I/r)

dans laquelle X, Y, $R_1$, $R_2$, $R_3$, $R_4$ et $R_{12}$ sont tels que définis précédemment, qui peut être soumis à nouveau, dans les mêmes conditions opératoires, à l'action de l'anhydride de formule $(R'_{12}CO)_2O$, pour conduire aux composés de formule (I/s) :

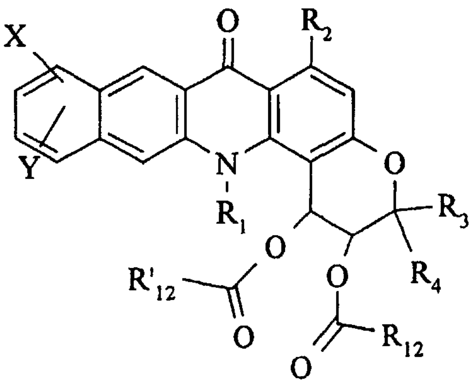

(I/s)

dans laquelle X, Y, $R_1$, $R_2$, $R_3$, $R_4$, $R_{12}$ et $R'_{12}$ sont tels que définis précédemment, les deux groupements $R_{12}$ et $R'_{12}$ pouvant être identiques ou différents,

- soit à des conditions de déhydratation en milieu acide pour conduire aux composés de formule (XII) :

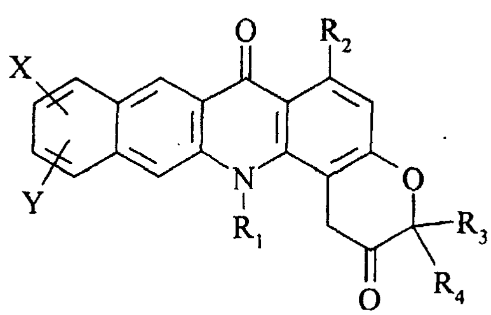

(XII)

dans laquelle X, Y, $R_1$, $R_2$, $R_3$ et $R_4$, sont tels que définis précédemment, que l'on réduit en présence de $NaBH_4$ pour conduire aux composés de formule (I/t) :

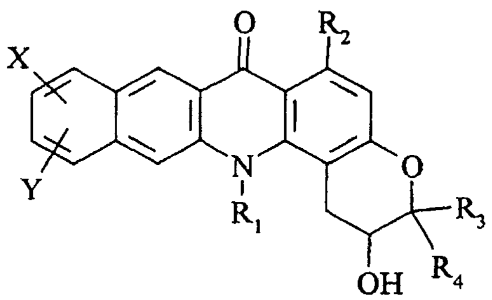

(I/t)

cas particulier des composés de formule (I), dans laquelle X, Y, $R_1$, $R_2$, $R_3$ et $R_4$, sont tels que définis précédemment,

d) ➪ soit à l'action d'un peracide, comme l'acide m-chloroperbenzoïque, pour conduire au composé de formule (I/u), cas particulier des composés de formule (I):

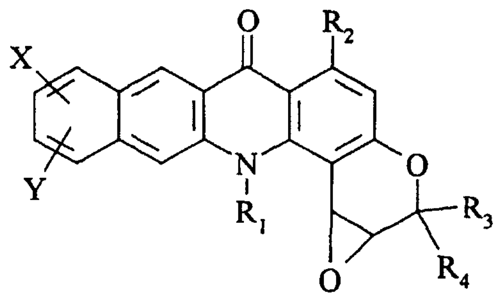

(I/u)

dans laquelle X, Y, $R_1$, $R_2$, $R_3$ et $R_4$, sont tels que définis précédemment,
composé de formule (I/u)

que l'on traite éventuellement par de l'ammoniac ou une amine primaire ou secondaire pour conduire aux composés de formule (I/v) et/ou (I/v') suivant la nature des réactifs, cas particulier des composés de formule (I):

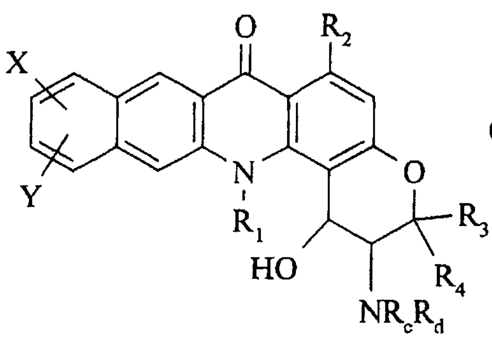

(I/v)

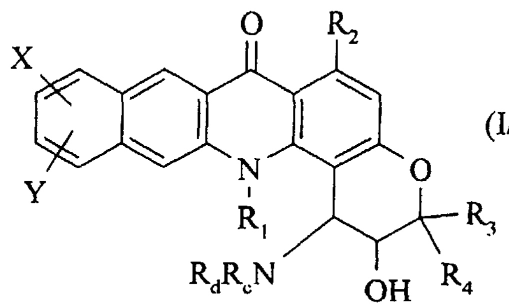

(I/v')

dans lesquelles X, Y, $R_1$, $R_2$, $R_3$ et $R_4$, sont tels que définis précédemment, et $R_c$ et $R_d$ représentent un atome d'hydrogène ou un groupement alkyle ($C_1$-$C_6$) linéaire ou ramifié,
composé de formule (I/v') pouvant également être obtenu directement à partir des composés de formule (I/k) par traitement de ceux-ci avec $NaN_3$ conduisant aux dérivés azide intermédiaires, suivi d'une hydrogénation en présence de palladium, conduisant alors aux composés de formule (I/v') dans laquelle Rc et Rd représentent un atome d'hydrogène,

composé de formule (I/v) et (I/v') pouvant être soumis :

- soit, dans le cas où Rc et Rd représentent un atome d'hydrogène, à l'action du N,N'-carbonyldiimidazole

ou du N,N'-thiocarbonyldiimidazole par exemple, pour conduire respectivement aux composés de formule (I/w) et (I/w'), cas particulier des composés de formule (I) :

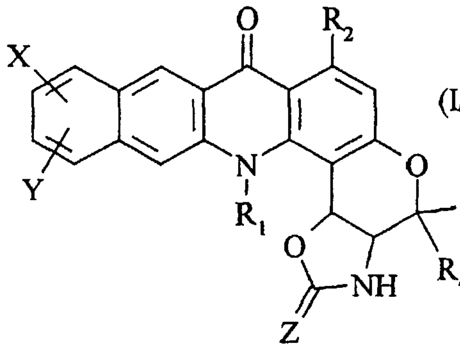
(I/w)

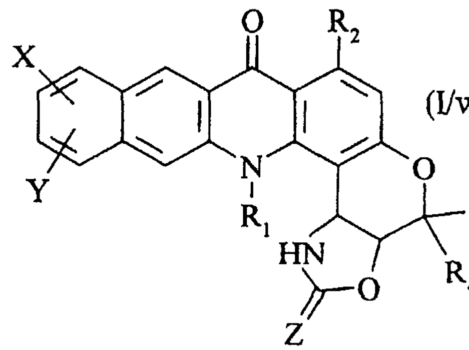
(I/w')

dans lesquelles X, Y, $R_1$, $R_2$, $R_3$ et $R_4$, sont tels que définis précédemment, et Z représente un atome d'oxygène ou un atome de soufre suivant la nature du réactif utilisé,

❖ soit à l'action d'un composé de formule (XI) $WO_2C$-B'-$CO_2W$ tel que défini précédemment, pour conduire respectivement au composé de formule (I/x) et (I/x'), cas particulier des composés de formule (I) :

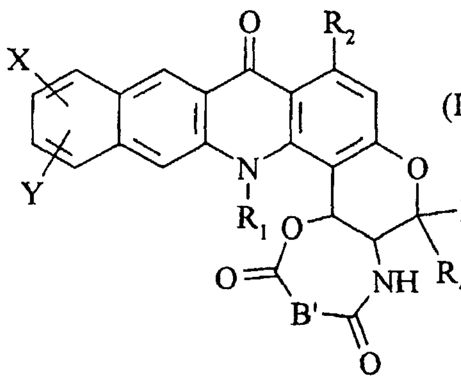
(I/x)

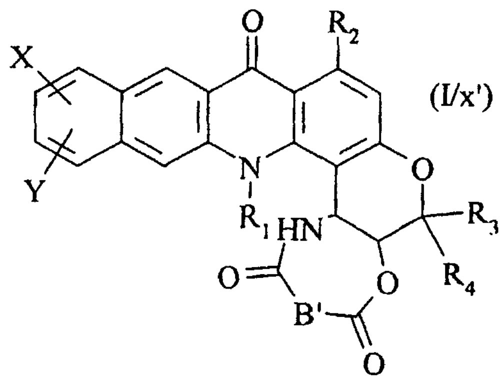
(I/x')

dans lesquelles B', X, Y, $R_1$, $R_2$, $R_3$ et $R_4$, sont tels que définis précédemment,

❖ soit à l'action du dibromure de triphénylphosphine en présence de triéthylamine pour conduire aux composés de formule (I/y), cas particulier des composés de formule (I) :

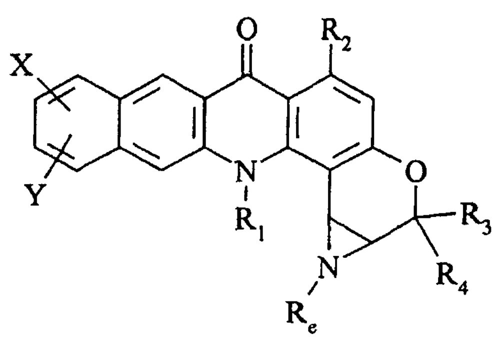
(I/y)

dans laquelle X, Y, $R_1$, $R_2$, $R_3$ et $R_4$, sont tels que définis précédemment, et $R_e$ représente un atome d'hydrogène ou un groupement alkyle ($C_1$-$C_6$)linéaire ou ramifié,

e) ⇨ soit à l'action de $NaN_3$ en présence d'eau oxygénée, suivi d'une étape de réduction par l'hydrure

de tri-n-butylétain, par exemple, pour conduire aux composés de formule (I/z), cas particulier des composés de formule (I) :

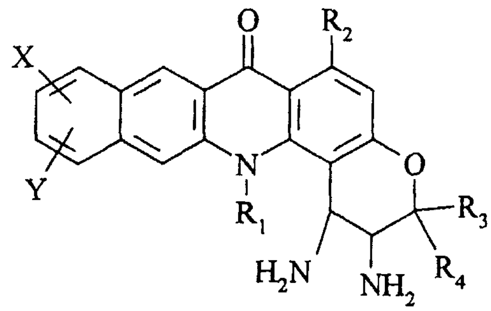

(I/z)

dans laquelle X, Y, $R_1$, $R_2$, $R_3$ et $R_4$ sont tels que définis précédemment, composés de formule (I/z) pouvant éventuellement être soumis à l'action du dioxyde de carbone, en présence de diphényl phosphite, pour conduire aux composés de formule (I/aa), cas particulier des composés de formule (I) :

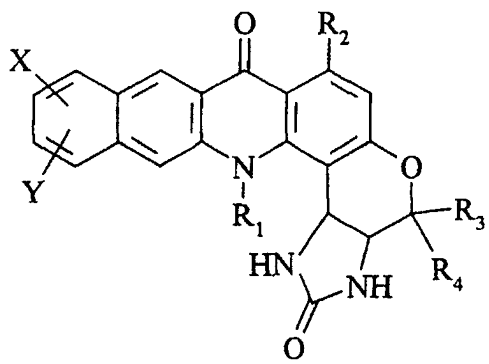

(I/aa)

dans laquelle X, Y, $R_1$, $R_2$, $R_3$ et $R_4$ sont tels que définis précédemment,

les composés (I/a-z), (I/g'), (I/v'-x'), (I/aa) et (*cis* I/h-I/i), forment l'ensemble des composés de l'invention que l'on purifie, le cas échéant, selon une technique classique de purification, qui peuvent, si on le désire, être séparés en leurs différents isomères optiques selon une technique classique de séparation et que l'on transforme, si on le souhaite, en leurs N-oxydes et, le cas échéant, en leurs sels d'addition à un acide ou à une base pharmaceutiquement acceptable.

**12.** Compositions pharmaceutiques contenant comme principe actif au moins un composé selon l'une quelconque des revendications 1 à 10, seul ou en combinaison avec un ou plusieurs excipients ou véhicules inertes non toxiques pharmaceutiquement acceptables.

**13.** Compositions pharmaceutiques selon la revendication 12 contenant au moins un principe actif selon l'une quelconque des revendications 1 à 10 utiles dans le traitement du cancer.

**Patentansprüche**

**1.** Verbindungen der Formel (I):

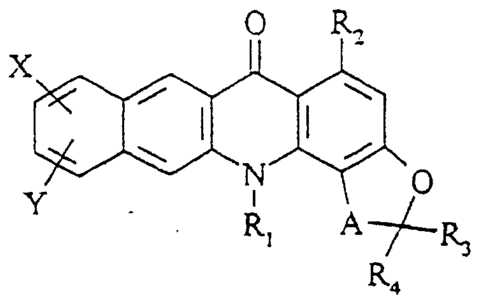

(I)

in der:

X und Y, die gleichartig oder verschieden sind, unabhängig voneinander ein Wasserstoffatom, ein Halogenatom, eine Hydroxygruppe, Mercaptogruppe, Cyanogruppe, Nitrogruppe, geradkettige oder verzweigte ($C_1$-$C_6$)-Alkylgruppe, geradkettige oder verzweigte ($C_1$-$C_6$)-Alkoxygruppe, geradkettige oder verzweigte ($C_1$-$C_6$)-Trihalogenalkylgruppe, Aminogruppe (die gegebenenfalls substituiert ist durch eine oder zwei gleichartige oder verschiedene geradkettige oder verzweigte ($C_1$-$C_6$)-Alkylgruppen, die ihrerseits gegebenenfalls durch eine geradkettige oder verzweigte ($C_1$-$C_6$)-Alkoxygruppe oder eine Gruppe der Formel $-NR'_7R'_8$, worin $R'_7$ und $R'_8$, die gleichartig oder verschieden sind, unabhängig voneinander ein Wasserstoffatom oder eine geradkettige oder verzweigte ($C_1$-$C_6$)-Alkylgruppe darstellen, substituiert sind), bedeuten oder gemeinsam eine Methylendioxygruppe oder eine Ethylendioxygruppe bilden,

$R_1$ ein Wasserstoffatom oder eine geradkettige oder verzweigte ($C_1$-$C_6$)-Alkylgruppe bedeutet,

$R_2$:

- ein Wasserstoffatom,
- eine Hydroxygruppe,
- eine geradkettige oder verzweigte ($C_1$-$C_6$)-Alkylgruppe.
- eine geradkettige oder verzweigte ($C_1$-$C_6$)-Alkoxygruppe, die gegebenenfalls substituiert ist durch eine Gruppe:

  * der Formel $NR_7R_8$, worin $R_7$ und $R_8$, die gleichartig oder verschieden sind, unabhängig voneinander ein Wasserstoffatom, eine geradkettige oder verzweigte ($C_1$-$C_6$)-Alkylgruppe oder eine geradkettige oder verzweigte ($C_1$-$C_6$)-Hydroxyalkylgruppe darstellen,
  * oder durch einen gesättigten oder ungesättigten, monocyclischen oder bicyclischen Heterocyclus mit 5 bis 7 Kettengliedern, der ein oder zwei Heteroatome ausgewählt aus Sauerstoff, Stickstoff und Schwefel enthält, substituiert ist,

- oder eine geradkettige oder verzweigte ($C_1$-$C_6$)-Alkylcarbonyloxygruppe,
- oder eine Aminogruppe, die gegebenenfalls substituiert ist durch:

  * eine oder zwei geradkettige oder verzweigte, gleichartige oder verschiedene ($C_1$-$C_6$)-Alkylgruppen,
  * eine geradkettige oder verzweigte ($C_1$-$C_6$)-Alkylcarbonylgruppe, die gegebenenfalls durch eine Gruppe $-NR_7R_8$, worin $R_7$ und $R_8$ die oben angegebenen Bedeutungen besitzen, substituiert ist,
  * durch eine Gruppe der Formel $-R_9-NR_7R_8$, worin $R_9$ eine geradkettige oder verzweigte ($C_1$-$C_6$)-Alkylengruppe und $R_7$ und $R_8$, die gleichartig oder verschieden sind, unabhängig voneinander ein Wasserstoffatom, eine geradkettige oder verzweigte ($C_1$-$C_6$)-Alkylgruppe oder eine geradkettige oder verzweigte ($C_1$-$C_6$)-Hydroxyalkylgruppe bedeuten,
  * durch eine geradkettige oder verzweigte ($C_1$-$C_6$)-Alkylengruppe, die durch einen gesättigten oder ungesättigten, monocyclischen oder bicyclischen Heterocyclus mit 5 bis 7 Kettengliedern, der ein oder zwei Heteroatome ausgewählt aus Sauerstoff, Stickstoff und Schwefel enthält, substituiert ist,
  * oder durch eine Gruppe der Formel

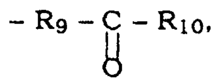

in der $R_9$ die oben angegebenen Bedeutungen besitzen und $R_{10}$ eine Hydroxygruppe oder eine geradkettige oder verzweigte ($C_1$-$C_6$)-Alkoxygruppe darstellt,

$R_3$ und $R_4$, die gleichartig oder verschieden sind, unabhängig voneinander ein Wasserstoffatom oder eine geradkettige oder verzweigte ($C_1$-$C_6$)-Alkylgruppe bedeuten,

A eine Gruppe -CH=CH- oder eine Ethylengruppe der Formel -CH($R_5$)-CH($R_6$) darstellt, in der $R_5$ und $R_6$, die gleichartig oder verschieden sind, unabhängig voneinander:

- ein Wasserstoffatom,
- eine Hydroxygruppe,
- eine geradkettige oder verzweigte ($C_1$-$C_6$)-Alkoxygruppe,
- eine geradkettige oder verzweigte ($C_1$-$C_6$)-Alkylcarbonyloxygruppe,
- eine Arylcarbonyloxygruppe,
- eine Aminogruppe, die gegebenenfalls substituiert ist durch eine oder zwei geradkettige oder verzweigte, gleichartige oder verschiedene ($C_1$-$C_6$)-Alkylgruppen oder eine geradkettige oder verzweigte ($C_1$-$C_6$)-Acylgruppe
- eine Mercaptogruppe, eine geradkettige oder verzweigte ($C_1$-$C_6$)-Alkylthiogruppe oder eine Arylthiogruppe bedeuten,

oder $R_5$ und $R_6$ gemeinsam:

- eine Gruppe

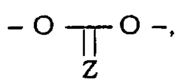, 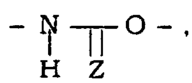, 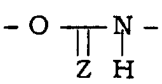,

worin Z ein Sauerstoffatom oder ein Schwefelatom bedeutet,

- eine Gruppe -O-($CH_2$)$_n$-O-, in der n eine ganze Zahl mit einem Wert zwischen und 4 einschließlich darstellt,
- eine Gruppe

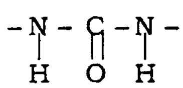,

- oder eine Gruppe

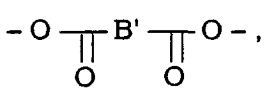, 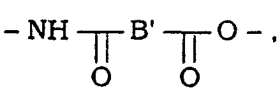, 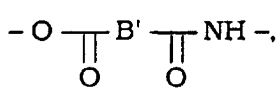

worin die Gruppen B' eine Einfachbindung, eine geradkettige oder verzweigte ($C_1$-$C_6$)-Alkylengruppe oder eine geradkettige oder verzweigte ($C_1$-$C_6$)-Alkenylengruppe bedeutet,

oder $R_5$ und $R_6$ gemeinsam mit den sie tragenden Kohlenstoffatomen eine Oxirangruppe oder eine Aziridingruppe bedeuten, die gegebenenfalls am Stickstoffatom durch eine geradkettige oder verzweigte ($C_1$-$C_6$)-Alkylgruppe substituiert ist,

mit der Maßgabe, daß der Begriff "Aryl" eine Phenyl- oder Naphthylgruppe darstellt, die gegebenenfalls ein oder

mehrere, gleichartige oder verschiedene Substituenten aufweist, ausgewählt aus Hydroxy, Halogen, Carboxy, Nitro, Amino, geradkettigem oder verzweigte ($C_1$-$C_6$)-Alkylamino oder -Dialkylamino, geradkettigem oder verzweigtem ($C_1$-$C_6$)-Alkoxy. geradkettigem oder verzweigtem ($C_1$-$C_6$)-Acyl und geradkettigem oder verzweigtem ($C_1$-$C_6$) -Alkylcarbonyloxy,
deren optische Isomere, N-Oxide sowie deren Additionssalze mit einer pharmazeutisch annehmbaren Säure oder Base.

**2.** Verbindungen der Formel (I) nach Anspruch 1, **dadurch gekennzeichnet. daß** $R_3$ und $R_4$, die gleichartig oder verschieden sind, eine geradkettige oder verzweigte ($C_1$-$C_6$)-Alkylgruppe bedeuten, deren optische Isomere, N-Oxide sowie deren Additionssalze mit einer pharmazeutisch annehmbaren Säure oder Base.

**3.** Verbindungen der Formel (I) nach Anspruch 1, **dadurch gekennzeichnet, daß** $R_2$ eine geradkettige oder verzweigte ($C_1$-$C_6$)-Alkoxygruppe oder eine gegebenenfalls durch ein oder zwei Substituenten, wie sie in Anspruch 1 definiert sind, substituierte Aminogruppe bedeutet, deren optische Isomere, N-Oxide sowie deren Additionssalze mit einer pharmazeutisch annehmbaren Säure oder Base.

**4.** Verbindungen der Formel (I) nach einem der Ansprüche 1 oder 3, **dadurch gekennzeichnet, daß** $R_3$ eine Aminogruppe darstellt, die durch eine Gruppe der Formel -$R_9$-$NR_7R_8$ substituiert ist, in der $R_9$ eine geradkettige oder verzweigte ($C_1$-$C_6$)-Alkylengruppe und $R_7$ und $R_8$, die gleich oder verschieden sind, ein Wasserstoffatom oder eine geradkettige oder verzweigte ($C_1$-$C_6$)-Alkylgruppe bedeuten, deren optische Isomere, N-Oxide sowie deren Additionssalze mit einer pharmazeutisch annehmbaren Säure oder Base.

**5.** Verbindungen der Formel (I) nach Anspruch 1, **dadurch gekennzeichnet, daß** A eine Gruppe -CH=CH- oder eine Gruppe der Formel -CH($R_5$)-CH($R_6$)- darstellt, worin $R_5$ und $R_6$ eine Hydroxygruppe oder eine geradkettige oder verzweigte ($C_1$-$C_6$)-Alkylcarbonyloxygruppe bedeuten, oder $R_5$ und $R_6$ gemeinsam eine Gruppe

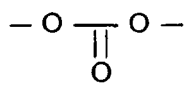

bilden, deren optische Isomere, N-Oxide sowie deren Additionssalze mit einer pharmazeutisch annehmbaren Säure oder Base.

**6.** Verbindung der Formel (I) nach einem der Ansprüche 1 bis 5, nämlich (±)-*cis*-1,2-Diacetoxy-6-methoxy-3,3,14-trimethyl-2,3,7,14-tetrahydro-1H-benzo[b]pyrano[3,2-h]acridin-7-on.

**7.** Verbindung der Formel (I) nach einem der Ansprüche 1 bis 5, nämlich *cis*-7-Methoxy-4,4,15-trimethyl-3a,8,15,15c-tetrahydro-4H-benzo[b][1,3]dioxolo[4',5':4,5]pyrano[3,2-h]acridin-2,8-dion.

**8.** Verbindung der Formel (I) nach einem der Ansprüche 1 bis 5, nämlich 6-Methoxy-3,3,14-trimethyl-7,14-dihydro-3H-benzo[b]pyrano[3,2-hlacridin-7-on.

**9.** Verbindung der Formel (I) nach einem der Ansprüche 1 bis 5, nämlich 6-(Diethylaminopropylamino)-3,3,14-trimethyl-7,14-dihydro-3H-benzo[b]pyrano[3,2-h]acridin-7-on.

**10.** Verbindung der Formel (I) nach einem der Ansprüche 1 bis 5, nämlich (±)-*cis*-Diacetoxy-6-(diethylaminopropylamino)-3,3,14-trimethyl-2,3,7,14-tetrahydro-1H-benzo[b]pyrano[3,2-h]acridin-7-on.

**11.** Verfahren zur Herstellung der Verbindungen der Formel (I) nach Anspruch 1, **dadurch gekennzeichnet, daß** man:

- *entweder ein Derivat der 3-Amino-2-naphthalin-carbonsäure (II):*

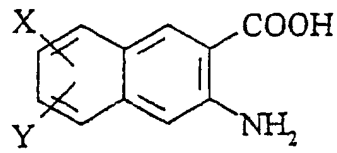

(II)

in der X und Y die bezüglich der Formel (I) angegebenen Bedeutungen besitzen, mit einem Phloroglucinderivat der Formel (III) umsetzt:

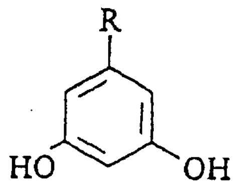

(III)

in der R ein Wasserstoffatom, eine Hydroxygruppe oder eine geradkettige oder verzweigte ($C_1$-$C_6$)-Alkylgruppe darstellt,
zur Bildung der Verbindungen der Formel (IV):

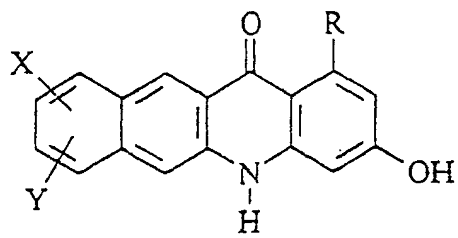

(IV)

in der X, Y und R die oben angegebenen Bedeutungen besitzen,
welche anschließend unter basischen Bedingungen in einem aprotischen Lösungsmittel, wie Dimethylformamid, mit einem Alkin der Formel V behandelt wird:

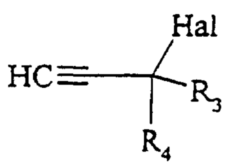

(V)

in der Hal ein Halogenatom darstellt und $R_3$ und $R_4$ die bezüglich der Formel (I) angegebenen Bedeutungen besitzen,
zur Bildung der Verbindungen der Formel (I/a), einem Sonderfall der Verbindungen der Formel (I):

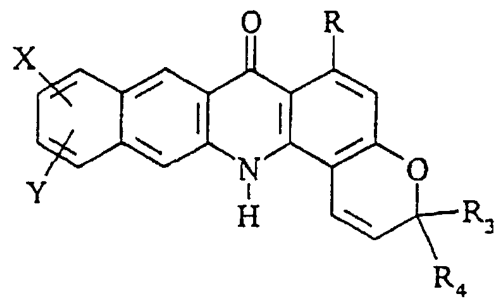

(I/a)

in der X, Y, R, $R_3$ und $R_4$ die oben angegebenen Bedeutungen besitzen,

- *oder ein 3-Halogen-2-naphthalin-carbonsäurederivat der Formel (VI):*

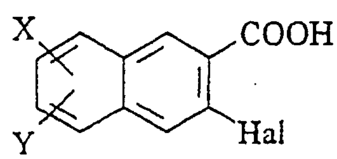

(VI)

in der X und Y die bezüglich der Formel (I) angegebenen Bedeutungen besitzen und Hal ein Halogenatom, wie Chlor oder Brom darstellt,
mit einem Aminochromenderivat der Formel (VII) umsetzt:

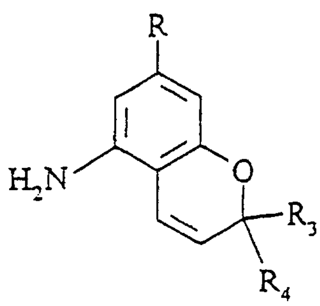

(VII)

in der $R_3$ und $R_4$ die bezüglich der Formel (I) angegebenen Bedeutungen besitzen und R die oben angegebenen Bedeutungen besitzt,
ebenfalls zur Bildung der Verbindungen der Formel (I/a), einem Sonderfall der Verbindungen der Formel (I):

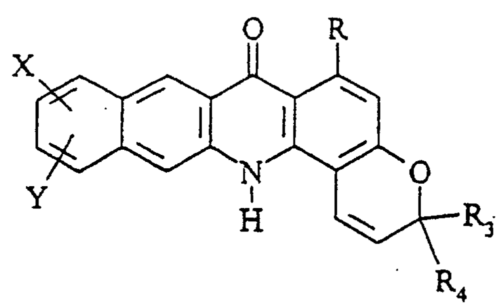

(I/a)

in der X, Y, R, $R_3$ und $R_4$ die oben angegebenen Bedeutungen besitzen,

bei welchen Verbindungen der Formel (I/a)

man das Stickstoffatom gegebenenfalls durch Einwirkung eines Alkylhalogenids oder eines Dialkylsulfats in Gegenwart eines Deprotonierungsmittels, wie Natriumhydrid, in einem polaren aprotischen Lösungsmittel substituiert in der Weise, daß man die Verbindungen der Formel (I/b) erhält, einem Sonderfall der Verbindungen der Formel (I):

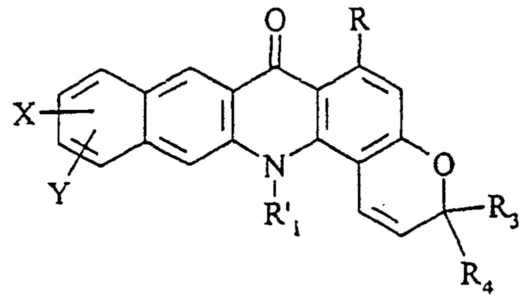

(I/b)

in der X, Y, R, $R_3$ und $R_4$ die oben angegebenen Bedeutungen besitzen und $R'_1$ eine geradkettige oder verzweigte ($C_1$-$C_6$)-Alkylgruppe darstellt,

welche Verbindungen der Formel (I/b):

man der Einwirkung eines Alkylierungsmittels, wie eines Dialkylsulfats, eines Acylierungsmittels, wie Essigsäureanhydrid, unterziehen kann oder unter den Bedingungen der Friedel-Crafts-Reaktion behandeln kann zur Bildung der Verbindungen der Formel (I/c), einem Sonderfall der Verbindungen der Formel (I):

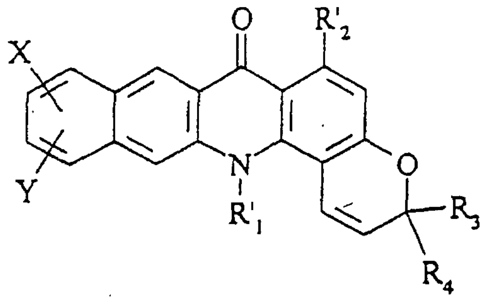

(I/c)

in der X, Y, $R'_1$, $R_3$ und $R_4$ die oben angegebenen Bedeutungen besitzen und $R'_2$ eine Alkoxygruppe, die gegebenenfalls durch eine Gruppe der Formel $NR_7R_8$, wie sie oben bezüglich der Formel (I) definiert worden ist, substituiert ist, oder eine geradkettige oder verzweigte ($C_1$-$C_6$)-Alkylcarbonyloxygruppe darstellt,

welche Verbindungen der Formel (I/c):

gegebenenfalls dann, wenn $R'_2$ eine Alkoxygruppe darstellt, beispielsweise mit einem Aminoderivat der Formel (VIII) behandelt werden kann:

$$HNR_aR_b \qquad \text{(VIII)}$$

in der $R_a$ ein Wasserstoffatom oder eine geradkettige oder verzweigte ($C_1$-$C_6$)-Alkylgruppe und $R_b$ eine Alkylgruppe, eine Gruppe $R_9$-$NR_7R_8$ (worin $R_9$ eine geradkettige oder verzweigte ($C_1$-$C_6$)-Alkylengruppe darstellt und $R_7$ und $R_8$, die gleichartig oder verschieden sind, ein Wasserstoffatom, eine geradkettige oder verzweigte ($C_1$-$C_6$)-Alkylgruppe oder eine geradkettige oder verzweigte ($C_1$-$C_6$)-Hydroxyalkylgruppe bedeuten), eine Heterocycloalkylengruppe (wobei die Begriffe Alkylen und Heterocyclus die bezüglich der Formel (I) angegebenen Bedeutungen besitzen) oder eine Gruppe

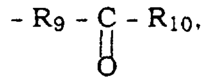

worin $R_9$ und $R_{10}$ die bezüglich der Formel (I) angegebenen Bedeutungen besitzen, darstellen, zur Bildung der Verbindungen der Formel (I/d), einem Sonderfall der Verbindungen der Formel (I):

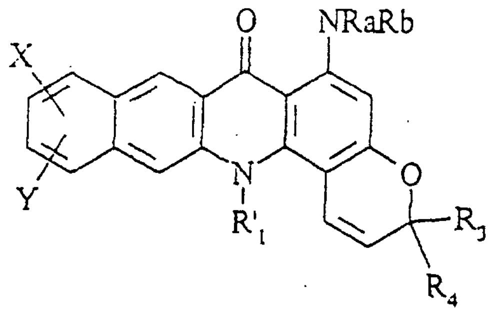

(I/d)

in der X, Y, $R'_1$, $R_3$, $R_4$, Ra und Rb die oben angegebenen Bedeutungen besitzen, wobei die Gesamtheit der Verbindungen der Formeln (I/a) bis (I/d) die Verbindungen der Formel (I/e) bilden:

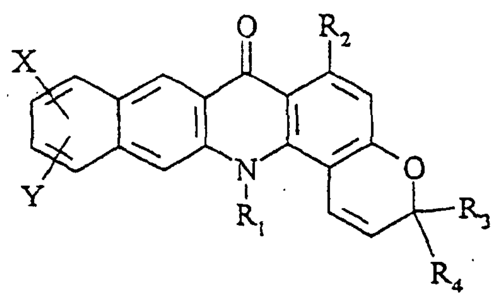

(I/e)

in der X, Y, $R_1$, $R_2$, $R_3$ und $R_4$ die gleichen Bedeutungen besitzen wie bei der allgemeinen Definition der Formel (I),
welche Verbindungen der Formel (I/e) man

a) ⇨ entweder der Einwirkung eines Reduktionsmittels unterwerfen kann zur Bildung der Verbindungen der Formel (I/f), einem Sonderfall der Verbindungen der Formel (I):

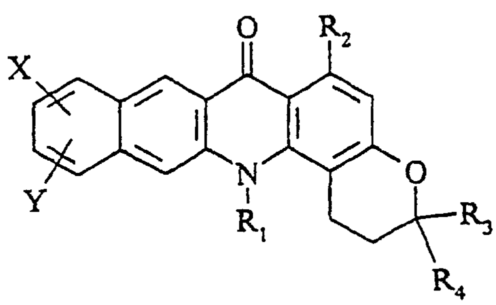

(I/f)

in der X. Y, $R_1$, $R_2$, $R_3$ und $R_4$ die oben angegebenen Bedeutungen besitzen,
b) ⇨ oder der Einwirkung von Osmiumtetroxid in einem polaren Medium in Gegenwart von 4-Methylmorpholin-N-oxid unterwirft zur Bildung der Verbindungen der Formeln (I/g) und (I/g'), Sonderfällen der Verbindungen der Formel (I):

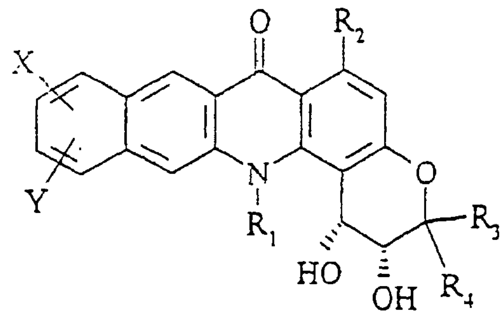

(I/g)

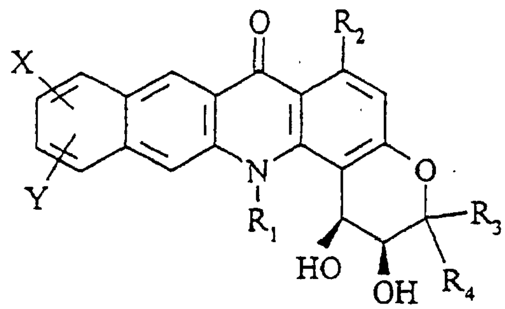

(I/g')

worin X, Y, $R_1$, $R_2$, $R_3$ und $R_4$ die oben angegebenen Bedeutungen besitzen,
wobei man die Verbindungen der Formeln (I/g) und (I/g') auch getrennt durch chirale Synthese erhalten kann, insbesondere durch asymmetrische *cis*-Dihydroxylierung ausgehend von der Verbindung (I/e) unter Anwendung chiraler Liganden des Pyridin- oder des Phthalazin-Typs, die durch *Cinchona*-Alkaloide, wie Dihydrochinin und sein rechtsdrehendes Diastereoisomeres das Dihydrochinidin disubstituiert sind,
wobei die Gesamtheit der Verbindungen der Formeln (I/g) und (I/g') die *cis*-Diolverbindungen der Formel (*cis*-I/h) bilden:

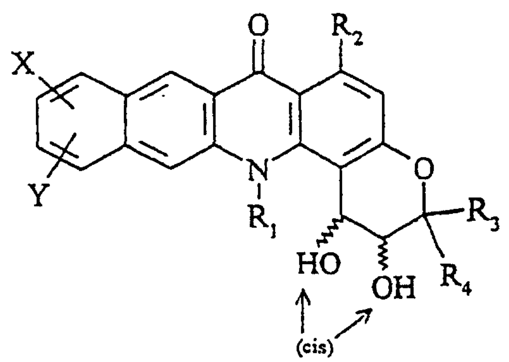

(cis-I/h)

in der X, Y, $R_1$, $R_2$, $R_3$ und $R_4$ die oben angegebenen Bedeutungen besitzen.
welche *cis*-Diolverbindungen der Formel (*cis*-I/h) man gegebenenfalls der Einwirkung von N,N'-Carbonyldiimidazol oder von N,N'-Thiocarbonyldiimidazol in Gegenwart von 2-Butanon unterwirft zur Bildung der Verbindungen der Formel (*cis*-I/i), einem Sonderfall der Verbindungen der Formel (I):

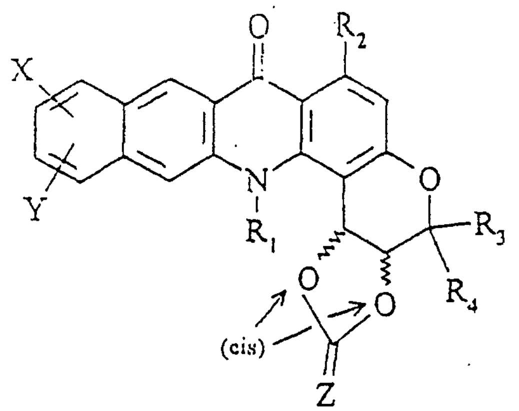

(cis I/i)

in der X, Y, $R_1$, $R_2$, $R_3$ und $R_4$ die oben angegebenen Bedeutungen besitzen und Z ein Sauerstoffatom oder ein Schwefelatom darstellt,

c) ⇨ oder der Einwirkung von Kaliumcarbonat in polarem Medium unterwirft zur Bildung der Verbindungen der Formel (IX):

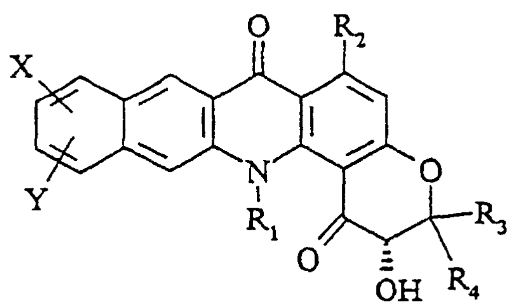

(IX)

in der X, Y, $R_1$, $R_2$, $R_3$ und $R_4$ die oben angegebenen Bedeutungen besitzen,

welche man reduzierenden Bedingungen beispielsweise in Gegenwart von $NaBH_4$ unterwirft zur Bildung der Verbindungen der Formel (I/j), einem Sonderfall der Verbindungen der Formel (I):

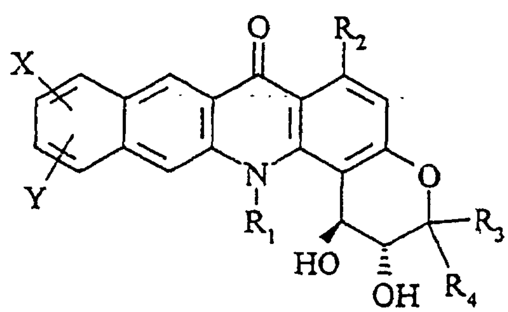

(I/j)

in der X. Y, $R_1$, $R_2$, $R_3$ und $R_4$ die oben angegebenen Bedeutungen besitzen,

wobei die Gesamtheit der Verbindungen der Formeln (I/j) und (*cis*-I/h) die Verbindungen der Formel (I/k) bilden:

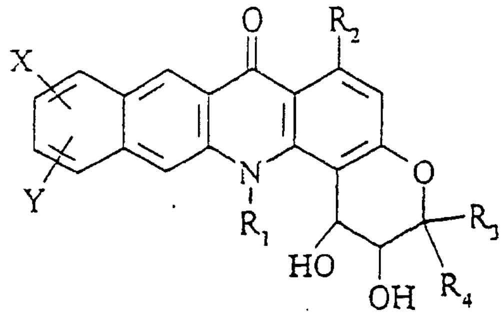

(I/k)

in der X, Y, $R_1$, $R_2$, $R_3$ und $R_4$ die oben angegebenen Bedeutungen besitzen,

welche Verbindungen der Formel (I/k) man:

- entweder der Einwirkung einer Verbindung der Formel (X) oder (XI):

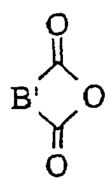

(X)

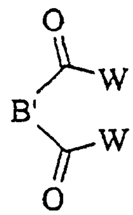

(XI)

worin B' die bezüglich der Formel (I) angegebenen Bedeutungen besitzt und W ein Halogenatom oder eine Hydroxygruppe darstellt, unterwirft,
zur Bildung der Verbindungen der Formel (I/1):

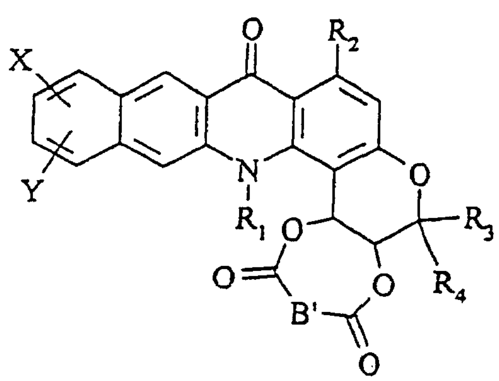

(I/l)

einem Sonderfall der Verbindungen der Formel (I), worin B', X, Y, $R_1$, $R_2$, $R_3$ und $R_4$ die oben angegebenen Bedeutungen besitzen,

- oder der Einwirkung eines geradkettigen ($C_1$-$C_6$)-Alkyldihalogenids unterwirft zur Bildung der Verbindungen der Formel (I/m), einem Sonderfall der Verbindungen der Formel (I):

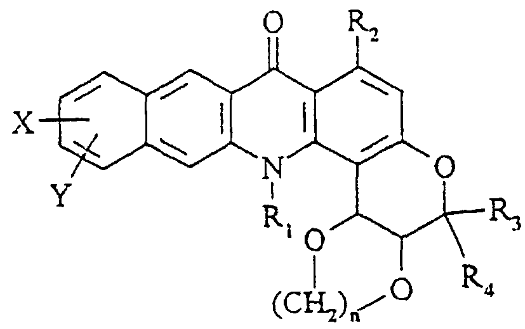

(I/m)

in der X, Y, $R_1$, $R_2$, $R_3$, $R_4$ und n die oben angegebenen Bedeutungen besitzen, ❖ oder der Einwirkung eines Alkohols der Formel $R_{11}$-OH, worin $R_{11}$ eine geadkettige oder verzweigte ($C_1$-$C_6$)-Alkylgruppe darstellt, unterwirft,
zur Bildung der Verbindungen der Formel (I/n):

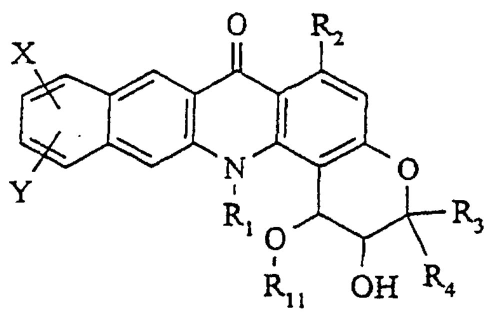

(I/n)

einem Sonderfall der Verbindungen der Formel (I), in der X, Y, $R_1$, $R_2$, $R_3$, $R_4$ und $R_{11}$ die oben angegebenen Bedeutungen besitzen,
bei welchen Verbindungen der Formel (I/n) man die Alkoholfunktion in Gegenwart einer schwachen Base, wie Pyridin, mit einem Anhydrid der Formel $(R_{12}CO)_2O$, worin $R_{12}$ eine geradkettige oder verzweigte ($C_1$-$C_6$)-Alkylgruppe oder eine Arylgruppe, wie sie oben definiert worden ist, darstellt, verestert,
zur Bildung der Verbindungen der Formel (I/o), einem Sonderfall der Verbindungen der Formel (I):

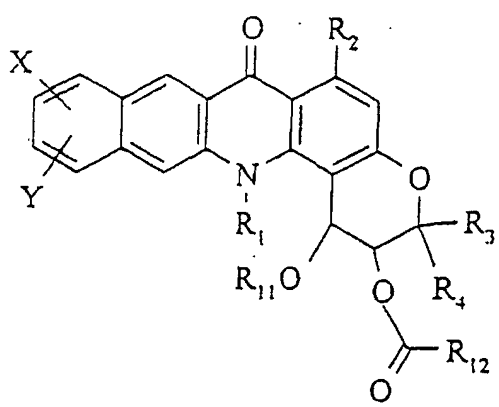

(I/o)

in der X, Y, $R_1$, $R_2$, $R_3$, $R_4$, $R_{11}$ und $R_{12}$ die oben angegebenen Bedeutungen besitzen,
❖ oder der Einwirkung eines Alkyliodids der Formel $R'_{11}$-I, worin $R'_{11}$ eine geradkettige oder verzweigte ($C_1$-$C_6$)-Alkylgruppe darstellt, in Gegenwart eines Silbersalzes unterwirft zur Bildung der Verbindungen der Formel (I/p):

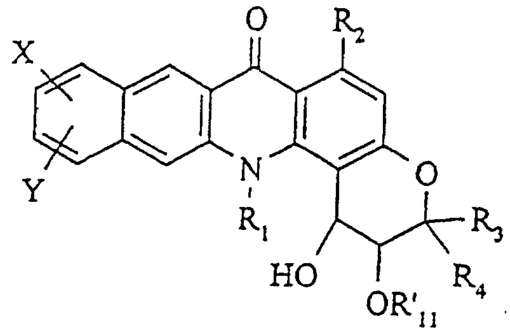

(I/p)

einem Sonderfall der Verbindungen der Formel (I), worin X, Y, $R_1$, $R_2$, $R_3$, $R_4$ und $R'_{11}$ die oben angegebenen Bedeutungen besitzen,
bei welchen Verbindungen der Forirel (I/p) man die Alkoholfunktion mit einem Anhydrid der Formel $(R'_{12}CO)_2O$, worin $R'_{12}$ eine geradkettige oder verzweigte ($C_1$-$C_6$)-Alkylgruppe oder eine Arylgruppe, wie sie oben definiert worden ist, bedeutet, verestert zur Bildung der Verbindungen der Formel (I/q):

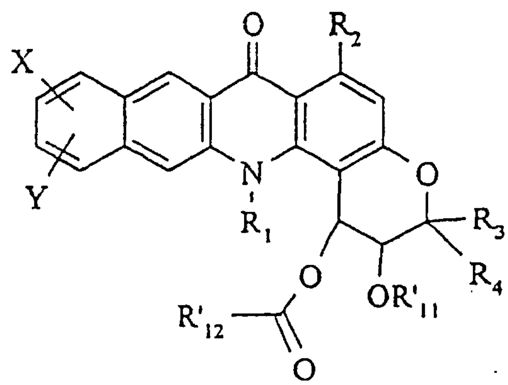

(I/q)

einem Sonderfall der Verbindungen der Formel (I), in der X, Y, $R_1$, $R_2$, $R_3$, $R_4$, $R'_{11}$ und $R'_{12}$ die oben angegebenen Bedeutungen besitzen,
❖ oder der direkten Einwirkung eines Anhydrids der Formel $(R_{12}CO)_2O$ in Gegenwart einer Base, wie Triethylamin, unterwirft zur Bildung der Verbindungen der Formel (I/r), einem Sonderfall der Verbindungen der Formel (I):

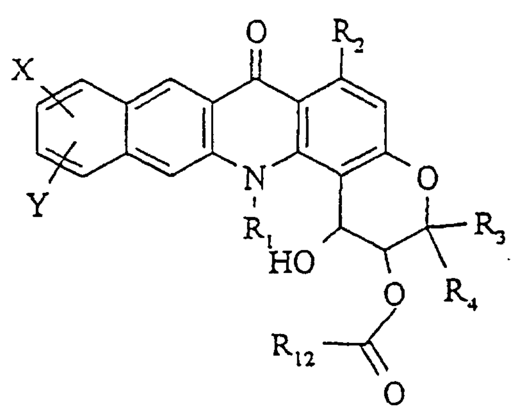

(I/r)

in der X, Y. $R_1$, $R_2$, $R_3$, $R_4$ und $R_{12}$ die oben angegebenen Bedeutungen besitzen.
welche man erneut unter den gleichen Verfahrensbedingungen der Einwirkung des Anhydrids der Formel $(R'_{12}CO)_2O$ unterwerfen kann zur Bildung der Verbindungen der Formel (I/s):

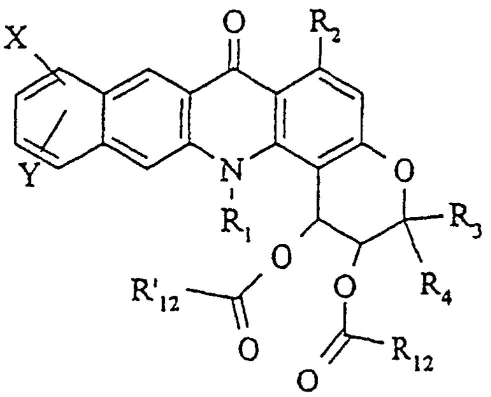

(I/s)

in der X, Y, $R_1$, $R_2$, $R_3$, $R_4$, $R_{12}$ und $R'_{12}$ die oben angegebenen Bedeutungen besitzen, wobei die beiden Gruppen $R_{12}$ und $R'_{12}$ gleichartig oder verschieden sein können,

❖ oder den Bedingungen der Dehydratation in einem sauren Medium unterwirft zur Bildung der Verbindungen der Formel (XII):

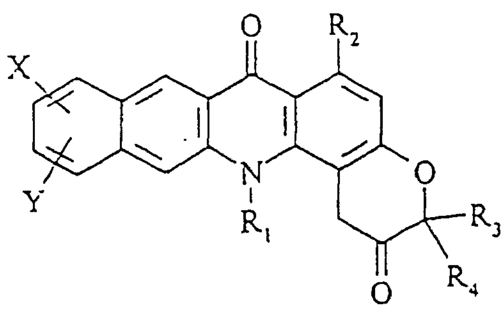

(XII)

in der X, Y, $R_1$, $R_2$, $R_3$ und $R_4$ die oben angegebenen Bedeutungen besitzen, welche man in Gegenwart von $NaBH_4$ reduziert zur Bildung der Verbindungen der Formel (I/t):

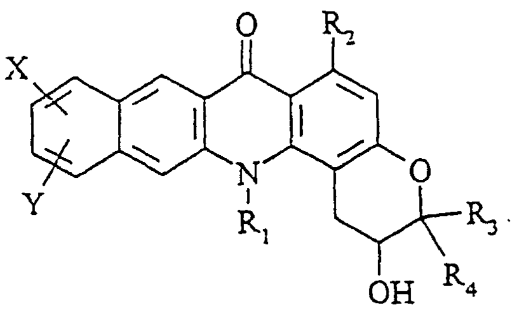

(I/t)

einem Sonderfall der Verbindungen der Formel (I), worin X, Y, $R_1$, $R_2$, $R_3$ und $R_4$ die oben angegebenen Bedeutungen besitzen,

d) ⇨ oder der Einwirkung einer Persäure, wie m-Chlorperbenzoesäure, unterwirft zur Bildung der Verbindungen der Formel (I/u), einem Sonderfall der Verbindungen der Formel (I):

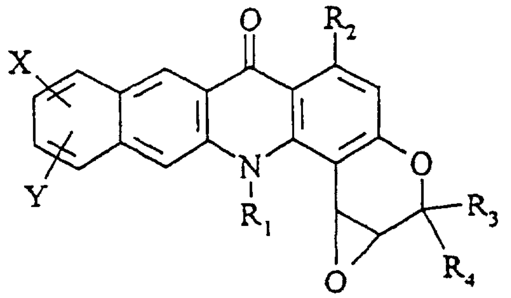

(I/u)

in der X, Y, $R_1$, $R_2$, $R_3$ und $R_4$ die oben angegebenen Bedeutungen besitzen, welche Verbindung der Formel (I/u) man gegebenenfalls mit Ammoniak oder einem primären oder sekundären Amin umsetzt, so daß man in Abhängigkeit von der Art der Reaktionsteilnehmer die Verbindungen der Formel (I/v) und/oder (I/v') erhält, einem Sonderfall der Verbindungen der Formel (I):

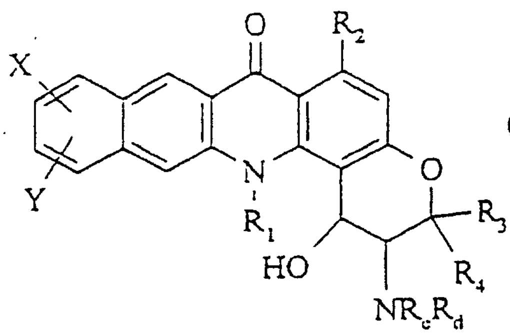

(I/v)

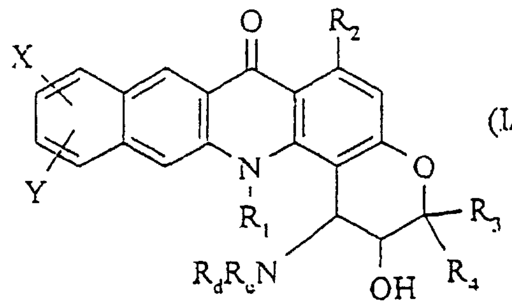

(I/v')

worin X, Y, $R_1$, $R_2$, $R_3$ und $R_4$ die oben angegebenen Bedeutungen besitzen und $R_c$ und $R_d$ Wasserstoffatome oder geradkettige oder verzweigte ($C_1$-$C_6$)-Alkylgruppen bedeuten,
welche Verbindung der Formel (I/v') man auch direkt erhalten kann ausgehend von den Verbindungen der Formel (I/k) durch Behandeln dieser Verbindungen mit $NaN_3$, was zu Azid-Zwischenprodukten führt, gefolgt von einer Hydrierung in Gegenwart von Palladium, was dann zu den Verbindungen der Formel (I/v') führt, in der Rc und Rd Wasserstoffatome bedeuten,

welche Verbindungen der Formeln (I/v) und (I/v'):

- entweder dann, wenn Rc und Rd Wasserstoffatome bedeuten, der Einwirkung von beispielsweise N, N'-Carbonyldiimidazol oder N,N'-Thiocarbonyldiimidazol unterworfen werden können zur Bildung der Verbindungen der Formel (I/w) bzw. (I/w'), einem Sonderfall der Verbindungen der Formel (I):

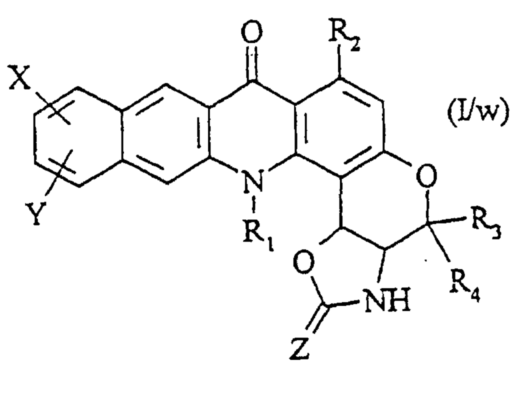

(I/w)

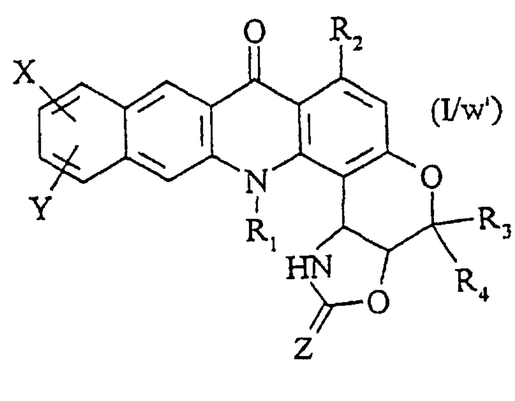

(I/w')

worin X, Y, $R_1$, $R_2$, $R_3$ und $R_4$ die oben angegebenen Bedeutungen besitzen und Z in Abhängigkeit von der Art des verwendeten Reagens ein Sauerstoffatom oder ein Schwefelatom darstellt,

❖ oder der Einwirkung einer Verbindung der Formel (XI) $WO_2C\text{-}B'\text{-}CO_2W$, wie sie oben definiert worden ist, unterworfen werden können zur Bildung der Verbindungen der Formel (I/x) bzw. (I/x'), einem Sonderfall der Verbindungen der Formel (I):

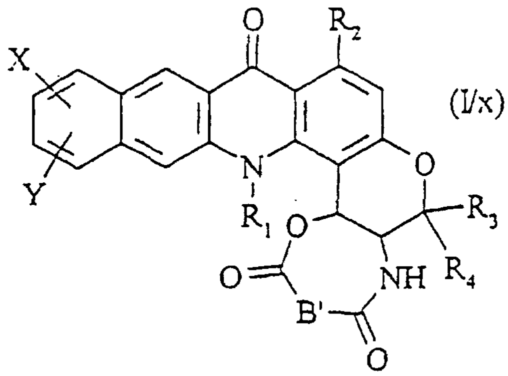

(I/x)

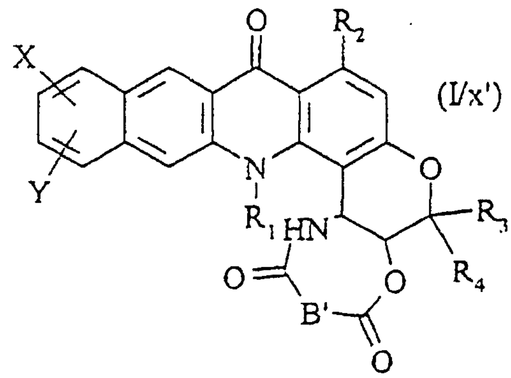

(I/x')

worin B', X, Y, $R_1$, $R_2$, $R_3$ und $R_4$ die oben angegebenen Bedeutungen besitzen,

❖ oder der Einwirkung von Triphenylphosphindibromid in Gegenwart von Triethylamin unterworfen werden können zur Bildung der Verbindungen der Formel (I/y), einem Sonderfall der Verbindungen der Formel (I):

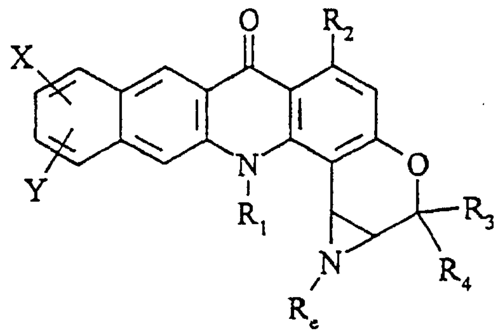

(I/y)

in der X, Y, $R_1$, $R_2$, $R_3$ und $R_4$ die oben angegebenen Bedeutungen besitzen und $R_c$ ein Wasserstoffatom oder eine geradkettige oder verzweigte ($C_1$-$C_6$)-Alkylgruppe bedeutet,

e) ⇨ oder der Einwirkung von $NaN_3$ in Gegenwart von Wasserstoffperoxid gefolgt von einer Reduktion mit beispielsweise Tri-n-butylzinnhydrid unterwirft zur Bildung der Verbindungen der Formel (I/z), einem Sonderfall der Verbindungen der Formel (I):

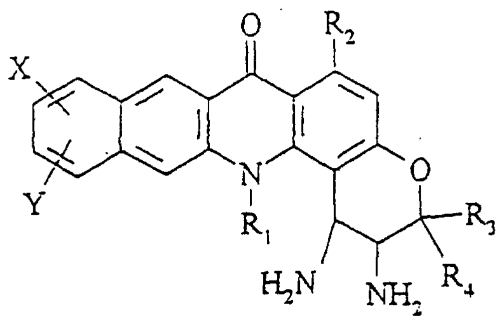

(I/z)

in der X, Y, $R_1$, $R_2$, $R_3$ und $R_4$ die oben angegebenen Bedeutungen besitzen, welche Verbindungen der

Formel (I/z) gegebenenfalls der Einwirkung von Kohlendioxid in Gegenwart von Diphenylphosphit unterworfen werden können zur Bildung der Verbindungen der Formel (I/aa), einem Sonderfall der Verbindungen der Formel (I):

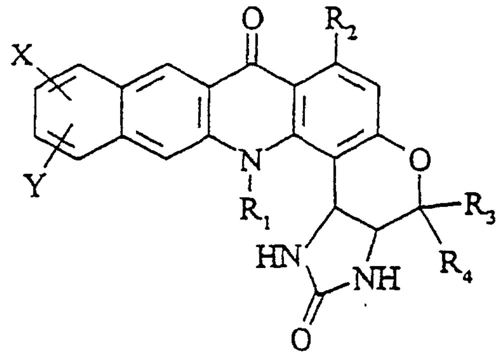

(I/aa)

in der X, Y, $R_1$, $R_2$, $R_3$ und $R_4$ die oben angegebenen Bedeutungen besitzen,

wobei die Verbindungen (I/a-z), (I/g'), (I/v'-x'), (I/aa) und (*cis* I/h-I/i) die Gesamtheit der erfindungsgemäßen Verbindungen bilden, welche man gegebenenfalls mit Hilfe einer klassischen Reinigungsmethode reinigt, welche gewünschtenfalls mit Hilfe einer klassischen Trennmethode in ihre verschiedenen optischen Isomeren getrennt werden können, welche gewünschtenfalls in ihre N-Oxide umgewandelt werden können und welche gegebenenfalls mit Hilfe einer pharmazeutisch annehmbaren Säure oder Base in ihre Additionssalze umgewandelt werden können.

**12.** Pharmazeutische Zubereitungen enthaltend als Wirkstoff mindestens eine Verbindung nach einem der Ansprüche 1 bis 10 allein oder in Kombination mit einem oder mehreren inerten, nichttoxischen, pharmazeutisch annehmbaren Trägermaterialien oder Bindemitteln.

**13.** Pharmazeutische Zubereitungen nach Anspruch 12, die zur Behandlung von Krebs geeignet sind, enthaltend als Wirkstoff mindestens eine Verbindung nach einem der Ansprüche 1 bis 10.

## Claims

**1.** Compound of formula (I):

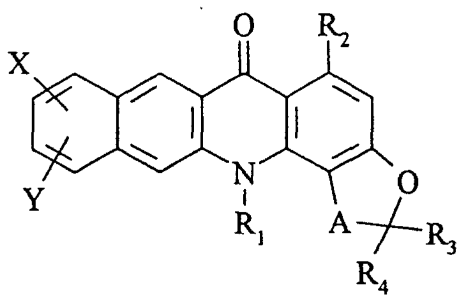

(I)

in which:

X and Y, which may be identical or different, each independently of the other represents a hydrogen atom, a halogen atom, a hydroxy group, a mercapto group, a cyano group, a nitro group, a linear or branched ($C_1$-$C_6$)alkyl group, a linear or branched ($C_1$-$C_6$)-alkoxy group, a linear or branched ($C_1$-$C_6$)trihaloalkyl group, an amino group (optionally substituted by one or two identical or different linear or branched ($C_1$-$C_6$)alkyl groups which are themselves optionally substituted by a linear or branched ($C_1$-$C_6$)-alkoxy group or by a group of the formula $-NR'_7R'_8$ wherein $R'_7$ and $R'_8$, which may be identical or different, each independently of the other represents a hydrogen atom or a linear or

branched ($C_1$-$C_6$)alkyl group), or X and Y together form a methylenedioxy group or an ethylenedioxy group,

$R_1$ represents a hydrogen atom or a linear or branched ($C_1$-$C_6$)alkyl group,

$R_2$ represents:

- a hydrogen atom,
- a hydroxy group,
- a linear or branched ($C_1$-$C_6$)alkyl group,
- a linear or branched ($C_1$-$C_6$)alkoxy group which is optionally substituted:
  * by a group of the formula $NR_7R_8$ wherein $R_7$ and $R_8$, which may be identical or different, each independently of the other represents a hydrogen atom, a linear or branched ($C_1$-$C_6$) alkyl group or a linear or branched ($C_1$-$C_6$)hydroxyalkyl group,
  * or by a saturated or unsaturated, monocyclic or bicyclic heterocycle having from 5 to 7 ring members and containing one or two hetero atoms selected from oxygen, nitrogen and sulphur,
- a linear or branched ($C_1$-$C_6$)alkylcarbonyloxy group,
- or an amino group which is optionally substituted:
  * by one or two identical or different linear or branched ($C_1$-$C_6$)alkyl groups,
  * by a linear or branched ($C_1$-$C_6$)alkylcarbonyl group which is optionally substituted by a $-NR_7R_8$ group wherein $R_7$ and $R_8$ are as defined above,
  * by a group of the formula $-R_9-NR_7R_8$ wherein $R_9$ represents a linear or branched ($C_1$-$C_6$) alkylene group and $R_7$ and $R_8$, which may be identical or different, each independently of the other represents a hydrogen atom, a linear or branched ($C_1$-$C_6$)alkyl group or a linear or branched ($C_1$-$C_6$)hydroxyalkyl group,
  * by a linear or branched ($C_1$-$C_6$)alkylene group which is substituted by a saturated or unsaturated, monocyclic or bicyclic heterocycle having from 5 to 7 ring members and containing one or two hetero atoms selected from oxygen, nitrogen and sulphur,
  * or by a group of the formula

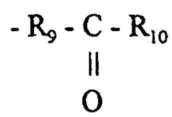

wherein $R_9$ is as defined above and $R_{10}$ represents a hydroxy group or a linear or branched ($C_1$-$C_6$)alkoxy group,

$R_3$ and $R_4$, which may be identical or different, each independently of the other represents a hydrogen atom or a linear or branched ($C_1$-$C_6$)alkyl group,

A represents a -CH=CH- group or an ethylene group of the formula $-CH(R_5)-CH(R_6)$ wherein $R_5$ and $R_6$, which may be identical or different, each independently of the other represents:

- a hydrogen atom,
- a hydroxy group,
- a linear or branched ($C_1$-$C_6$)alkoxy group,
- a linear or branched ($C_1$-$C_6$)alkylcarbonyloxy group,
- an arylcarbonyloxy group,
- an amino group optionally substituted by one or two identical or different linear or branched ($C_1$-$C_6$)alkyl groups or by a linear or branched ($C_1$-$C_6$)acyl group,
- a mercapto group, a linear or branched ($C_1$-$C_6$)alkylthio group or an arylthio group,

or $R_5$ and $R_6$ together form:

- a

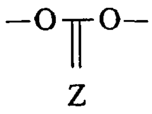, 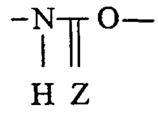 or 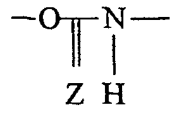

group
wherein Z represents an oxygen atom or a sulphur atom,
- a $-O-(CH_2)_n-O-$ group wherein n is an integer from 1 to 4 inclusive,
- a

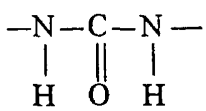

group,
- or a

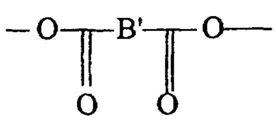, 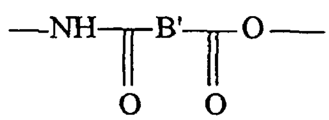

or

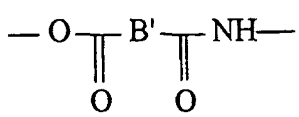

group wherein B' represents a single bond, a linear or branched ($C_1$-$C_6$)alkylene group or a linear or branched ($C_1$-$C_6$)alkenylene group,

or $R_5$ and $R_6$ form together with the carbon atoms carrying them an oxirane group or an aziridine group, optionally substituted on the nitrogen atom by a linear or branched ($C_1$-$C_6$)alkyl group,

wherein the term "aryl" denotes a phenyl or naphthyl group optionally containing one or more identical or different substituents selected from hydroxy, halogen, carboxy, nitro, amino, linear or branched ($C_1$-$C_6$)alkylamino, linear or branched di-($C_1$-$C_6$)alkylamino, linear or branched ($C_1$-$C_6$)alkoxy, linear or branched ($C_1$-$C_6$)acyl and linear or branched ($C_1$-$C_6$)alkylcarbonyloxy,
their optical isomers, N-oxides, and addition salts thereof with a pharmaceutically acceptable acid or base.

2. Compounds of formula (I) according to claim 1, **characterised in that** $R_3$ and $R_4$, which may be identical or different, represent a linear or branched ($C_1$-$C_6$)alkyl group, their optical isomers, N-oxides, and addition salts thereof with a pharmaceutically acceptable acid or base.

3. Compounds of formula (I) according to claim 1, **characterised in that** $R_2$ represents a linear or branched ($C_1$-$C_6$) alkoxy group, or an amino group optionally substituted by one or two substituents as defined in claim 1, their optical isomers, N-oxides, and addition salts thereof with a pharmaceutically acceptable acid or base.

4. Compounds of formula (I) according to either claim 1 or claim 3, **characterised in that** $R_2$ represents an amino group substituted by a group of the formula $-R_9-NR_7R_8$ wherein $R_9$ represents a linear or branched ($C_1$-$C_6$)alkylene group and $R_7$ and $R_8$, which may be identical or different, represent a hydrogen atom or a linear or branched

($C_1$-$C_6$)alkyl group, their optical isomers, N-oxides, and addition salts thereof with a pharmaceutically acceptable acid or base.

5. Compounds of formula (I) according to claim 1, **characterised in that** A represents a -CH=CH- group or a group of the formula -CH($R_5$)-CH($R_6$)- wherein $R_5$ and $R_6$ represent a hydroxy group or a linear or branched ($C_1$-$C_6$) alkylcarbonyloxy group, or $R_5$ and $R_6$ together form a

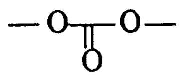

group,
their optical isomers, N-oxides, and addition salts thereof with a pharmaceutically acceptable acid or base.

6. Compound of formula (I) according to any one of claims 1 to 5 which is (±)-*cis*-1,2-diacetoxy-6-methoxy-3,3,14-trimethyl-2,3,7,14-tetrahydro-1H-benzo[b]pyrano[3,2-h]-acridin-7-one.

7. Compound of formula (I) according to any one of claims 1 to 5 which is *cis*-7-methoxy-4,4,15-trimethyl-3a,8,15,15c-tetrahydro-4H-benzo[b][1,3]dioxolo[4',5':4,5]pyrano[3,2-h]-acridine-2,8-dione.

8. Compound of formula (I) according to any one of claims 1 to 5 which is 6-methoxy-3,3,14-trimethyl-7,14-dihydro-3H-benzo[b]pyrano[3,2-h]acridin-7-one.

9. Compound of formula (I) according to any one of claims 1 to 5 which is 6-(diethylaminopropylamino)-3,3,14-trimethyl-7,14-dihydro-3H-benzo[b]pyrano[3,2-h]acridin-7-one.

10. Compound of formula (I) according to any one of claims 1 to 5 which is (±)-*cis*-diacetoxy-6-(diethylaminopropylamino)-3,3,14-trimethyl-2,3,7,14-tetrahydro-1H-benzo[b]pyrano[3,2-h]acridin-7-one.

11. Process for the preparation of compounds of formula (I) according to claim 1, **characterised in that**:

- *either a 3-amino-2-naphthalenecarboxylic acid compound (II):*

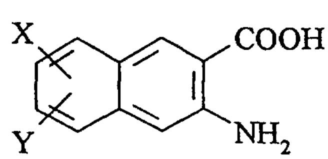

(II)

in which X and Y are as defined in formula (I),
is reacted with a phloroglucinol compound of formula (III):

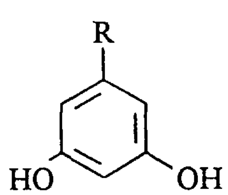

(III)

in which R represents a hydrogen atom, a hydroxy group or a linear or branched ($C_1$-$C_6$)-alkyl group, to yield the compounds of formula (IV):

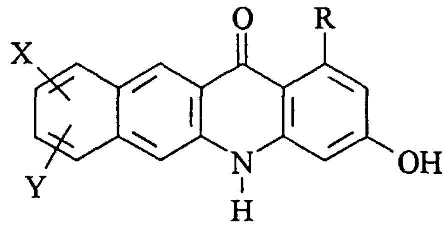

(IV)

in which X, Y and R are as defined above, which are then treated under basic conditions in an aprotic solvent, such as dimethylformamide, with an alkyne of formula (V):

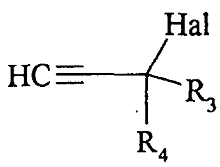

(V)

in which Hal represents a halogen atom and $R_3$ and $R_4$ are as defined in formula (I), to yield the compounds of formula (I/a), a particular case of the compounds of formula (I):

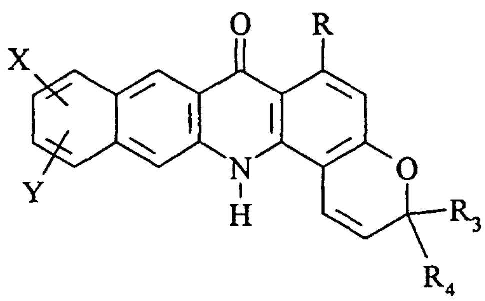

(I/a)

in which X, Y, R, $R_3$ and $R_4$ are as defined above,

- *or a 3-halo-2-naphthalenecarboxylic acid compound of formula (VI):*

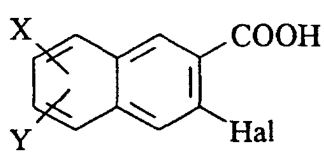

(VI)

in which X and Y are as defined in formula (I) and Hal represents a halogen atom, such as chlorine or bromine, is reacted with an amino-chromene compound of formula (VII):

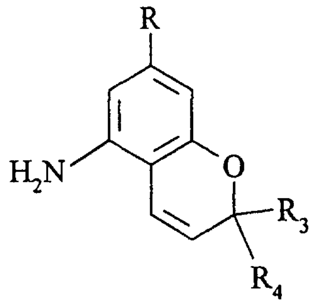

(VII)

in which $R_3$ and $R_4$ are as defined in formula (I) and R has the same meaning as above,
to yield likewise the compounds of formula (I/a), a particular case of the compounds of formula (I):

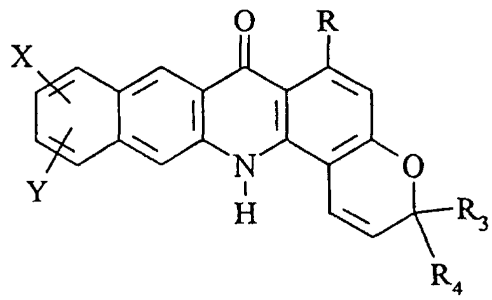

(I/a)

in which X, Y, R, $R_3$ and $R_4$ are as defined above,

<u>in which compounds of formula (I/a)</u>
the nitrogen atom is optionally substituted by the action of an alkyl halide or a dialkyl sulphate in the presence of a deprotonating agent, such as sodium hydride, in a polar aprotic solvent, in such a manner as to obtain the compounds of formula (I/b), a particular case of the compounds of formula (I):

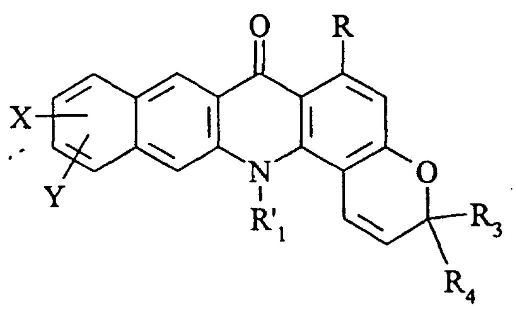

(I/b)

in which X, Y, R, $R_3$ and $R_4$ are as defined above and $R'_1$ represents a linear or branched $(C_1\text{-}C_6)$alkyl group,

<u>which compounds of formula (I/b)</u>
may be subjected to the action of an alkylating agent, such as a dialkyl sulphate, of an acylating agent, such as acetic anhydride, or may be treated under Friedel-Crafts reaction conditions to yield the compounds of formula (I/c), a particular case of the compounds of formula (I):

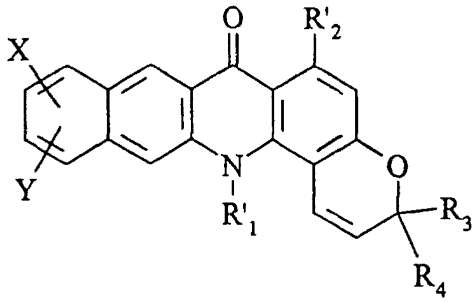

(I/c)

in which X, Y, R'$_1$, $R_3$ and $R_4$ are as defined above and R'$_2$ represents an alkoxy group optionally substituted by a group of the formula $NR_7R_8$ as defined in formula (I), or R'$_2$ represents linear or branched ($C_1$-$C_6$)alkyl-carbonyloxy,

<u>which compound of formula (I/c)</u>

may optionally be treated, when R'$_2$ represents an alkoxy group, for example, with an amino compound of formula (VIII):

$$HNR_aR_b \quad (VIII)$$

in which $R_a$ represents a hydrogen atom or a linear or branched ($C_1$-$C_6$)alkyl group and $R_b$ represents an alkyl group, a $R_9$-$NR_7R_8$ group (wherein $R_9$ represents a linear or branched ($C_1$-$C_6$)alkylene group and $R_7$ and $R_8$, which may be identical or different, represent a hydrogen atom, a linear or branched ($C_1$-$C_6$)alkyl group or a linear or branched ($C_1$-$C_6$)-hydroxyalkyl group), a heterocycloalkylene group (the terms alkylene and heterocycle having the same meaning as in formula (I)), or a

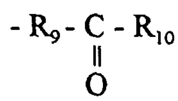

group wherein $R_9$ and $R_{10}$ are as defined in formula (I),

to yield the compounds of formula (I/d), a particular case of the compounds of formula (I):

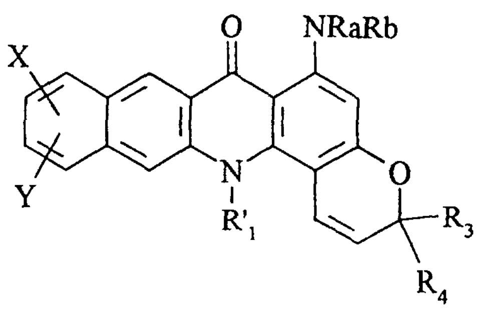

(I/d)

in which X, Y, R'$_1$, $R_3$, $R_4$, Ra and Rb are as defined above,

the totality of the compounds of formulae (I/a) to (I/d) forming the compound of formula (I/e):

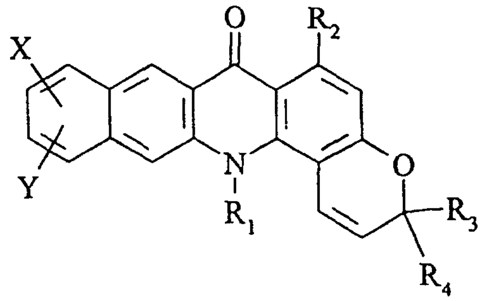

(I/e)

in which X, Y, $R_1$, $R_2$, $R_3$ and $R_4$ have the same meaning as in the general definition of formula (I),

which compound of formula (I/e) may be subjected

a) ⇨ either to the action of a reducing agent to yield the compounds of formula (I/f), a particular case of the compounds of formula (I):

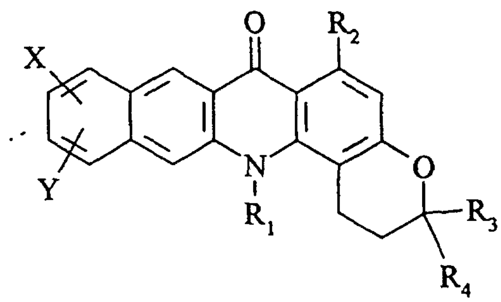

(I/f)

in which X, Y, $R_1$, $R_2$, $R_3$ and $R_4$ are as defined above,

b) ⇨ or to the action of osmium tetroxide in a polar medium and in the presence of 4-methylmorpholine N-oxide, to yield the compounds of formulae (I/g) and (I/g'), which are particular cases of the compounds of formula (I):

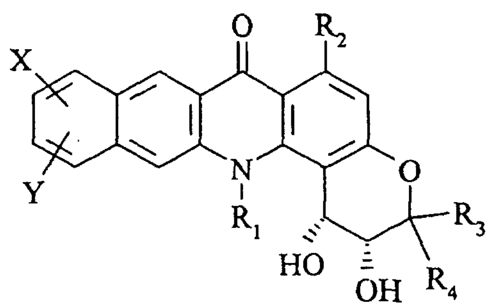

(I/g)

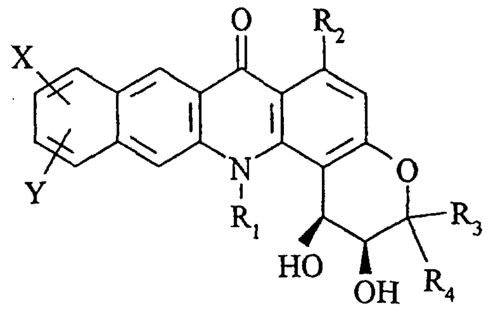

(I/g')

in which X, Y, $R_1$, $R_2$, $R_3$ and $R_4$ are as defined above,
which compounds of formulae (I/g) and (I/g') may also be obtained separately by chiral synthesis and, especially, by asymmetric *cis* dihydroxylation starting from a compound (I/e) using chiral ligands of the pyridine or phthalazine type disubstituted by *Cinchona* alkaloids, such as dihydroquinine and its dextrorotatory diastereoisomer dihydroquinidine,
the totality of the compounds of formulae (I/g) and (I/g') forming the *cis*-diol compounds of formula (*cis*-I/h) :

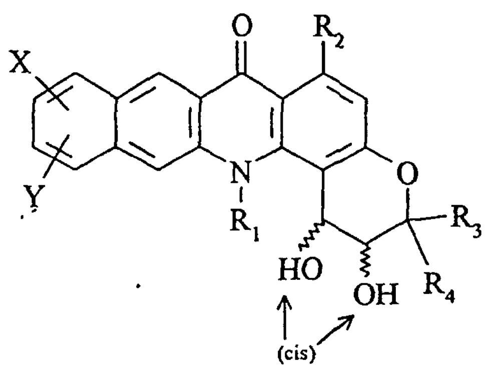


(cis-I/h)

in which X, Y, $R_1$, $R_2$, $R_3$ and $R_4$ are as defined above,
which cis-diol compounds of formula (*cis*-I/h) are optionally subjected to the action of N,N'-carbonyldiimidazole or N,N'-thiocarbonyldiimidazole in the presence of 2-butanone to yield the compounds of formula (*cis*-I/i), a particular case of the compounds of formula (I):

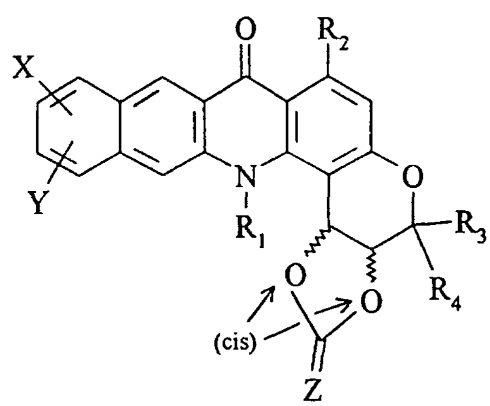


(cis I/i)

in which X, Y, $R_1$, $R_2$, $R_3$ and $R_4$ are as defined above and Z represents an oxygen atom or a sulphur atom,

c) ⇨ or to the action of potassium permanganate in a polar medium to yield the compounds of formula (IX):

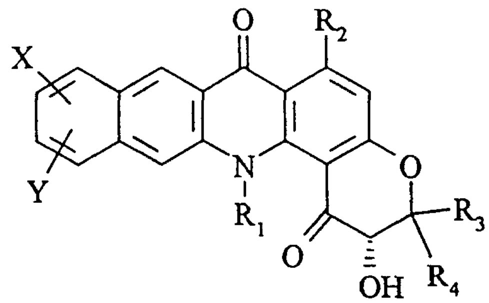

(IX)

in which X, Y, $R_1$, $R_2$, $R_3$ and $R_4$ are as defined above,
which are subjected to reducing conditions in the presence of $NaBH_4$, for example, to yield the compounds of formula (I/j), a particular case of the compounds of formula (I):

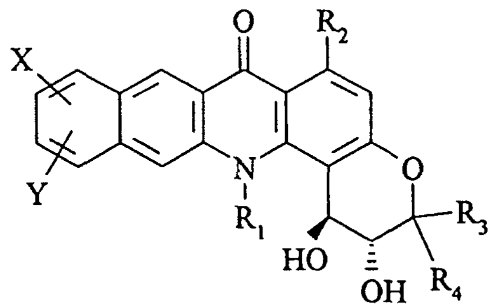

(I/j)

in which X, Y, $R_1$, $R_2$, $R_3$ and $R_4$ are as defined above, the totality of the compounds of formulae (I/j) and (*cis*-I/h) forming the compounds of formula (I/k):

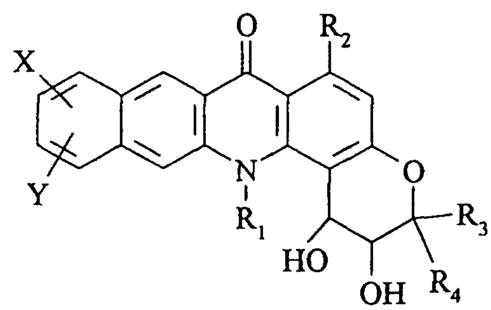

(I/k)

in which X, Y, $R_1$, $R_2$, $R_3$ and $R_4$ are as defined above,

which compounds of formula (I/k) are subjected:

- either to the action of a compound of formula (X) or (XI):

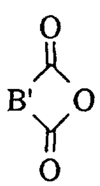

(X)

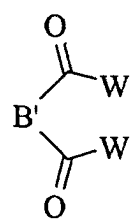

(XI)

in which B' is as defined in formula (I) and W represents a halogen atom or a hydroxy group,

to yield the compounds of formula (I/l):

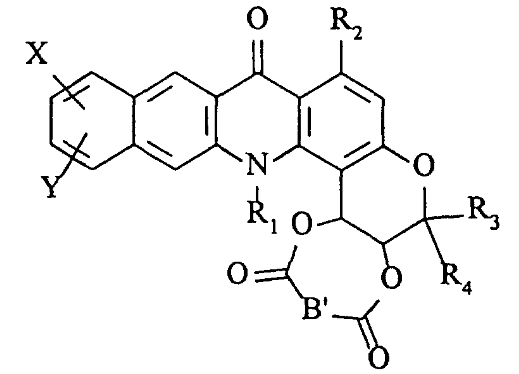

(I/l)

which is a particular case of the compounds of formula (I) in which B', X, Y, $R_1$, $R_2$, $R_3$ and $R_4$ are as defined above,

❖ or to the action of a linear ($C_1$-$C_6$)alkyl dihalide to yield the compounds of formula (I/m), a particular case of the compounds of formula (I):

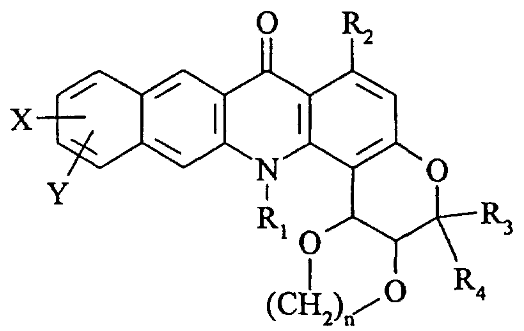

(I/m)

in which X, Y, $R_1$, $R_2$, $R_3$, $R_4$ and n are as defined above,

❖ or to the action of an alcohol of the formula $R_{11}$-OH wherein $R_{11}$ represents a linear or branched ($C_1$-$C_6$) alkyl group,
to yield the compounds of formula (I/n):

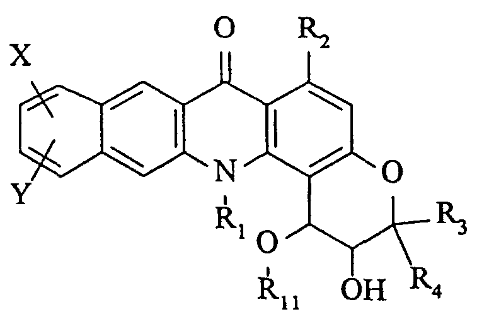

(I/n),

which is a particular case of the compounds of formula (I) in which X, Y, $R_1$, $R_2$, $R_3$, $R_4$ and $R_{11}$ are as defined above,
the alcohol function of which compounds of formula (I/n) is esterified in the presence of a weak base, such as pyridine, by an anhydride of the formula $(R_{12}CO)_2O$ wherein $R_{12}$ represents a linear or branched ($C_1$-$C_6$) alkyl group or an aryl group as defined above,
to yield the compounds of formula (I/o), a particular case of the compounds of formula (I):

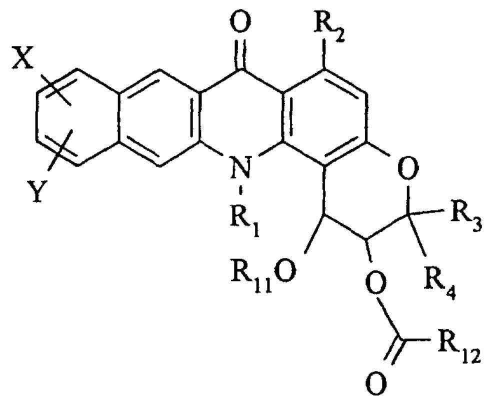

(I/o)

in which X, Y, $R_1$, $R_2$, $R_3$, $R_4$, $R_{11}$ and $R_{12}$ are as defined above,

❖ or to the action of an alkyl iodide of the formula $R'_{11}$-I wherein $R'_{11}$ represents a linear or branched ($C_1$-$C_6$)alkyl group, in the presence of silver salt, to yield the compounds of formula (I/p):

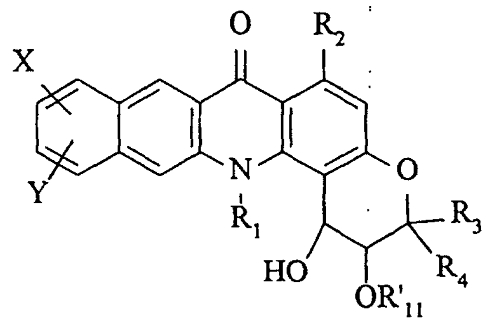

(I/p),

which is a particular case of the compounds of formula (I) in which X, Y, $R_1$, $R_2$, $R_3$, $R_4$ and $R'_{11}$ are as defined above,
the alcohol function of which compounds of formula (I/p) is esterified by an anhydride of the formula $(R'_{12}CO)_2O$ wherein $R'_{12}$ represents a linear or branched ($C_1$-$C_6$)alkyl group or an aryl group as defined above, to yield the compounds of formula (I/q):

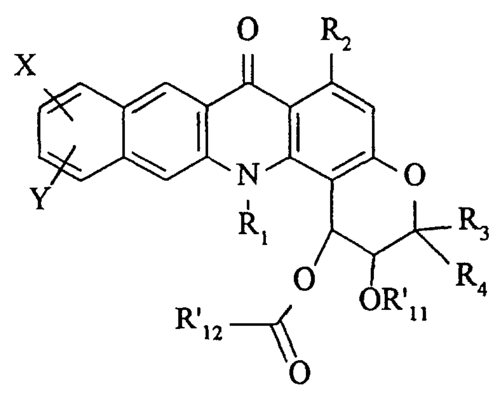

(I/q),

which is a particular case of the compounds of formula (I) in which X, Y, $R_1$, $R_2$, $R_3$, $R_4$, $R'_{11}$ and $R'_{12}$ are as defined above,

❖ or to the direct action of an anhydride of the formula $(R_{12}CO)_2O$ in the presence of a base, such as triethylamine, in order to obtain the compounds of formula (I/r), a particular case of the compounds of formula (I):

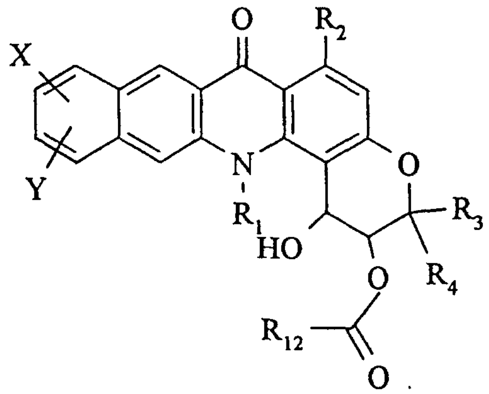

(I/r)

in which X, Y, $R_1$, $R_2$, $R_3$, $R_4$ and $R_{12}$ are as defined above,
which may again be subjected, under the same operating conditions, to the action of an anhydride of the formula $(R'_{12}CO)_2O$ to yield the compounds of formula (I/s):

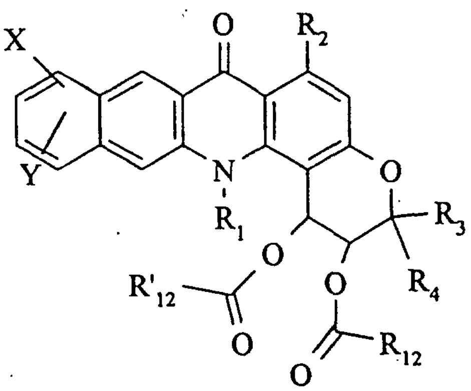

(I/s)

in which X, Y, $R_1$, $R_2$, $R_3$, $R_4$, $R_{12}$ and $R'_{12}$ are as defined above, the two groups $R_{12}$ and $R'_{12}$ being identical or different,

- or to dehydrating conditions in an acid medium to yield the compounds of formula (XII):

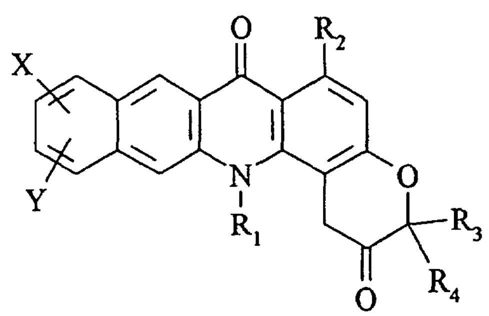

(XII)

in which X, Y, $R_1$, $R_2$, $R_3$ and $R_4$ are as defined above,
which are reduced in the presence of $NaBH_4$ to yield the compounds of formula (I/t):

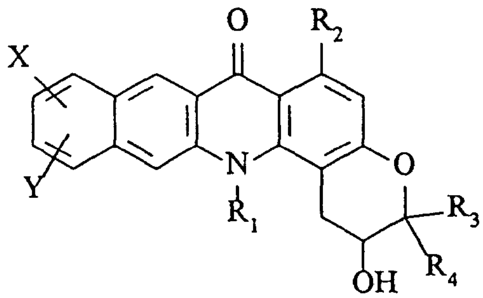

(I/t),

which is a particular case of the compounds of formula (I) in which X, Y, $R_1$, $R_2$, $R_3$ and $R_4$ are as defined above,

d) ⇨ or to the action of a peracid, such as m-chloroperbenzoic acid, to yield a compound of formula (I/u), a particular case of the compounds of formula (I):

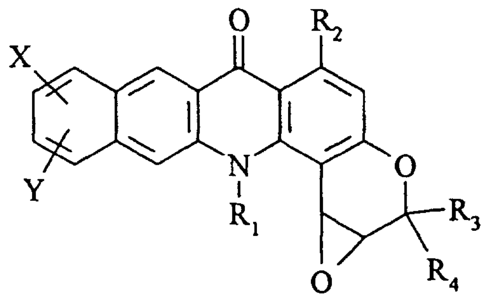

(I/u)

in which X, Y, $R_1$, $R_2$, $R_3$ and $R_4$ are as defined above, which compound of formula (I/u) is optionally treated with ammonium hydroxide or a primary or secondary amine to yield, according to the nature of the reagents, the compounds of formula (I/v) and/or (I/v'), which are particular cases of the compounds of formula (I):

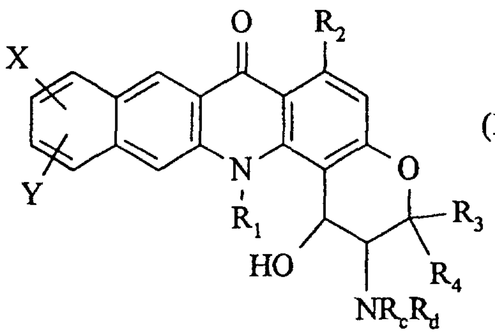

(I/v)

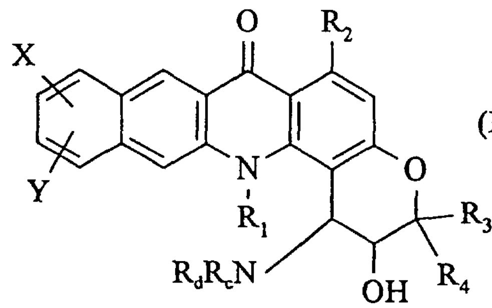

(I/v')

in which X, Y, $R_1$, $R_2$, $R_3$ and $R_4$ are as defined above and $R_c$ and $R_d$ represent a hydrogen atom or a linear or branched $(C_1\text{-}C_6)$alkyl group,

which compound of formula (I/v') may also be obtained directly starting from the compounds of formula (I/k) by treatment with $NaN_3$ to yield the intermediate azide compounds, followed by hydrogenation in the presence of palladium, yielding the compounds of formula (I/v') in which Rc and Rd represent a hydrogen atom,

which compounds of formulae (I/v) and (I/v') may be subjected:

- either, when Rc and Rd represent a hydrogen atom, to the action of N,N'-carbonyldiimidazole or N,N'-thiocarbonyldiimidazole, for example, to yield the compounds of formulae (I/w) and (I/w'), respectively, which are particular cases of the compounds of formula (I):

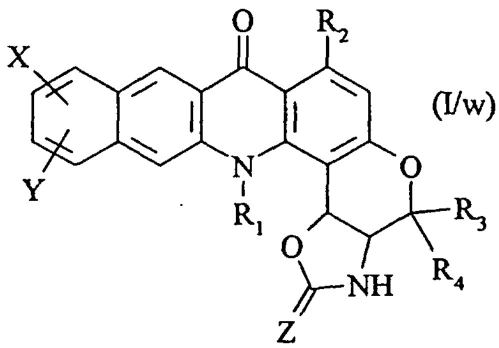 (I/w)

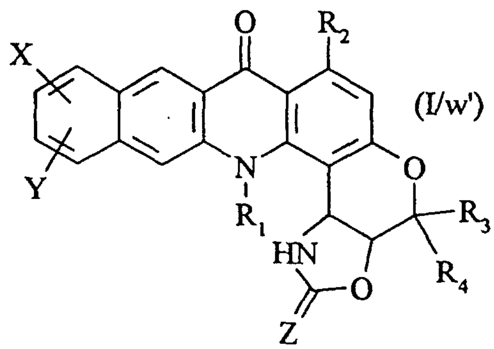 (I/w')

in which X, Y, $R_1$, $R_2$, $R_3$ and $R_4$ are as defined above and Z represents an oxygen atom or a sulphur atom, according to the nature of the reagent used,

- or to the action of a compound of formula (XI) $WO_2C$-B'-$CO_2W$ as defined above, to yield the compounds of formulae (I/x) and (I/x'), respectively, which are particular cases of the compounds of formula (I):

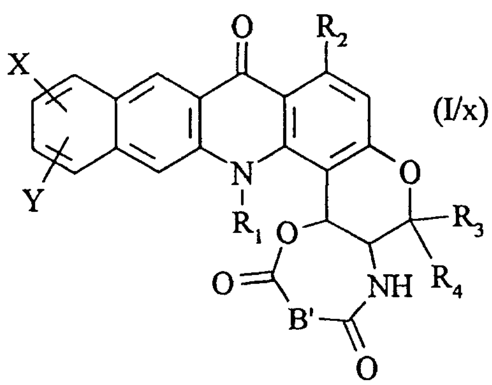 (I/x)

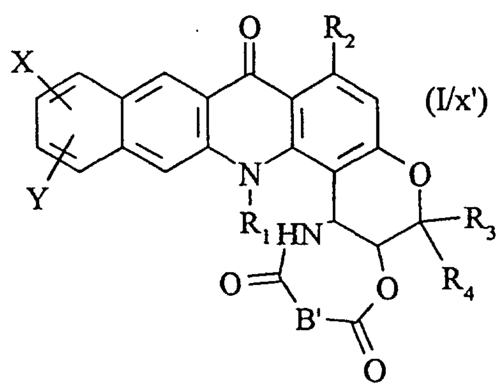 (I/x')

in which B', X, Y, $R_1$, $R_2$, $R_3$ and $R_4$ are as defined above,

- or to the action of triphenylphosphine dibromide in the presence of triethylamine to yield the compounds of formula (I/y), a particular case of the compounds of formula (I):

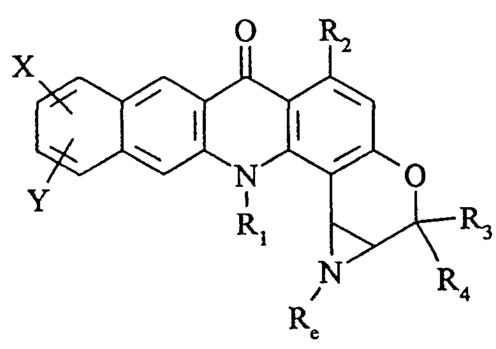

(I/y)

in which X, Y, $R_1$, $R_2$, $R_3$ and $R_4$ are as defined above and $R_e$ represents a hydrogen atom or a linear or branched ($C_1$-$C_6$)alkyl group,

e) ⇨ or to the action of $NaN_3$ in the presence of hydrogen peroxide, followed by a reducing step by means of tri-n-butyltin hydride, for example, to yield the compounds of formula (I/z), a particular case of the compounds of formula (I):

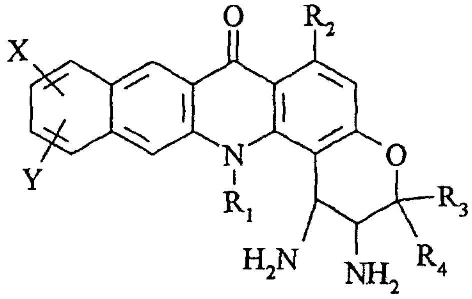

(I/z)

in which X, Y, $R_1$, $R_2$, $R_3$ and $R_4$ are as defined above,
which compounds of formula (I/z) may optionally be subjected to the action of carbon dioxide, in the presence of diphenyl phosphite, to yield the compounds of formula (I/aa), a particular case of the compounds of formula (I):

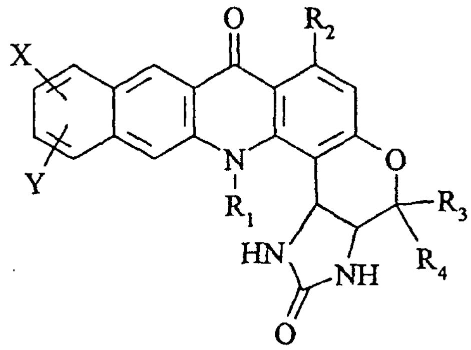

(I/aa)

in which X, Y, $R_1$, $R_2$, $R_3$ and $R_4$ are as defined above,

the compounds (I/a-z), (I/g'), (I/v'-x'), (I/aa) and *(cis* I/h-I/i) forming the totality of the compounds of the invention, which are purified, where appropriate, in accordance with a conventional purification technique, may, if desired, be separated into their various optical isomers in accordance with a conventional separating technique and, if desired, are converted into their N-oxides and, where appropriate, into their addition salts with a pharmaceutically acceptable acid or base.

**12.** Pharmaceutical compositions comprising as active ingredient at least one compound according to any one of claims I to 10, on its own or in combination with one or more inert, non-toxic, pharmaceutically acceptable excipients or carriers.

**13.** Pharmaceutical compositions according to claim 12 comprising at least one active ingredient according to any one of claims 1 to 10 for use in the treatment of cancer.